# FASCICULE DE BREVET EUROPEEN

(11) **EP 1 548 006 B1**
(45) Date de publication et mention de la délivrance du brevet: **05.09.2007**
(21) Numéro de dépôt: 04292885.3
(22) Date de dépôt: 06.12.2004
(51) Int. Cl.: C07D 213/77

(54) **Synthèse de composés tétraazapentaméthiniques, procédé de coloration de fibres keratiniques mettant en oeuvre cette synthèse**
Synthese von Tetraazapentamethin-derivate, Verfahren zum Färben von Keratinfasern, welche solche Synthese verwendet
Synthesis of tetraazapentamethine derivatives, keratin fibers dyeing process which uses such synthesis

(30) Priorité: 12.12.2003 FR 0314655
(43) Date de publication de la demande: 29.06.2005
(73) Titulaire: L'ORÉAL, 75008 Paris (FR)
(72) Inventeur: Pereira, Rui, 77600 Bussy Saint Georges (FR); Burgaud, Hervé 28, 77230 Damartin en Goele (FR)
(74) Mandataire: Wattremez, Catherine

(56) Documents cités:
- EP-A- 1 378 544
- FR-A- 1 291 555
- HUENIG S ET AL: "TETRAAZA-PENTAMETHINFARBSTOFFE" JUSTUS LIEBIGS ANNALEN DER CHEMIE, VERLAG CHEMIE GMBH. WEINHEIM, DE, vol. 667, 1963, pages 72-85, XP002237035 ISSN: 0075-4617

## Description

La présente invention concerne le domaine de la teinture des fibres kératiniques, elle concerne plus particulièrement un procédé de préparation de composés tétraazapentaméthiniques à partir des azines correspondantes, ainsi qu'un procédé de teinture des fibres kératiniques dans lequel on met en oeuvre le procédé de préparation de ces composés.

Il est connu de colorer les fibres kératiniques, notamment humaines, et en particulier les cheveux, avec des compositions tinctoriales contenant des colorants directs qui sont des molécules colorées et colorantes ayant une affinité pour les fibres. La composition est appliquée sur les fibres, laissée poser pendant une durée suffisante pour obtenir un degré de coloration approprié, puis généralement les fibres sont rincées.

Comme colorants directs classiquement employés, on peut citer par exemple des colorants directs nitrés benzéniques, anthraquinoniques, nitropyridiniques, azoïques, xanthéniques, acridiniques, aziniques ou triarylméthaniques.

L'utilisation de colorants directs permet d'obtenir des colorations particulièrement chromatiques, qui sont cependant temporaires ou semi-permanentes, à cause de la nature des liaisons entre les colorants directs et la fibre kératinique. Ces interactions font que la désorption des colorants de la surface et/ou du coeur de la fibre se fait relativement facilement.

Les colorations présentent également et en général, une faible puissance tinctoriale et une tenue aux lavages ou à la transpiration toujours considérée comme insuffisante.

Ces colorants directs sont en outre généralement sensibles à la lumière car la résistance du chromophore vis-à-vis des attaques photochimiques est faible, ce qui conduit à un affadissement de la coloration des cheveux dans le temps.

En outre, la sensibilité de ces colorants à la lumière dépend de leur répartition uniforme ou en aggrégats dans et/ou sur la fibre kératinique.

Il peut aussi y avoir des problèmes de stabilité du colorant direct dans la composition tinctoriale même, ce qui conduit à un changement de couleur du colorant ainsi qu'à une mauvaise conservation de la composition tinctoriale.

La présente invention a pour objet de résoudre les problèmes ci-dessus.

Elle a donc pour premier objet un procédé de préparation de composés tétraazapentaméthiniques de formule (I) suivante : 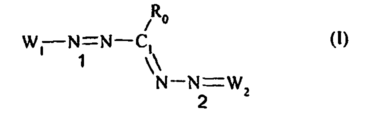 dans laquelle
- W₁ représente un radical hétéroaromatique cationique de formule (II) ou (III) : 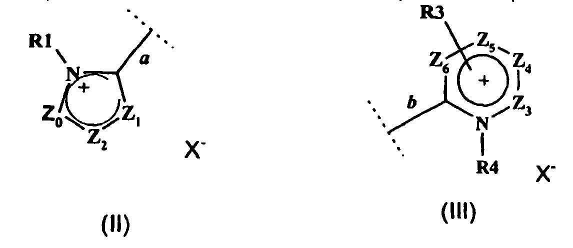
- W₂ représente un radical hétéroaromatique de formule (IV) ou (V) : 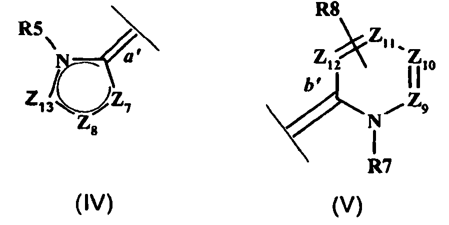 dans lesquelles :
- Z₀ représente un radical CR₂, un atome d'azote ou un radical NR₂₁,
- Z₁ représente un atome d'oxygène, de soufre ou un radical NR₉,
- Z₂ représente un atome d'azote ou un radical CR₁₀,
- Z₃ représente un atome d'azote ou un radical CR₁₁,
- Z₄ représente un atome d'azote ou un radical CR₁₂,
- Z₅ représente un atome d'azote ou un radical CR₁₃,
- Z₆ représente un atome d'azote ou un radical CR_{14,}
- Z₇ représente un atome d'oxygène, de soufre ou un radical NR₁₅,
- Z₈ représente un atome d'azote ou un radical CR₁₆,
- Z₉ représente un atome d'azote ou un radical CR₁₇,
- Z₁₀ représente un atome d'azote ou un radical CR₁₈,
- Z₁₁ représente un atome d'azote ou un radical CR₁₉,
- Z₁₂ représente un atome d'azote ou un radical CR₂₀,
- Z₁₃ représente un radical CR₆, un atome d'azote ou un radical NR₂₂,
- étant entendu que chacun des cycles des formules (II), (III), (IV) et (V) ne comportent pas plus de trois atomes d'azote et que deux des trois atomes d'azote peuvent être contigus,
- la liaison a du radical hétéroaromatique cationique à 5 chaînons de la formule (II) étant reliée à l'atome d'azote N₁ de la formule (I),
- la liaison b du radical hétéroaromatique cationique à 6 chaînons de la formule (III) étant reliée à l'atome d'azote N₁ de la formule (I).
- la double liaison a' du radical hétéroaromatique à 5 chaînons de la formule (IV) étant reliée à l'atome d'azote N₂ de la formule (I),
- la double liaison b' du radical hétéroaromatique à 6 chaînons de la formule (V) étant reliée à l'atome d'azote N₂ de la formule (I),
- la liaison b, reliant le radical hétéroaromatique cationique de la formule III à l'atome d'azote N₁ de la formule (I), étant située en position ortho ou para de l'atome d'azote portant le radical R₄ lorsque Z₅ représente un radical CR₁₃ ; la liaison b étant située en position ortho de l'atome d'azote portant le radical R₄ lorsque Z₅ représente un atome d'azote,
- la liaison b', reliant le radical hétéroaromatique de la formule V à l'atome d'azote N₂ de la formule (I), étant située en position ortho ou para de l'atome d'azote portant le radical R₇ lorsque Z₁₁ représente un radical CR₁₉ ; la liaison b' étant située en position ortho de l'atome d'azote portant le radical R₇ lorsque Z₁₁ représente un atome d'azote,
- R₂, R₆, R₁₀ et R₁₆ représentent, indépendamment l'un de l'autre, un atome d'hydrogène ; un radical alkyle en C₁-C₄ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle non substitué ou substitué par un ou deux radicaux choisis parmi les radicaux hydroxy, alcoxy en C₁-C₂, (poly)-hydroxyalcoxy en C₂-C₄, amino, (di)alkylamino en C₁-C₂ ; un radical carboxy ; un radical sulfonylamino,
- R₁, R₄, R₅, R₇, R₉, R₁₅, R₂₁, et R₂₂ représentent, indépendamment l'un de l'autre, un radical alkyle en C₁-C₈ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique;
- R₀, R₃, R₈, R₁₁, R₁₂, R₁₃, R₁₄, R₁₇, R₁₈, R₁₉, et R₂₀ représentent, indépendamment l'un de l'autre, un atome d'hydrogène, une chaîne hydrocarbonée en C₁-C₁₆ linéaire ou ramifiée, cette chaîne pouvant être saturée ou insaturée par une à trois insaturations, cette chaîne étant non substituée ou substituée par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique, sulfonylamino, (poly)-hydroxyalkylamino en C₂-C₄ ou un atome d'halogène tel que chlore, fluor ou brome ; un radical phényle non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique, sulfonylamino, (poly)-hydroxyalkylamino en C₂-C₄ ou un atome d'halogène tel que chlore, fluor ou brome ; un radical hétéroaryle choisi parmi les radicaux pyrazolyle, pyrrolyle, imidazolyle, thiazolyle, oxazolyle, triazolyle, pyridinyle, pyrimidinyle, triazinyle, pyrazinyle, pyridazinyle, en outre cette chaîne hydrocarbonée peut être interrompue par un ou deux atomes d'oxygène, d'azote, de soufre ou par un radical SO₂,
- étant entendu que R₀, R₃, R₈, R₁₁, R₁₂, R₁₃, R₁₄, R₁₇, R₁₈, R₁₉, et R₂₀ ne comportent pas de liaison peroxyde, ni de radicaux diazo ou nitroso,
- R₂ avec R₁₀, R₁₁ avec R₁₂, R₆ avec R₁₆, et R₁₇ avec R₁₈ pouvant former indépendamment les uns des autres un cycle aromatique carboné à 5 ou 6 chaînons non substitué ou substitué par un ou deux radicaux hydroxy, amino, (di)alkyl (C₁-C₂) amino, alcoxy C₁-C₂, (poly)hydroxyalkyl C₂-C₄ amino,
- X est un anion organique ou minéral ;
   Le procédé selon l'invention consiste à faire réagir, en présence d'au moins un agent oxydant, au moins un composé azine de formule (VI) et/ou (VII) : 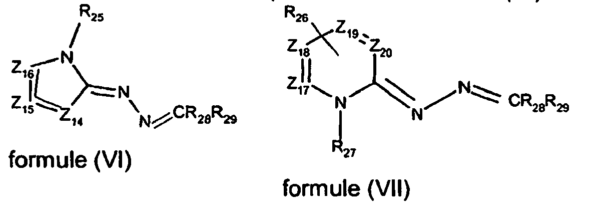 dans lesquelles :
- Z₁₄, Z₁₅, Z₁₆, R₂₅ ont respectivement les mêmes significations que Z₇, Z₈, Z₁₃, R₅ de la formule (IV), ou que Z₁, Z₂, Z₀, R₁ de la formule (II), définies ci-dessus,
- Z₁₇, Z₁₈, Z₁₉, Z₂₀, R₂₆, R₂₇ ont respectivement les mêmes significations que Z₉, Z₁₀, Z₁₁, Z₁₂, R₈, R₇ de la formule (V), ou que Z₃, Z₄, Z₅, Z₆, R₃, R₄ de la formule (III), définies ci-dessus,
- R₂₈, R₂₉, indépendamment l'un de l'autre, représente un atome d'hydrogène, un radical alkyle en C₁-C₆, de préférence en C₁-C₄, un radical alcoxy, linéaire ou ramifié, en C₁-C₆, de préférence en C₁-C₄, à la condition que l'un au moins des deux radicaux soit différent de l'hydrogène.

La présente a de même pour objet un procédé de coloration de fibres kératiniques, notamment humaines, consistant à appliquer, sur des fibres sèches ou humides, une composition tinctoriale comprenant au moins un composé de formule (VI) ou (VII) en présence d'au moins un agent oxydant, puis de laisser poser pendant une durée suffisante pour développer la coloration désirée.

La présente invention permet notamment de s'affranchir des problèmes de stabilité du colorant direct dans la composition tinctoriale. En effet, ledit colorant direct se trouve sous la forme d'un précurseur dont la stabilité est améliorée par rapport à celle du colorant lui-même.

Par ailleurs, les colorants sont synthétisés de manière simple lors de l'application de la composition tinctoriale sur les fibres, ou bien encore peu de temps avant cette dernière.

Il existe des procédés connus de synthèse de dérivés tétraazapenthaméthiniques mettant en jeu une azine avec un équivalent molaire d'hydrazone, en présence d'un agent oxydant, comme par exemple celui décrit dans FR 1 291 555.

Mais contrairement à ce type de procédés, et ce de manière totalement inattendue, le procédé de l'invention ne nécessite pas la présence d'hydrazone.

Enfin, les colorants synthétisés selon le procédé de l'invention permettent d'obtenir des colorations très chromatiques, sans oublier les nuances "fondamentales" comme les noirs et les marrons. En outre, ils sont stables à la lumière et résistants aux intempéries et à la transpiration.

D'autres caractéristiques et avantages de la présente invention apparaîtront plus clairement à la lecture de la description et des exemples qui vont suivre.

Au sens de la présente demande, un anion organique ou minéral est par exemple choisi parmi un halogénure tel que chlorure, bromure, fluorure, iodure ; un hydroxyde ; un sulfate ; un hydrogénosulfate ; un alkyl(C₁-C₆)sulfate tel que par exemple un méthylsulfate ou un éthylsulfate ; un acétate ; un tartrate ; un oxalate ; un alkyl(C₁-C₆)sulfonate tel que méthylsulfonate ; un arylsulfonate non substitué ou substitué par un radical alkyle en C₁-C₄ tel que par exemple un 4-toluylsulfonate.

Les composés de formule (I) dont la synthèse constitue le premier objet de l'invention vont tout d'abord être décrits.

Conformément à un mode de réalisation de l'invention, R₀ représente un atome d'hydrogène ; un radical alkyle en C₁-C₆ linéaire ou ramifié, non substitué ou substitué par un à trois radicaux choisis parmi les radicaux hydroxy, alcoxy en C₁-C₂, (poly)hydroxyalcoxy en C₂-C₄, amino, (di)alkylamino en C₁-C₂, (poly)hydroxyalkylamino en C₂-C₄ , carboxy ou sulfonique ; un radical phényle non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique ou un atome d'halogène tel que chlore, fluor ou brome, un radical hétéroaryle éventuellement cationique choisi parmi les radicaux pyrazolyle, pyrrolyle, imidazolyle, thiazolyle, oxazolyle, triazolyle, pyridinyle, pyrimidinyletriazinyle, pyrazinyle, pyridazinyle.

Plus particulièrement, R₀ représente un atome d'hydrogène ; un radical alkyle en C₁-C₃ linéaire ou ramifié, non substitué ou substitué par un à deux radicaux choisis parmi les radicaux hydroxy, alcoxy en C₁-C₂, amino, (di)alkylamino en C₁-C₂, carboxy ou sulfonique ; un radical phényle non substitué ou substitué par un à deux radicaux choisis parmi les radicaux hydroxy, amino, (di)alkylamino en C₁-C₂ ou (poly)-hydroxyalkylamino en C₂-C₄, un radical hétéroaryle éventuellement cationique choisi parmi les radicaux pyrazolyle, pyrrolyle, imidazolyle, pyridinyle.

Selon une variante avantageuse de l'invention, R₀ représente un atome d'hydrogène; un méthyle, éthyle, propyle, butyle, 2-hydroxyéthyle, 2-aminoéthyle ;1-carboxyméthyle, 2-carboxyéthyle, 2-sulfonyléthyle, 2-méthoxyéthyle ; un radical phényle non substitué ou substitué par un à deux radicaux choisis parmi les radicaux amino, (di)alkylamino en C₁-C₂, (poly)-hydroxyalkylamino en C₂-C₄, un radical hétéroaryle éventuellement cationique choisi parmi les radicaux pyrazolyle, pyrrolyle, imidazolyle, pyridinyle.

De préférence, R₀ représente un atome d'hydrogène ; un radical méthyle, éthyle, 2-méthoxyéthyle ; un radical phényle non substitué ou substitué par un radical un amino, (di)méthylamino , (di)(2-hydroxyéthyl)amino, un radical hétéroaryle éventuellement cationique choisi parmi les radicaux imidazolyle, pyridinyle.

Selon un mode de réalisation particulier de l'invention, les radicaux R₂, R₆, R₁₀ et R₁₆, identiques ou non, représentent, un atome d'hydrogène, un radical phényle, un radical alkyle en C₁-C₄ non substitué ou substitué par un à deux radicaux choisis parmi les radicaux hydroxy, amino, (di)alkylamino en C₁-C₂, carboxy.

Plus avantageusement, les radicaux R₂, R₆, R₁₀ et R₁₆ représentent, identiques ou non, un atome d'hydrogène, un radical méthyle, phényle, 2-hydroxyméthyle, un carboxy, un radical phényle.

Les radicaux R₁, R₄, R₅, R₇, R₉, R₁₅, R₂₁ et R₂₂, indentiques ou non, représentent plus particulièrement un radical alkyle en C₁-C₄ non substitué ou substitué par un à deux radicaux choisis parmi les radicaux hydroxy, alcoxy en C₁-C₂, amino, (di)alkylamino en C₁-C₂, carboxy, sulfonique.

De préférence, les radicaux R₁, R₄, R₅, R₇, R₉, R₁₅, R₂₁ et R₂₂, identiques ou non, représentent un radical méthyle, éthyle, 2-hydroxyéthyle, 1-carboxyméthyle, 2-carboxyéthyle, 2-sulfonyléthyle.

Pour ce qui a trait aux radicaux R₃, R₈, R₁₁, R₁₂, R₁₃, R₁₄, R₁₇, R₁₈, R₁₉, et R₂₀, ces derniers, identiques ou non, représentent un atome d'hydrogène ; un radical alkyle en C₁-C₄ linéaire ou ramifié, non substitué ou substitué par un à deux radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique; un radical phényle non substitué ou substitué par un à deux radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique ou un atome d'halogène tel que chlore, fluor ou brome ; un radical sulfonylamino ; un radical (poly)-hydroxyalkylamino en C₂-C_{4.}

De préférence, R₃, R₈, R₁₁, R₁₂, R₁₃, R₁₄, R₁₇, R₁₈, R₁₉, et R₂₀, identiques ou non, représentent un atome d'hydrogène, un radical alkyle en C₁-C₄ non substitué ou substitué par un à deux radicaux choisis parmi les radicaux hydroxy, un radical alcoxy en C₁-C₂ ; un radical amino ; un radical (di)alkylamino en C₁-C₂ ; un radical carboxy ; un radical (poly)-hydroxyalkylamino en C₂-C₄.

Conformément à une variante plus particulière de l'invention, les radicaux R₃, R₈, R₁₁, R₁₂, R₁₃, R₁₄, R₁₇, R₁₈, R₁₉, et R₂₀, identiques ou non, représentent un atome d'hydrogène, un radical méthyle, 2-hydroxyméthyle, un carboxy, un radical méthoxy, éthoxy, 2-hydroxyéthyloxy, un radical amino, méthylamino, diméthylamino, 2-hydroxyéthylamino.

Une première classe de composés particulièrement avantageux de formule (I) correspond à ceux pour lesquels, W₁ est un radical 2-pyridinium et W₂ est un radical 2-pyridine.

De préférence, ces composés sont les suivants:
- Chlorure de 2-[5-(N-methyl-2-pyridinylidène)-1-formazano]-N-méthylpyridinium
- Chlorure de 2-[5-(N-methyl-2-pyridinylidène)-3-méthyl-1-formazano]-N-méthyl pyridinium
- Chlorure de 2-[5-(N-methyl-2-pyridinylidène)-3-éthyl-1-formazano]-N-méthylpyridinium
- Chlorure de 2-[5-(N-méthyl-2-pyridinylidene)-3-isopropyl-1 -formazano]-N-méthyl pyridinium
- Chlorure de 2-[5-(N-methyl-2-pyridinylidène)-3-phényl-1-formazano]-N-méthyl pyridinium
- Chlorure de 2-[5-(N-méthyl-2-pyridinylidène)-3-(4'-methoxyphényl)-1-formazano]-N-méthylpyridinium
- Chlorure de 2-[5-(N-méthyl-2-pyridinylidène)-3-(4'-hydroxyphényl)-1-formazano]-N-méthylpyridinium
- Chlorure de 2-[5-(N-méthyl-2-pyridinylidène)-3-(4'-N,N-dimethylaminophényl)-1-formazano]-N-méthylpyridinium
- Chlorure de 2-[5-(4-pyrrolidino-N-méthyl-2-pyridinylidène)-1-formazano]-4-pyrrolidino-N-méthylpyridinium
- Chlorure de 2-[5-(4-pyrrolidino-N-méthyl-2-pyridinylidène)-3-méthyl-1-formazano]- 4-pyrrolidino-N-méthylpyridinium
- Chlorure de 2-[5-(4-pyrrolidino-N-méthyl-2-pyridinylidène)-3-éthyl-1-formazano]- 4-pyrrolidino-N-méthylpyridinium
- Chlorure de 2-[5-(4-pyrrolidino-N-méthyl-2-pyridinylidène)-3-isopropyl-1-formazano]- 4-pyrrolidino-N-méthylpyridinium
- Chlorure de 2-[5-(4-pyrrolidino-N-méthyl-2-pyridinylidène)-3-phenyl-1-formazano]- 4-pyrrolidino-N-méthylpyridinium
- Chlorure de 2-[5-(4-pyrrolidino-N-methyl-2-pyridinylidène)-3-(4'-methoxyphenyl)-1-formazano]- 4-pyrrolidino-N-méthylpyri-dinium
- Chlorure de 2-[5-(4-pyrrolidino-N-méthyl-2-pyridinylidène)-3-(4'-hydroxyphényl)-1-formazano]- 4-pyrrolidino-N-méthylpyridinium
- Chlorure de 2-[5-(4-pyrrolidino-N-méthyl-2-pyridinylidène)-3-(4'-N,N-diméthylamino phényl)-1-formazano]- 4-pyrrolidino-N-méthylpyridinium
- Chlorure de 2-[5-(N-hydroxyéthyl-2-pyridinylidène)-1-formazano]-N-hydroxyethyl pyridinium
- Chlorure de 2-[5-(N-hydroxyethyl-2-pyridinylidène)-3-méthyl-1-formazano]-N-hydroxy éthylpyridinium
- Chlorure de 2-[5-(N-hydroxyethyl-2-pyridinylidène)-3-éthyl-1-formazano]-N- hydroxy éthylpyridinium
- Chlorure de 2-[5-(N- hydroxyéthyl -2-pyridinylidène)-3-phényl-1-formazano]-N- hydroxy éthylpyridinium.
- Chlorure de 2-[5-(N- hydroxyéthyl -2-pyridinylidène)-3-isopropyl-1-formazano]-N-hydroxyéthylpyridinium
- Chlorure de 2-[5-(N- hydroxyéthyl-2-pyridinylidène)-3-(4'-méthoxyphényl)-1-formazano]-N- hydroxyéthyl -pyridinium
- Chlorure de 2-[5-(N- hydroxyéthyl -2-pyridinylidène)-3-(4'-hydroxyphényl)-1-formazano]-N- hydroxyéthyl -pyridinium
- Chlorure de 2-[5-(N- hydroxyéthyl -2-pyridinylidène)-3-(4'-N,N-diméthylaminophényl)-1-formazano]-N- hydroxyéthyl -pyridinium
- Chlorure de 2-[5-(N-carboxyéthyl-2-pyridinylidène)-1-formazano]-N- carboxyéthyl-pyridinium
- Chlorure de 2-[5-(N- carboxyéthyl -2-pyridinylidène)-3-méthyl-1-formazano]-N- carboxy éthyl -pyridinium
- Chlorure de 2-[5-(N- carboxyéthyl -2-pyridinylidène)-3-éthyl-1-formazano]-N- carboxy éthyl -pyridinium
- Chlorure de 2-[5-(N- carboxyéthyl -2-pyridinylidène)-3-phényl-1-formazano]-N- carboxy éthyl -pyridinium
- Chlorure de 2-[5-(N- carboxyéthyl -2-pyridinylidène)-3-isopropyl-1-formazano]-N-carboxyéthyl -pyridinium
- Chlorure de 2-[5-(N- carboxyéthyl -2-pyridinylidène)-3-(4'-méthoxyphényl)-1-formazano]-N- carboxyéthyl -pyridinium
- Chlorure de 2-[5-(N- carboxyéthyl -2-pyridinylidène)-3-(4'-hydroxyphényl)-1-formazano]-N- carboxyéthyl -pyridinium
- Chlorure de 2-[5-(N- carboxyéthyl -2-pyridinylidène)-3-(4'-N,N-diméthylaminophényl)-1-formazano]-N- carboxyéthyl -pyridinium.

Une autre classe de composés de formule (I) avantageux correspond à ceux pour lesquels W₁ est un radical 4-pyridinium et W₂ est un radical 4-pyridine.

De préférence, ces composés sont choisis parmi les suivants :
- Chlorure de 4-[5-(N-méthyl-4-pyridinylidène)-1-formazano]-N-méthylpyridinium
- Chlorure de 4-[5-(N-méthyl-4-pyridinylidène)-3-méthyl-1-formazano]-N-méthyl pyridinium
- Chlorure de 4-[5-(N-méthyl-4-pyridinylidène)-3-éthyl-1-formazano]-N-méthylpyridinium
- Chlorure de 4-[5-(N-méthyl-4-pyridinylidène)-3-isopropyl-1-formazano]-N-méthyl pyridinium
- Chlorure de 4-[5-(N-méthyl-4-pyridinylidène)-3-phényl-1-formazano]-N-méthyl pyridinium
- Chlorure de 4-[5-(N-méthyl-4-pyridinylidène)-3-(4'-méthoxyphényl)-1-formazano]-N-méthylpyridinium
- Chlorure de 4-[5-(N-méthyl-4-pyridinylidène)-3-(4'-hydroxyphényl)-1-formazano]-N-méthylpyridinium
- Chlorure de 4-[5-(N-méthyl-4-pyridinylidène)-3-(4'-N,N-diméthylaminophényl)-1-formazano]-N-méthylpyridinium
- Chlorure de 4-[5-(N-hydroxyéthyl-4-pyridinylidène)-1-formazano]-N-hydroxyéthyl pyridinium
- Chlorure de 4-[5-(N-hydroxyéthyl-4-pyridinylidène)-3-méthyl-1-formazano]-N-hydroxy éthylpyridinium
- Chlorure de 4-[5-(N-hydroxyéthyl-4-pyridinylidène)-3-éthyl-1-formazano]-N- hydroxy éthylpyridinium
- Chlorure de 4-[5-(N- hydroxyéthyl -4-pyridinylidène)-3-phényl-1-formazano]-N- hydroxy éthylpyridinium
- Chlorure de 4-[5-(N- hydroxyéthyl -4-pyridinylidène)-3-isopropyl-1-formazano]-N-hydroxyéthylpyridinium
- Chlorure de 4-[5-(N- hydroxyéthyl-4-pyridinylidène)-3-(4'-méthoxyphényl)-1-formazano]-N- hydroxyéthyl -pyridinium
- Chlorure de 4-[5-(N- hydroxyéthyl -4-pyridinylidène)-3-(4'-hydroxyphényl)-1-formazano]-N- hydroxyéthyl -pyridinium
- Chlorure de 4-[5-(N- hydroxyéthyl -4-pyridinylidène)-3-(4'-N,N-diméthylaminophényl)-1-formazano]-N- hydroxyéthyl -pyridinium
- Chlorure de 4-[5-(N-carboxyéthyl-4-pyridinylidène)-1-formazano]-N- carboxyéthyl-pyridinium
- Chlorure de 4-[5-(N- carboxyéthyl -4-pyridinylidène)-3-méthyl-1-formazano]-N-carboxyéthyl -pyridinium
- Chlorure de 4-[5-(N- carboxyéthyl -4-pyridinylidène)-3-éthyl-1-formazano]-N-carboxyéthyl -pyridinium
- Chlorure de 4-[5-(N- carboxyéthyl -4-pyridinylidène)-3-phényl-1-formazano]-N-carboxyéthyl -pyridinium
- Chlorure de 4-[5-(N- carboxyéthyl -4-pyridinylidène)-3-isopropyl-1-formazano]-N-carboxyéthyl -pyridinium
- Chlorure de 4-[5-(N- carboxyéthyl -4-pyridinylidène)-3-(4'-méthoxyphényl)-1-formazano]-N- carboxyéthyl -pyridinium
- Chlorure de 4-[5-(N- carboxyéthyl -4-pyridinylidène)-3-(4'-hydroxyphényl)-1-formazano]-N- carboxyéthyl -pyridinium
- Chlorure de 4-[5-(N- carboxyéthyl -4-pyridinylidène)-3-(4'-N,N-diméthylaminophényl)-1-formazano]-N- carboxyéthyl -pyridinium.

Une autre classe de composés de formule (I) avantageux correspond à ceux pour lesquels W₁ est un radical 2-imidazolium et W₂ est un radical 2-imidazole.

De préférence, ces composés sont choisis parmi les suivants :
- Chlorure de 2-[5-(1,3-diméthyl-2-imidazolidène)-1-formazano]-1,3-diméthyl-imidazolium
- Chlorure de 2-[5-(1,3-diméthyl-2-imidazolidène)-3-méthyl-1-formazano]-1,3-diméthyl-imidazolium
- Chlorure de 2-[5-(1,3-diméthyl-2-imidazolidène)-3-éthyl-1-formazano]-1,3-diméthyl-imidazolium
- Chlorure de 2-[5-(1,3-diméthyl-2-imidazolidène)-3-isopropyl-1-formazano]-1,3-diméthyl-imidazolium
- Chlorure de 2-[5-(1,3-diméthyl-2-imidazolidène)-3-phényl-1-formazano]-1,3-diméthyl-imidazolium
- Chlorure de 2-[5-(1,3-diméthyl-2-imidazolidène)-3-(4'-methoxyphenyl)-1-formazano]-1,3-diméthyl-imidazolium
- Chlorure de 2-[5-(1,3-diméthyl-2-imidazolidène)-3-(4'-hydroxyphényl)-1-formazano]-1,3-diméthyl-imidazolium
- Chlorure de 2-[5-(1,3-diméthyl-2-imidazolidène)-3-(4'-N,N-diméthylaminophényl)-1-formazano]-1,3-diméthyl-imidazolium
- Chlorure de 2-[5-(1,3-dihydroxyéthyl-2-imidazolidène)-1-formazano]-1,3-dihydroxyéthyl imidazolium
- Chlorure de 2-[5-(1,3-dihydroxyéthyl-2-imidazolidène)-3-méthyl-1-formazano]-1,3-dihydroxyéthylimidazolium
- Chlorure de 2-[5-(1,3-dihydroxyéthyl-2-imidazolidène)-3-éthyl-1-formazano]-1,3-dihydroxyéthyl -imidazolium
- Chlorure de 2-[5-(1,3-dihydroxyéthyl -2-imidazolidène)-3-phenyl-1-formazano]-1,3-dihydroxyéthyl -imidazolium
- Chlorure de 2-[5-(1,3-dihydroxyéthyl -2-imidazolidène)-3-isopropyl-1-formazano]-1,3-dihydroxyéthyl -imidazolium
- Chlorure de 2-[5-(1,3-dihydroxyéthyl-2-imidazolidène)-3-(4'-méthoxyphényl)-1-formazano]-1,3-dihydroxyéthyl -imidazolium
- Chlorure de 2-[5-(1,3-dihydroxyéthyl -2-imidazolidène)-3-(4'-hydroxyphényl)-1-formazano]-1,3-dihydroxyéthyl -imidazolium
- Chlorure de 2-[5-(1,3-dihydroxyéthyl -2-imidazolidène)-3-(4'-N,N-diméthylamino phényl)-1-formazano]-1,3-dihydroxyéthyl -imidazolium
- Chlorure de 2-[5-(1,3-dicarboxyéthyl-2-imidazolidène)-1-formazano]-1,3-dicarboxyéthyl imidazolium
- Chlorure de 2-[5-(1,3-dicarboxyéthyl -2-imidazolidène)-3-méthyl-1-formazano]-1,3-dicarboxyéthyl -imidazolium
- Chlorure de 2-[5-(1,3-dicarboxyéthyl -2-imidazolidène)-3-éthyl-1 -formazano]-1,3-dicarboxyéthyl -imidazolium
- Chlorure de 2-[5-(1,3-dicarboxyéthyl -2-imidazolidène)-3-phényl-1-formazano]-1,3-dicarboxyéthyl -imidazolium
- Chlorure de 2-[5-(1,3-dicarboxyéthyl -2-imidazolidène)-3-isopropyl-1-formazano]-1,3-dicarboxyéthyl -imidazolium
- Chlorure de 2-[5-(1,3-dicarboxyéthyl -2-imidazolidène)-3-(4'-méthoxyphényl)-1-formazano]-1,3-dicarboxyéthyl -imidazolium
- Chlorure de 2-[5-(1,3-dicarboxyéthyl -2-imidazolidène)-3-(4'-hydroxyphényl)-1-formazano]-1,3-dicarboxyéthyl -imidazolium
- Chlorure de 2-[5-(1,3-dicarboxyéthyl -2-imidazolidène)-3-(4'-N,N-diméthylamino phényl)-1-formazano]-1,3-dicarboxyéthyl -imidazolium.

Une autre classe de composés de formule (I) avantageux correspond à ceux pour lesquels W₁ est un radical 5-pyrazolium et W₂ est un radical 5-pyrazole.

De préférence, ces composés sont les suivants :
- Chlorure de 5-[5-(1,2-diméthyl-5-pyrazolidène)-1-formazano]-1,2-diméthyl-pyrazolinium
- Chlorure de 5-[5-(1,2-diméthyl-5-pyrazolidène)-3-méthyl-1-formazano]-1,2-diméthyl-pyrazolinium
- Chlorure de 5-[5-(1,2-diméthyl-5-pyrazolidène)-3-éthyl-1-formazanol-1,2-diméthyl-pyrazolinium
- Chlorure de 5-[5-(1,2-diméthyl-5-pyrazolidène)-3-isopropyl-1-formazano]-1,2-diméthyl-pyrazolinium
- Chlorure de 5-[5-(1,2-diméthyl-5-pyrazolidene)-3-phényl-1-formazano]-1,2-diméthyl-pyrazolinium
- Chlorure de 5-[5-(1,2-diméthyl-5-pyrazolidène)-3-(4'-méthoxyphényl)-1-formazano]-1,2-diméthyl-pyrazolinium
- Chlorure de 5-[5-(1,2-diméthyl-5-pyrazolidène)-3-(4'-hydroxyphényl)-1-formazano]-1,2-diméthyl-pyrazolinium
- Chlorure de 5-[5-(1,2-diméthyl-5-pyrazolidène)-3-(4'-N,N-diméthylaminophényl)-1-formazano]-1,2-diméthyl-pyrazolinium
- Chlorure de 5-[5-(1,2-dihydroxyéthyl-5-pyrazolidène)-1-formazano]-1,2-dihydroxyéthyl pyrazolinium
- Chlorure de 5-[5-(1,2-dihydroxyéthyl-5-pyrazolidène)-3-méthyl-1-formazano]-1,2-dihydroxyéthylpyrazolinium
- Chlorure de 5-[5-(1,2-dihydroxyéthyl-5-pyrazolidène)-3-éthyl-1-formazano]-1,2-dihydroxyéthyl -pyrazolinium
- Chlorure de 5-[5-(1,2-dihydroxyéthyl -5-pyrazolidène)-3-phényl-1-formazano]-1,2-dihydroxyéthyl -pyrazolinium
- Chlorure de 5-[5-(1,2-dihydroxyéthyl -5-pyrazolidène)-3-isopropyl-1-formazano]-1,2-dihydroxyéthyl -pyrazolinium
- Chlorure de 5-(5-(1,2-dihydroxyéthyl-5-pyrazolidène)-3-(4'-méthoxyphényl)-1-formazano]-1,2-dihydroxyéthyl -pyrazolinium
- Chlorure de 5-[5-(1,2-dihydroxyéthyl -5-pyrazolidène)-3-(4'-hydroxyphényl)-1-formazano]-1,2-dihydroxyéthyl -pyrazolinium
- Chlorure de 5-[5-(1,2-dihydroxyéthyl -5-pyrazolidène)-3-(4'-N,N-diméthylaminophényl)-1-formazano]-1,2-dihydroxyéthyl -pyrazolinium
- Chlorure de 5-[5-(1,2-dicarboxyéthyl-5-pyrazolidène)-1-formazano]-1,2-dicarboxyéthyl-pyrazolinium
- Chlorure de 5-[5-(1,2-dicarboxyéthyl -5-pyrazolidène)-3-méthyl-1-formazano]-1,2-dicarboxyéthyl -pyrazolinium
- Chlorure de 5-[5-(1,2-dicarboxyéthyl -5-pyrazolidène)-3-éthyl-1-formazano]-1,2-dicarboxyéthyl -pyrazolinium
- Chlorure de 5-[5-(1,2-dicarboxyéthyl -5-pyrazolidène)-3-phényl-1-formazano]-1,2-dicarboxyéthyl -pyrazolinium
- Chlorure de 5-[5-(1,2-dicarboxyéthyl -5-pyrazolidène)-3-isopropyl-1-formazano]-1,2-dicarboxyéthyl -pyrazolinium
- Chlorure de 5-[5-(1,2-dicarboxyéthyl -5-pyrazolidène)-3-(4'-méthoxyphényl)-1-formazano]-1,2-dicarboxyéthyl -pyrazolinium
- Chlorure de 5-[5-(1,2-dicarboxyéthyl -5-pyrazolidène)-3-(4'-hydroxyphényl)-1-formazano]-1,2-dicarboxyéthyl -pyrazolinium
- Chlorure de 5-[5-(1,2-dicarboxyéthyl -5-pyrazolidène)-3-(4'-N,N-diméthylaminophényl)-1-formazano]-1,2-dicarboxyéthyl - pyrazolinium.

Une autre classe de composés de formule (I) avantageux correspond à ceux pour lesquels W₁ est un radical 2-benzimidazolium et W₂ est un radical 2-pyridine.

De préférence, ces composés sont choisis parmi les suivants :
- Chlorure de 2-[5-(N-méthyl-2-pyridinylidène)-1-formazano]-1,3-diméthyl-benzimidazolium
- Chlorure de 2-[5-(N-méthyl-2-pyridinylidène)-3-méthyl-1-formazano]-1,3-diméthyl-benzimidazolium
- Chlorure de 2-[5-(N-méthyl-2-pyridinylidène)-3-éthyl-1-formazano]-1,3-diméthyl-benzimidazolium
- Chlorure de 2-[5-(N-méthyl-2-pyridinylidène)-3-isopropyl-1-formazano]-1,3-diméthyl-benzimidazolium
- Chlorure de 2-[5-(N-méthyl-2-pyridinylidène)-3-phényl-1-formazano]-1,3-diméthyl-benzimidazolium
- Chlorure de 2-[5-(N-méthyl-2-pyridinylidène)-3-(4'-méthoxyphényl)-1-formazano]-1,3-diméthyl-benzimidazolium
- Chlorure de 2-[5-(N-méthyl-2-pyridinylidène)-3-(4'-hydroxyphényl)-1-formazano]-1,3-diméthyl-benzimidazolium
- Chlorure de 2-[5-(N-méthyl-2-pyridinylidène)-3-(4'-N,N-diméthylaminophényl)-1-formazano]-1,3-diméthyl-benzimidazolium.
- Chlorure de 2-[5-(N-hydroxyéthyl-2-pyridinylidène)-1-formazano]-1,3-dihydroxyéthyl benzimidazolium
- Chlorure de 2-[5-(N-hydroxyéthyl-2-pyridinylidène)-3-méthyl-1-formazano]-1,3-dihydroxyéthylbenzimidazolium
- Chlorure de 2-[5-(N-hydroxyethyl-2-pyridinylidène)-3-éthyl-1-formazanol-1,3-dihydroxyéthyl -benzimidazolium
- Chlorure de 2-[5-(N-hydroxyéthyl-2-pyridinylidène)-3-phényl-1-formazano]-1,3-dihydroxyéthyl -benzimidazolium
- Chlorure de 2-[5-(N-hydroxyethyl-2-pyridinylidène)-3-isopropyl-1-formazano]-1,3-dihydroxyéthyl -benzimidazolium
- Chlorure de 2-[5-(N-hydroxyéthyl-2-pyridinylidène)-3-(4'-méthoxyphényl)-1-formazano]-1,3-dihydroxyéthyl -benzimidazolium
- Chlorure de 2-[5-(N-hydroxyéthyl-2-pyridinylidène)-3-(4'-hydroxyphényl)-1-formazano]-1,3-dihydroxyéthyl -benzimidazolium
- Chlorure de 2-[5-(N-hydroxyéthyl-2-pyridinylidène)-3-(4'-N,N-diméthylaminophényl)-1-formazano]-1,3-dihydroxyéthyl -benzimidazolium
- Chlorure de 2-[5-(N-carboxyéthyl-2-pyridinylidène)-1-formazano]-1,3-dicarboxyéthyl-benzimidazolium
- Chlorure de 2-[5-(N-carboxyéthyl-2-pyridinylidène)-3-méthyl-1-formazano]-1,3-dicarboxyéthyl -benzimidazolium
- Chlorure de 2-[5-(N-carboxyéthyl-2-pyridinylidène)-3-éthyl-1-formazano]-1,3-dicarboxyéthyl -benzimidazolium
- Chlorure de 2-[5-(N-carboxyéthyl-2-pyridinylidène)-3-phényl-1-formazano]-1,3-dicarboxyéthyl -benzimidazolium
- Chlorure de 2-[5-(N-carboxyéthyl-2-pyridinylidène)-3-isopropyl-1 -formazanol-1,3-dicarboxyéthyl -benzimidazolium
- Chlorure de 2-[5-(N-carboxyéthyl-2-pyridinylidène)-3-4'-méthoxyphényl)-1-formazano]-1,3-dicarboxyéthyl -benzimidazolium
- Chlorure de 2-[5-(N-carboxyéthyl-2-pyridinylidène)-3-(4'-hydroxyphényl)-1-formazano]-1,3-dicarboxyéthyl -benzimidazolium
- Chlorure de 2-[5-(N-carboxyéthyl-2-pyridinylidène)-3-(4'-N,N-diméthylaminophényl)-1-formazano]-1,3-dicarboxyéthyl -benzimidazolium.

Une autre classe de composés de formule (I) avantageux correspond à ceux pour lesquels W₁ est un radical 2-benzimidazolium et W₂ est un radical 4-pyridine.

De préférence, ces composés sont choisis parmi les suivants :
- Chlorure de 2-[5-(N-methyl-4-pyridinylidène)-1-formazano]-1,3-diméthyl-benzimidazolium
- Chlorure de 2-[5-(N-méthyl-4-pyridinylidène)-3-méthyl-1-formazano]-1,3-diméthyl-benzimidazolium
- Chlorure de 2-[5-(N-méthyl-4-pyridinylidène)-3-éthyl-1-formazano]-1,3-diméthyl-benzimidazolium
- Chlorure de 2-[5-(N-methyl-4-pyridinylidène)-3-isopropyl-1-formazano]-1,3-diméthyl-benzimidazolium
- Chlorure de 2-[5-(N-méthyl-4-pyridinylidène)-3-phényl-1-formazano]-1,3-diméthyl-benzimidazolium
- Chlorure de 2-[5-(N-méthyl-4-pyridinylidène)-3-(4'-méthoxyphényl)-1-formazano]-1,3-diméthyl-benzimidazolium
- Chlorure de 2-[5-(N-méthyl-4-pyridinylidène)-3-(4'-hydroxyphényl)-1-formazano]-1,3-diméthyl-benzimidazolium
- Chlorure de 2-[5-(N-méthyl-4-pyridinylidène)-3-(4'-N,N-diméthylaminophényl)-1-formazano]-1,3-diméthyl-benzimidazolium
- Chlorure de 2-[5-(N-hydroxyéthyl-4-pyridinylidène)-1-formazano]-1,3-dihydroxyéthyl benzimidazolium
- Chlorure de 2-[5-(N-hydroxyethyl-4-pyridinylidène)-3-méthyl-1-formazano]-1,3-dihydroxyéthylbenzimidazolium
- Chlorure de 2-[5-(N-hydroxyéthyl-4-pyridinylidène)-3-éthyl-1-formazano]-1,3-dihydroxyéthyl -benzimidazolium
- Chlorure de 2-[5-(N-hydroxyéthyl-4-pyridinylidène)-3-phényl-1-formazano]-1,3-dihydroxyéthyl -benzimidazolium
- Chlorure de 2-[5-(N-hydroxyéthyl-4-pyridinylidène)-3-isopropyl-1-formazano]-1,3-dihydroxyéthyl -benzimidazolium
- Chlorure de 2-[5-(N-hydroxyéthyl-4-pyridinylidène)-3-(4'-méthoxyphényl)-1-formazano]-1,3-dihydroxyéthyl -benzimidazolium
- Chlorure de 2-[5-(N-hydroxyéthyl-4-pyridinylidène)-3-(4'-hydroxyphényl)-1-formazano]-1,3-dihydroxyéthyl -benzimidazolium
- Chlorure de 2-[5-(N-hydroxyéthyl-4-pyridinylidène)-3-(4'-N,N-diméthylaminophényl)-1-formazano]-1,3-dihydroxyéthyl -benzimidazolium
- Chlorure de 2-[5-(N-carboxyéthyl-4-pyridinylidène)-1-formazano]-1,3-dicarboxyéthyl-benzimidazolium
- Chlorure de 2-[5-(N-carboxyéthyl-4-pyridinylidène)-3-méthyl-1-formazanol-1,3-dicarboxyéthyl -benzimidazolium
- Chlorure de 2-[5-(N-carboxyéthyl-4-pyridinylidène)-3-éthyl-1-formazano]-1,3-dicarboxy éthyl -benzimidazolium
- Chlorure de 2-[5-(N-carboxyéthyl-4-pyridinylidène)-3-phényl-1-formazano]-1,3-dicarboxyéthyl -benzimidazolium
- Chlorure de 2-[5-(N-carboxyéthyl-4-pyridinylidène)-3-isopropyl-1-formazano]-1,3-dicarboxyéthyl -benzimidazolium
- Chlorure de 2-[5-(N-carboxyéthyl-4-pyridinylidène)-3-(4'-méthoxyphényl)-1-formazano]-1,3-dicarboxyéthyl -benzimidazolium
- Chlorure de 2-[5-(N-carboxyéthyl-4-pyridinylidène)-3-(4'-hydroxyphényl)-1-formazano]-1,3-dicarboxyéthyl -benzimidazolium.
- Chlorure de 2-[5-(N-carboxyéthyl-4-pyridinylidène)-3-(4'-N,N-diméthylaminophényl)-1-formazano]-1,3-dicarboxyéthyl -benzimidazolium

Une autre classe de composés de formule (I) avantageux correspond à ceux pour lesquels W₁ est un radical 2-imidazolium et W₂ est un radical 2-pyridine.

De préférence, ces composés sont choisis parmi les composés suivants:
- Chlorure de 2-[5-(N-méthyl-2-pyridinylidène)-1-formazano]-1,3-diméthyl-imidazolium
- Chlorure de 2-[5-(N-méthyl-2-pyridinylidène)-3-méthyl-1-formazano]-1,3-diméthyl-imidazolium
- Chlorure de 2-[5-(N-méthyl-2-pyridinylidène)-3-éthyl-1-formazano]-1,3-diméthyl-imidazolium
- Chlorure de 2-[5-(N-méthyl-2-pyridinylidène)-3-isopropyl-1-formazano]-1,3-diméthyl-imidazolium
- Chlorure de 2-[5-(N-méthyl-2-pyridinylidène)-3-phényl-1-formazano]-1,3-diméthyl-imidazolium
- Chlorure de 2-[5-(N-méthyl-2-pyridinylidène)-3-(4'-méthoxyphényl)-1-formazano]-1,3-diméthyl-imidazolium
- Chlorure de 2-[5-(N-méthyl-2-pyridinylidène)-3-(4'-hydroxyphényl)-1-formazano]-1,3-diméthyl-imidazolium
- Chlorure de 2-[5-(N-méthyl-2-pyridinylidène)-3-(4'-N,N-diméthylaminophényl)-1-formazano]-1,3-diméthyl-imidazolium.
- Chlorure de 2-[5-(N-hydroxyéthyl-2-pyridinylidène)-1-formazano]-1,3-dihydroxyéthyl imidazolium
- Chlorure de 2-[5-(N-hydroxyéthyl-2-pyridinylidène)-3-méthyl-1-formazano]-1,3-dihydroxyéthylimidazolium
- Chlorure de 2-[5-(N-hydroxyéthyl-2-pyridinylidène)-3-éthyl-1-formazanol-1,3-dihydroxyéthyl -imidazolium
- Chlorure de 2-[5-(N-hydroxyéthyl-2-pyridinylidène)-3-phényl-1-formazano]-1,3-dihydroxyéthyl -imidazolium
- Chlorure de 2-[5-(N-hydroxyéthyl-2-pyridinylidène)-3-isopropyl-1-formazano]-1,3-dihydroxyéthyl -imidazolium
- Chlorure de 2-[5-(N-hydroxyéthyl-2-pyridinylidène)-3-(4'-méthoxyphényl)-1-formazano]-1,3-dihydroxyéthyl -imidazolium
- Chlorure de 2-[5-(N-hydroxyéthyl-2-pyridinylidène)-3-(4'-hydroxyphényl)-1-formazano]-1,3-dihydroxyéthyl -imidazolium
- Chlorure de 2-[5-(N-hydroxyéthyl-2-pyridinylidène)-3-(4'-N,N-diméthylaminophényl)-1-formazano]-1,3-dihydroxyéthyl -imidazolium
- Chlorure de 2-[5-(N-carboxyéthyl-2-pyridinylidène)-1-formazano]-1,3-dicarboxyéthyl-imidazolium
- Chlorure de 2-[5-(N-carboxyéthyl-2-pyridinylidène)-3-méthyl-1-formazano]-1,3-dicarboxyéthyl -imidazolium
- Chlorure de 2-[5-(N-carboxyéthyl-2-pyridinylidène)-3-éthyl-1-formazanol-1,3-dicarboxyéthyl -imidazolium
- Chlorure de 2-[5-(N-carboxyéthyl-2-pyridinylidène)-3-phényl-1-formazano]-1,3-dicarboxyéthyl -imidazolium
- Chlorure de 2-[5-(N-carboxyéthyl-2-pyridinylidène)-3-isopropyl-1-formazano]-1,3-dicarboxyéthyl -imidazolium
- Chlorure de 2-[5-(N-carboxyéthyl-2-pyridinylidène)-3-(4'-méthoxyphényl)-1-formazano]-1,3-dicarboxyéthyl -imidazolium
- Chlorure de 2-[5-(N-carboxyéthyl-2-pyridinylidène)-3-(4'-hydroxyphényl)-1-formazano]-1,3-dicarboxyéthyl -imidazolium
- Chlorure de 2-[5-(N-carboxyéthyl-2-pyridinylidène)-3-(4'-N,N-diméthylaminophényl)-1-formazano]-1,3-dicarboxyéthyl -imidazolium

Une autre classe de composés de formule (I) avantageux correspond à ceux pour lesquels W₁ est un radical 2-imidazolium et W₂ est un radical 4-pyridine.

De préférence, ces composés sont choisis parmi les composés suivants:
- Chlorure de 2-[5-(N-méthyl-4-pyridinylidène)-1-formazano]-1,3-diméthyl-imidazolium
- Chlorure de 2-[5-(N-méthyl-4-pyridinylidène)-3-méthyl-1-formazano]-1,3-diméthyl-imidazolium
- Chlorure de 2-[5-(N-méthyl-4-pyridinylidène)-3-éthyl-1-formazano]-1,3-diméthyl-imidazolium
- Chlorure de 2-[5-(N-méthyl-4-pyridinylidène)-3-isopropyl-1-formazano]-1,3-diméthyl-imidazolium
- Chlorure de 2-[5-(N-méthyl-4-pyridinylidène)-3-phényl-1-formazano]-1,3-diméthyl-imidazolium
- Chlorure de 2-[5-(N-méthyl-4-pyridinylidène)-3-(4'-méthoxyphényl)-1-formazano]-1,3-diméthyl-imidazolium
- Chlorure de 2-[5-(N-méthyl-4-pyridinylidène)-3-(4'-hydroxyphényl)-1-formazano]-1,3-diméthyl-imidazolium
- Chlorure de 2-[5-(N-méthyl-4-pyridinylidène)-3-(4'-N,N-diméthylaminophényl)-1-formazano]-1,3-diméthyl-imidazolium
- Chlorure de 2-[5-(N-hydroxyéthyl-4-pyridinylidène)-1-formazano]-1,3-dihydroxyéthyl imidazolium
- Chlorure de 2-[5-(N-hydroxyéthyl-4-pyridinylidène)-3-méthyl-1-formazano]-1,3-dihydroxyéthylimidazolium
- Chlorure de 2-[5-(N-hydroxyeéhyl-4-pyridinylidène)-3-éthyl-1-formazano]-1,3-dihydroxyéthyl -imidazolium
- Chlorure de 2-[5-(N-hydroxyéthyl-4-pyridinylidène)-3-phényl-1-formazano]-1,3-dihydroxyéthyl -imidazolium
- Chlorure de 2-[5-(N-hydroxyéthyl-4-pyridinylidène)-3-isopropyl-1-formazano]-1,3-dihydroxyéthyl -imidazolium
- Chlorure de 2-[5-(N-hydroxyéthyl-4-pyridinylidène)-3-(4'-méthoxyphényl)-1-formazano]-1,3-dihydroxyéthyl -imidazolium
- Chlorure de 2-[5-(N-hydroxyéthyl-4-pyridinylidène)-3-(4'-hydroxyphényl)-1-formazano]-1,3-dihydroxyéthyl -imidazolium
- Chlorure de 2-[5-(N-hydroxyéthyl-4-pyridinylidène)-3-(4'-N,N-diméthylaminophényl)-1-formazano]-1,3-dihydroxyéthyl -imidazolium
- Chlorure de 2-[5-(N-carboxyéthyl-4-pyridinylidène)-1-formazano]-1,3-dicarboxyéthyl-imidazolium
- Chlorure de 2-[5-(N-carboxyéthyl-4-pyridinylidène)-3-méthyl-1-formazano]-1,3-dicarboxyéthyl -imidazolium
- Chlorure de 2-[5-(N-carboxyéthyl-4-pyridinylidène)-3-éthyl-1-formazanol-1,3-dicarboxyéthyl -imidazolium
- Chlorure de 2-[5-(N-carboxyéthyl-4-pyridinylidène)-3-phényl-1-formazano]-1,3-dicarboxyéthyl -imidazolium
- Chlorure de 2-[5-(N-carboxyéthyl-4-pyridinylidène)-3-isopropyl-1-formazano]-1,3-dicarboxyéthyl -imidazolium
- Chlorure de 2-[5-(N-carboxyéthyl-4-pyridinylidène)-3-(4'-méthoxyphényl)-1-formazano]-1,3-dicarboxyéthyl -imidazolium
- Chlorure de 2-[5-(N-carboxyéthyl-4-pyridinylidène)-3-(4'-hydroxyphényl)-1-formazano]-1,3-dicarboxyéthyl -imidazolium
- Chlorure de 2-[5-(N-carboxyéthyl-4-pyridinylidène)-3-(4'-N,N-diméthylaminophényl)-1-formazano]-1,3-dicarboxyéthyl-imidazolium.

Une autre classe de composés de formule (I) avantageux correspond à ceux pour lesquels W₁ est un radical 2-benzimidazolium et W₂ est un radical 2-benzimidazole.

De préférence, ces composés sont choisis parmi les suivants :
- Chlorure de 2-[5-(1,3-diméthyl-2-benzimidazolidène)-1-formazano]-1,3-diméthyl-benzimidazolium
- Chlorure de 2-[5-(1,3-diméthyl-2-benzimidazolidène)-3-méthyl-1-formazano]-1,3-diméthyl-benzimidazolium
- Chlorure de 2-[5-(1,3-diméthyl-2-benzimidazolidène)-3-éthyl-1-formazano]-1,3-diméthyl-benzimidazolium
- Chlorure de 2-[5-(1,3-diméthyl-2-benzimidazolidène)-3-isopropyl-1-formazano]-1,3-diméthyl-benzimidazolium
- Chlorure de 2-[5-(1,3-diméthyl-2-benzimidazolidene)-3-phényl-1-formazano]-1,3-diméthyl-benzimidazolium
- Chlorure de 2-[5-(1,3-diméthyl-2-benzimidazolidène)-3-(4'-méthoxyphényl)-1-formazano]-1,3-diméthyl-benzimidazolium
- Chlorure de 2-[5-(1,3-diméthyl-2-benzimidazolidène)-3-(4'-hydroxyphényl)-1-formazano]-1,3-diméthyl-benzimidazolium
- Chlorure de 2-[5-(1,3-diméthyl-2-benzimidazolidène)-3-(4'-N,N-dimethylaminophényl)-1-formazano]-1,3-diméthyl-benzimidazotium
- Chlorure de 2-[5-(1,3-dihydroxyéthyl-2-benzimidazolidène)-1-formazano]-1,3-dihydroxyéthylbenzimidazolium
- Chlorure de 2-[5-(1,3-dihydroxyéthyl-2-benzimidazolidène)-3-méthyl-1-formazano]-1,3-dihydroxyéthylbenzimidazolium
- Chlorure de 2-[5-(1,3-dihydroxyéthyl-2-benzimidazolidène)-3-ethyl-1-formazano]-1,3-dihydroxyéthyl -benzimidazolium
- Chlorure de 2-[5-(1,3-dihydroxyéthyl -2-benzimidazolidène)-3-phenyl-1-formazano]-1,3-dihydroxyéthyl -benzimidazolium
- Chlorure de 2-[5-(1,3-dihydroxyéthyl -2-benzimidazolidène)-3-isopropyl-1-formazano]-1,3-dihydroxyéthyl -benzimidazolium
- Chlorure de 2-[5-(1,3-dihydroxyéthyl-2-benzimidazolidène)-3-(4'-methoxyphényl)-1-formazano]-1,3-dihydroxyéthyl -benzimidazolium
- Chlorure de 2-[5-(1,3-dihydroxyéthyl -2-benzimidazolidène)-3-(4'-hydroxyphényl)-1-formazano]-1,3-dihydroxyéthyl -benzimidazolium
- Chlorure de 2-[5-(1,3-dihydroxyéthyl -2-benzimidazolidène)-3-(4'-N,N-dimethylaminophenyl)-1-formazano]-1,3-dihydroxyéthyl -benzimidazolium
- Chlorure de 2-[5-(1,3-dicarboxyéthyl-2-benzimidazolidène)-1-formazano]-1,3-dicarboxyéthyl -benzimidazolium
- Chlorure de 2-[5-(1,3-dicarboxyéthyl -2-benzimidazolidène)-3-méthyl-1-formazano]-1,3-dicarboxyéthyl -benzimidazolium
- Chlorure de 2-[5-(1,3-dicarboxyéthyl -2-benzimidazolidène)-3-ethyl-1-formazano]-1,3-dicarboxyéthyl -benzimidazolium
- Chlorure de 2-[5-(1,3-dicarboxyéthyl -2-benzimidazolidène)-3-phenyl-1-formazano]-1,3-dicarboxyéthyl -benzimidazolium
- Chlorure de 2-[5-(1,3-dicarboxyéthyl -2-benzimidazolidène)-3-isopropyl-1-formazano]-1,3-dicarboxyéthyl -benzimidazolium
- Chlorure de 2-[5-(1,3-dicarboxyéthyl -2-benzimidazolidène)-3-(4'-methoxyphényl)-1-formazano]-1,3-dicarboxyéthyl -benzimidazolium
- Chlorure de 2-[5-(1,3-dicarboxyéthyl -2-benzimidazolidène)-3-(4'-hydroxyphényl)-1-formazano]-1,3-dicarboxyéthyl -benzimidazolium
- Chlorure de 2-[5-(1,3-dicarboxyéthyl -2-benzimidazolidène)-3-(4'-N,N-diméthylamino phényl)-1-formazano]-1,3-dicarboxyéthyl - benzimidazolium.

Comme indiqué auparavant, le procédé de synthèse des composés qui viennent d'être décrits consiste donc à faire réagir au moins un composé azine de formule (VI) et/ou (VII) : 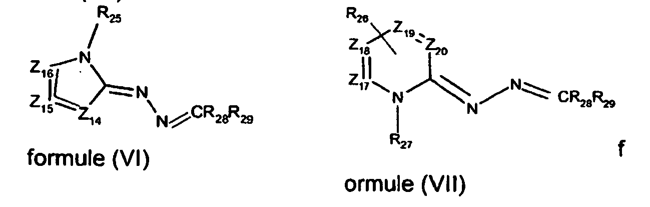 dans lesquelles :
- Z₁₄, Z₁₅, Z₁₆, R₂₅ ont respectivement les mêmes significations que Z₇, Z₈, Z₁₃, R₅ de la formule (IV), ou que Z₁, Z₂, Z₀, R₁ de la formule (II), définies ci-dessus,
- Z₁₇, Z₁₈, Z₁₉, Z₂₀, R₂₆, R₂₇ ont respectivement les mêmes significations que Z₉, Z₁₀, Z₁₁, Z₁₂, R₈, R₇ de la formule (V), ou que Z₃, Z₄, Z₅, Z₆, R₃, R₄ de la formule (III), définies ci-dessus,
- R₂₈, R₂₉, indépendamment l'un de l'autre, représente un atome d'hydrogène, un radical alkyle en C₁-C₆, de préférence en C₁-C₄, un radical alcoxy, linéaire ou ramifié, en C₁-C₆, de préférence en C₁-C₄, à la condition que l'un au moins des deux radicaux soit différent de l'hydrogène.

Tout ce qui a été indiqué auparavant quant à la nature des radicaux Z₁ à Z₁₆, R₁, R₃, R₄, R₅, R₇ et R₈ reste valable dans cette partie de la description et l'on pourra s'y reporter.

De préférence, R₂₈, R₂₉, identiques ou non, représentent de préférence un atome d'hydrogène, un radical méthyle, un radical méthoxy.

Selon une première variante du procédé de l'invention, l'agent oxydant mis en oeuvre est choisi parmi le peroxyde d'hydrogène ; le peroxyde d'urée ; les peracides organiques tels que l'acide peracétique ; les persels tels que permanganate, perborate; les persulfates ; les chromates ou les bichromates ; les hypochlorites ; les hypobromites ; les ferricyanures ; les bioxydes de manganèse ou de plomb, seuls ou en mélanges.

De préférence, l'agent oxydant est le peroxyde d'hydrogène.

Selon une deuxième variante de l'invention, le procédé est mis en oeuvre en utilisant, en tant qu'agent oxydant, des enzymes, en présence d'un substrat pour ladite enzyme.

Selon un premier mode de réalisation, l'enzyme est choisie parmi les oxydases produisant du peroxyde d'hydrogène, en présence d'un substrat approprié pour ladite enzyme.

Parmi les enzymes de ce type, on peut citer, sans intention de s'y limiter les enzymes suivantes :

| | |
|---|---|
| PYRANOSE OXIDASE | EC 1.1.3.10 |
| L-SORBOSE OXIDASE | EC 1.1.3.11 |
| PYRIDOXINE 4 - OXIDASE | EC 1.1.3.12 |
| ALCOHOL OXIDASE | EC 1.1.3.13 |
| CATECHOL OXIDASE (DIMERIZING). | EC 1.1.3.14 |
| (S)-2-HYDROXY-ACID OXIDASE. | EC 1.1.3.15 |
| ECDYSONE OXIDASE | EC 1.1.3.16 |
| CHOLINE OXIDASE | EC 1.1.3.17 |
| SEC - ALCOHOL OXIDASE | EC 1.1.3.18 |
| 4 - HYDROXYMANDALATE OXIDASE | EC 1.1.3.19 |
| LONG-CHAIN-ALCOHOL OXIDASE. | EC 1.1.3.20 |
| GLYCEROL-3-PHOSPHATE OXIDASE. | EC 1.1.3.21 |
| XANTHINE OXIDASE | EC 1.1.3.22 |
| THIAMIN OXIDASE | EC 1.1.3.23 |
| L - GALACTONOLACTONE OXIDASE | EC 1.1.3.24 |
| CELLOBIOSE OXIDASE | EC 1.1.3.25 |
| HYDROXYPHYTANATE OXIDASE | EC 1.1.3.27 |
| NUCLEOSIDE OXIDASE. | EC 1.1.3.28 |
| N-ACYLHEXOSAMINE OXIDASE | EC 1.1.3.29 |
| MALATE OXIDASE | EC 1.1.3.3 |
| POLYVINYL-ALCOHOL OXIDASE. | EC 1.1.3.30 |
| METHANOL OXIDASE. | EC 1.1.3.31 |
| D-ARABINONO-1,4-LACTONE OXIDASE. | EC 1.1.3.37 |
| VANILLYL-ALCOHOL OXIDASE. | EC 1.1.3.38 |
| NUCLEOSIDE OXIDASE (H(2)O(2)-FORMING). | EC 1.1.3.39 |
| GLUCOSE OXIDASE | EC 1.1.3.4 |
| D-MANNITOL OXIDASE. | EC 1.1.3.40 |
| XYLITOL OXIDASE. | EC 1.1.3.41 |
| HEXOSE OXIDASE | EC 1.1.3.5 |
| CHOLESTROL OXIDASE | EC 1.1.3.6 |
| ARYL ALCOHOL AOXIDASE | EC 1.1.3.7 |
| L-GULONOLACTONE OXIDASE. | EC 1.1.3.8 |
| GALACTOSE OXIDASE | EC 1.1.3.9 |
| PTERIDINE OXIDASE | EC 1.17.3.1 |
| RETINAL OXIDASE | EC 1.2.3.11 |
| ALDEHYDE OXIDASE. | EC 1.2.3.1 |
| CARBON MONOXIDE OXIDASE | EC 1.2.3.10 |
| VANILLATE DEMETHYLASE | EC 1.2.3.12 |
| 4-HYDROXYPHENYLPYRUVATE OXIDASE. | EC 1.2.3.13 |
| PYRUVATE OXIDASE | EC 1.2.3.3 |
| OXALATE OXIDASE | EC 1.2.3.4 |
| GLYOXALATE OXIDASE | EC 1.2.3.5 |
| PYRUVATE OXIDASE (CO-A ACETYLATING) | EC 1.2.3.6 |
| INDOLE-3-ACETALDEHYDE OXIDASE. | EC 1.2.3.7 |
| PYRIDOXAL OXIDASE. | EC 1.2.3.8 |
| ARYL ALDEHYDE OXIDASE | EC 1.2.3.9 |
| DUHYDROOROTATE OXIDASE | EC 1.3.3.1 |
| LATHOSTEROL OXIDASE | EC 1.3.3.2 |
| COPROPORPHYRINOGEN OXIDASE. | EC 1.3.3.3 |
| PROTOPORPHYRINOGEN OXIDASE. | EC 1.3.3.4 |
| BILIRUBIN OXIDASE. | EC 1.3.3.5 |
| ACYL CO-A OXIDASE | EC 1.3.3.6 |
| DIHYDROURACIL OXIDASE | EC 1.3.3.7 |
| TETRAHYDROBERBERINE OXIDASE | EC 1.3.3.8 |
| SECOLOGANIN SYNTHASE. | EC 1.3.3.9 |
| ASPARTATE OXIDASE | EC 1.4.3.1 |
| PUTRESCINE OXIDASE | EC 1.4.3.10 |
| L-GLUTAMATE OXIDASE | EC 1.4.3.11 |
| CYCLOHEXYLAMINE OXIDASE. | EC 1.4.3.12 |
| PROTEIN-LYSINE 6-OXIDASE | EC 1.4.3.13 |
| L-LYSINE OXIDASE | EC 1.4.3.14 |
| D-GLUTAMATE (D-ASPARTATE) OXIDASE | EC 1.4.3.15 |
| L-ASPARTATE OXIDASE | EC 1.4.3.16 |
| TRYPTOPHAN ALPHA,BETA-OXIDASE | EC 1.4.3.17 |
| GLYCINE OXIDASE. | EC 1.4.3.19 |
| L-AMINO ACID OXIDASE | EC 1.4.3.2 |
| D-AMINO ACID OXIDASE | EC 1.4.3.3 |
| AMINE OXIDASE (FLAVIN CONTAINING) | EC 1.4.3.4 |
| PYRIDOXAMINE PHOSPHATE OXIDASE | EC 1.4.3.5 |
| AMINE OXIDASE (COPPER CONTAINING) | EC 1.4.3.6 |
| D-GLUTAMATE OXIDASE | EC 1.4.3.7 |
| ETHANOLAMINE OXIDASE | EC 1.4.3.8 |
| SARCOSINE OXIDASE | EC 1.5.3.1 |
| DIMETHYLGLYCINE OXIDASE | EC 1.5.3.10 |
| POLYAMINE OXIDASE | EC 1.5.3.11 |
| DIHYDROBENZOPHENANTHRIDINE OXIDASE. | EC 1.5.3.12 |
| N-METHY-L-L-AMINO ACID OXIDASE | EC 1.5.3.2 |
| N-METHYL-LYSINE OXIDASE | EC 1.5.3.4 |
| (S)-6-HYDROXYNICOTINE OXIDASE | EC 1.5.3.5 |
| (R)-6-HYDROXYNICOTINE OXIDASE. | EC 1.5.3.6 |
| L-PIPECOLATE OXIDASE | EC 1.5.3.7 |
| RETICULATE OXIDASE | EC 1.5.3.9 |
| NITROETHANE OXIDASE | EC 1.7.3.1 |
| ACETYLINDOXYL OXIDASE. | EC 1.7.3.2 |
| URATE OXIDASE | EC 1.7.3.3 |
| HYDROXYLAMINE OXIDASE. | EC 1.7.3.4 |
| 3-ACYL-NITROPROPANOATE OXIDASE. | EC 1.7.3.5 |
| SULFITE OXIDASE | EC 1.8.3.1 |
| THIOL OXIDASE | EC 1.8.3.2 |
| GLUTATHIONE OXIDASE. | EC 1.8.3.3 |
| METHANETHIOL OXIDASE | EC 1.8.3.4 |
| PRENYLCYSTEINE OXIDASE. | EC 1.8.3.5 |

Selon un deuxième mode de réalisation, l'enzyme est choisie parmi les enzymes oxydoreductases à 4 électrons.

A titre d'exemples de telles enzymes, on peut notamment citer les enzymes du groupe EC 1.10.3 comme Catechol oxydase (polyphénol oxydase) EC 1.10.3.1, Laccase EC 1.10.3.2, L ascorbate oxydase EC 1.10.3.3 et Aminophenol oxydase EC 1.10.3.4., tyrosinase (monophenol oxydase) EC1.10.3.1 ou 1.14.18.1

Selon un deuxième mode de réalisation, l'enzyme est choisie parmi les peroxydases.

Parmi les enzymes utilisables, on peut mentionner, sans intention de s'y limiter:

| | |
|---|---|
| NADH peroxidase. | E.C 1.11.1.1 |
| NADPH peroxidase. | E.C 1.11.1.2 |
| Fatty acid peroxidase. | E.C 1.11.1.3 |
| Cytochrome-c peroxidase. | E.C 1.11.1.5 |
| Peroxidase. | E.C 1.11.1.7 |
| lodide peroxidase. | E.C 1.11.1.8 |
| Glutathione peroxidase. | E.C 1.11.1.9 |
| Chloride peroxidase. | E.C 1.11.1.10 |
| L-ascorbate peroxidase. | E.C 1.11.1.11 |
| Phospholipid-hydroperoxide glutathione peroxidase. | E.C 1.11.1.12 |
| Manganese peroxidase. | E.C 1.11.1.13 |
| Diarylpropane peroxidase. | E.C 1.11.1.14 |
| Tryptophan 2,3-dioxygenase. | E.C 1.13.11.11 |
| Lipoxygenase. | E.C 1.13.11.12 |

Selon les réactifs utilisés, la réaction est conduite avec ou sans système oxydant, avec ou sans cofacteur pour l'enzyme, avec ou sans système de régénération du cofacteur.

A noter que par les termes "sans système oxydant", on entend qu'aucun système oxydant autre que l'oxygène atmosphérique n'est utilisé pour la réaction.

De préférence, la réaction est effectuée en milieu aérobie à pH compris entre 3 et 11 et à température comprise entre 6°C et 80°C.

La concentration en substrat de l'enzyme, que ce dernier soit ou non différent de l'azine, est habituellement comprise entre 0,001 M et 3 M de préférence entre 0,1 M et 1 M.

La teneur en cofacteur pour les dites enzymes peut être comprise entre 0,01 mM et 1 M de préférence 0,1 mM et 10mM.

La réaction peut être réalisée entre pH 3 et pH 11 de préférence entre pH 5 et pH 9.5.

Habituellement la réaction est effectuée en milieu aqueux et/ou en présence d'un solvant protique, de préférence des alcools, les polyols, les éthers de polyols.

De préférence, le pH est ajusté, voire maintenu constant si nécessaire, au moyen d'agent alcalinisant, acidifiant, et encore plus avantageusement à l'aide d'un tampon classique.

La température pour la réaction peut être comprise entre 10°C et 80°C de préférence entre 20°C et 65°C.

Selon un mode de réalisation de l'invention, lors de la mise en oeuvre de la synthèse des composés de formules (I), les réactifs, à savoir la ou les azine de formule (VI) et/ou (VII) ; le système enzymatique (la ou les enzymes, leur substrat, leur cofacteur, l'oxydant et/ou le cas échéant, le système de régénération du cofacteur) ; l'oxydant, sont mélangés, le pH et la température sont ajustés.

Il n'est bien sûr pas exclu de mettre en oeuvre le procédé de l'invention en continu.

Un deuxième objet de la présente demande consiste en un procédé de coloration de fibres kératiniques, notamment humaines, mettant en oeuvre les étapes suivantes :
a) on applique sur les fibres sèches ou humides, une composition tinctoriale comprenant au moins une azine de formule (VI) et/ou (VII) détaillées précédemment, en présence d'un agent oxydant ;
b) on laisse poser pendant une durée suffisante pour développer la coloration désirée.

L'agent oxydant peut être soit mélangé au moment de l'emploi à la composition tinctoriale.

Il peut aussi être appliqué sur les fibres, simultanément ou séquentiellement à la composition tinctoriale.

De préférence, la teneur en composé(s) de formule (VI) et/ou (VII) est telle que la composition comprend une teneur en composé(s) de formule (I) comprise entre 0,001 à 10% en poids, de préférence de 0,05 à 5% en poids, par rapport au poids total de la composition tinctoriale.

Avantageusement, la teneur dans la composition tinctoriale en composé(s) de formule (VI) et/ou (VII) est comprise entre 0,001 et 10 % en poids, de préférence entre 0,05 et 5 % en poids, par rapport au poids total de la composition tinctoriale.

La composition tinctoriale mise en oeuvre dans le procédé selon l'invention peut en outre comprendre au moins un colorant direct additionnel.

De préférence, il est choisi parmi des composés différents du ou des composés de formule (I) qui sont synthétisés durant la réaction (soit par la famille chimique, soit par l'un au moins des substituants si la famille chimique est la même).

On peut utiliser tout type de colorant direct utilisé classiquement dans le domaine de la coloration capillaire. A titre d'exemples, on peut citer les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs triarylméthaniques, les colorants indoaminiques et les colorants directs naturels.

Le ou les colorants directs additionnels, lorsqu'ils sont présents, représentent plus particulièrement de 0,001 à 20% en poids par rapport au poids total de la composition tinctoriale, et de préférence de 0,005 à 10% en poids par rapport au poids total de la composition tinctoriale.

La composition peut en outre comprendre au moins une base d'oxydation, éventuellement associée à au moins un coupleur.

Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

Lorsque la composition tinctoriale comprend une ou plusieurs bases d'oxydation, leur teneur représente plus particulièrement de 0,001 à 10% en poids par rapport au poids total de la composition tinctoriale, et de préférence de 0,005 à 6% en poids par rapport au poids total de la composition tinctoriale.

Parmi les coupleurs utilisables, on peut notamment citer les métaphénylènediamines, les métaaminophénols, les métadiphénols, les coupleurs naphtaléniques et les coupleurs hétérocycliques.

Dans le cas où la composition tinctoriale comprend de tels composés, leur teneur représente avantageusement de 0,001 à 10% en poids par rapport au poids total de la composition tinctoriale et de préférence de 0,005 à 6% en poids par rapport au poids total de la composition tinctoriale.

D'une manière générale, les colorants directs, les bases d'oxydation et les coupleurs peuvent être utilisés sous la forme d'un sel d'addition, notamment avec un acide. Ces acides sont habituellement choisis parmi les chlorhydrates, les bromhydrates, les sulfates, les citrates, les succinates, les tartrates, les lactates, les tosylates, les benzènesulfonates, les phosphates et les acétates.

Le milieu de la composition tinctoriale, appelé aussi support, est généralement constitué par de l'eau ou par un mélange d'eau et d'au moins un solvant organique pour solubiliser les composés qui ne seraient pas suffisamment solubles dans l'eau.

A titre de solvant organique, on peut par exemple citer les alcools en C₁-C₄ tels que l'éthanol et l'isopropanol, les polyols et éthers de polyols comme le 2-butoxyéthanol, le propylèneglycol, le monométhyléther de propylèneglycol, le monoéthyléther et le monométhyléther de diéthylèneglycol, ainsi que les alcools aromatiques comme l'alcool benzylique ou le phénoxyéthanol, et leurs mélanges.

Les solvants organiques peuvent être présents dans des proportions de préférence comprises entre 1 et 40% en poids environ par rapport au poids total de la composition tinctoriale, et encore plus préférentiellement entre 5 et 30% en poids environ par rapport au poids total de la composition tinctoriale.

La composition tinctoriale selon l'invention peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les polymères associatifs anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants cités ci-dessus sont en général présents en quantité comprise pour chacun d'eux entre 0,01 et 20% en poids par rapport au poids total de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale appliquée durant le procédé selon l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium et de potassium et les composés de formule (III) suivante : 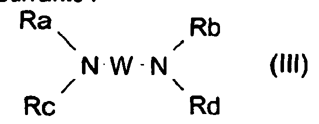 dans laquelle W est un reste propylène éventuellement substitué par un groupement hydroxyle ou un radical alkyle en C₁-C₄, Rₐ, R_{b}, R_{c} et R_{d}, identiques ou différents, représentent un atome d'hydrogène, un radical alkyle en C₁-C₄ ou hydroxyalkyle en C₁-C₄.

Parmi les systèmes tampons classiques volatils ou non, on peut citer par exemple le triéthylammonium acétate, le triéthylammonium formiate, les tampons phosphates sans toutefois s'y limiter.

De plus, la composition tinctoriale peut se présenter sous des formes diverses, telles que sous forme de liquides, de crèmes, de gels ou sous toute autre forme appropriée pour réaliser une coloration des fibres kératiniques, notamment humaines, et de préférence les cheveux.

La composition oxydante, qu'elle soit mélangée à la composition tinctoriale avant l'application ou non, peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants, alcalinisants ou tampons, habituellement utilisés en coloration des fibres kératiniques et tels que définis précédemment.

Le temps de pose lors de l'étape b) dépend de plusieurs critères, dont, entre autres, la température d'application et de pose de la composition tinctoriale.

Cependant, à titre d'exemple, les durées sont en général comprises entre 3 et 50 minutes environ, de préférence entre 5 et 30 minutes environ.

La température à laquelle la composition est appliquée puis laissée poser, est habituellement comprise entre la température ambiante et 80°C.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La réaction est arrêtée lorsque la couleur recherchée est atteinte. En général, le temps de pause est compris entre 1 à 60 minutes, et de préférence entre 5 et 45 minutes

Avantageusement, une fois l'étape b) réalisée, on rince éventuellement les fibres, de préférence on les lave avec un shampooing et on les rince, puis on les sèche ou on les laisse sécher.

Des exemples non limitatifs de mise en oeuvre de la présente invention vont maintenant être présentés.

### EXEMPLES DE SYNTHESE

### Exemple 1

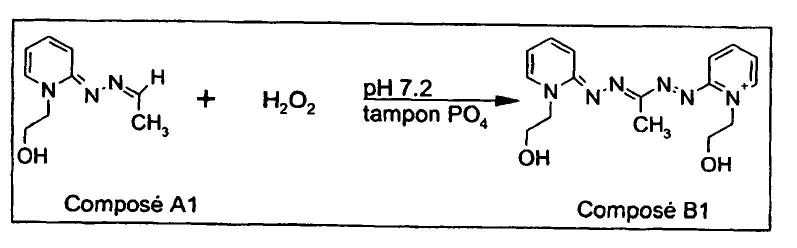

On dissout 0,58g d'azine (composé A1) dans 12,5ml de tampon phosphate (0,2M-pH 7), puis on ajuste le pH à 7,2 avec une solution d'hydroxyde de sodium à 1 M.

On ajoute 10,3ml de peroxyde d'hydrogène (solution à 5 volumes).

La réaction a lieu à température ambiante.

La synthèse est arrêtée après 24 heures de réaction.

L'analyse par spectrométrie de masse en mode d'ionisation electrospray positif (ESI+) du milieu de synthèse permet de détecter l'ion pseudomoléculaire M+= 329. Cet ion correspond au composé (B1) formé.

Le composé (B1) synthétisé est analysé par LC/UV. Il est détecté par son spectre UV/visible à son maximum d'absorption λₘₐₓ = 578nm.

L'analyse par RMN proton ¹H du composé B1, effectuée dans le méthanol deutérié (CD₃OD), est conforme au produit attendu.

### Exemple 2

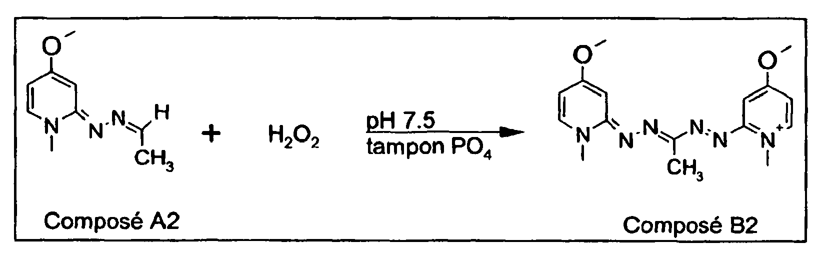

On dissout 0,1g azine (composé A2) dans 5ml d'un tampon phosphate (0,2M - pH 7) et on ajuste le pH à 7,5 avec une solution d'hydroxyde de sodium à 1 M.

On ajoute 0,56 ml de peroxyde d'hydrogène (solution à 20 volumes).

La réaction a lieu à température ambiante.

La synthèse est arrêtée après une heure de réaction.

L'analyse par spectrométrie de masse en mode d'ionisation electrospray positif (ESI+) du milieu de synthèse permet de détecter l'ion pseudomoléculaire M+= 329. Il correspond au composé B2 formé.

Le composé B2 synthétisé est analysé par LC/UV. Il est détecté par son spectre UV/visible à son maximum d'absorption λₘₐₓ = 564nm.

L'analyse par RMN proton ¹H du composé B2, effectué dans le méthanol deutérié (CD₃OD), est conforme au produit attendu.

### Exemple 3

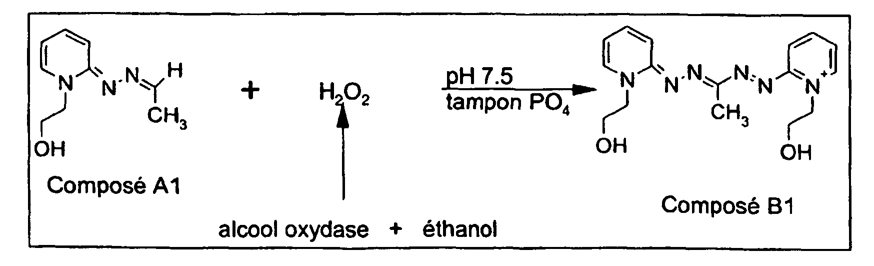

On dissout 25mg de l'azine (composé A1) dans 5ml de tampon phosphate (0,2M-pH 7) et l'on ajuste le pH à 7,5 avec de l'hydroxyde de sodium à 1M.

On ajoute alors 125µl d'éthanol absolu, puis 60 U d'enzyme alcool oxydase, et l'on aère la solution par bullage d'air.

La réaction a lieu à température ambiante.

La synthèse est arrêtée après 24 heures de réaction.

L'analyse par spectrométrie de masse en mode d'ionisation electrospray positif (ESI+) du milieu de synthèse permet de détecter l'ion pseudomoléculaire M+= 329. Il correspond au composé (B1) formé.

Le composé (B1) synthétisé est analysé par LC/UV. Il est détecté par son spectre UV/visible à son maximum d'absorption λₘₐₓ = 578nm.

### Exemple 4

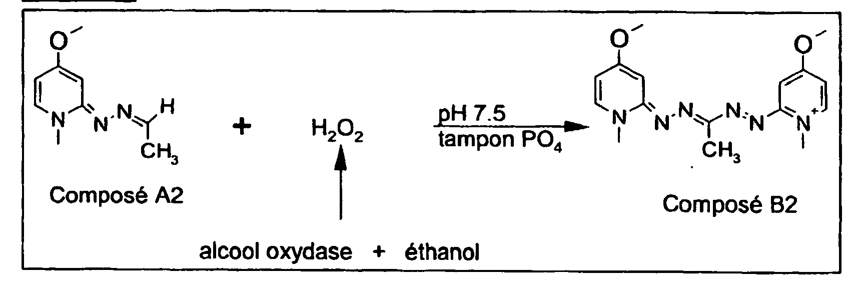

On dissout 50 mg de l'azine (Produit A2) dans 2,5ml de tampon phosphate (0,2M-pH 7) puis on ajuste le pH à 7,5 avec de l'hydroxyde de sodium à 1 M.

On ajoute alors 250µl d'éthanol absolu, puis 120 U d'enzyme alcool oxydase, et l'on aère la solution par bullage d'air.

La réaction a lieu à température ambiante.

La synthèse est arrêtée après 1 heure de réaction.

L'analyse par spectrométrie de masse en mode d'ionisation electrospray positif (ESI+) du milieu de synthèse permet de détecter l'ion pseudomoléculaire M+= 329. Il correspond au composé B2 formé.

Le composé B2 synthétisé est analysé par LC/UV. Il est détecté par son spectre UV/visible à son maximum d'absorption λₘₐₓ = 564nm.

### Exemple 5

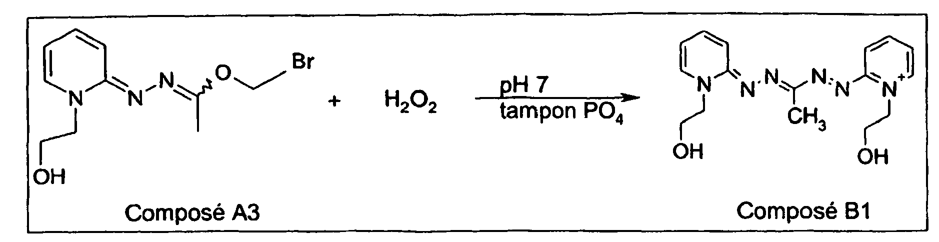

On dissout 10 mg d'azine (composé A3) dans 2ml de tampon phosphate (0,2M-pH 7).

On ajoute alors 100µl de peroxyde d'hydrogène à 20 volumes.

La réaction se développe à température ambiante.

Après 1 heure de réaction la solution est bleue.

L'analyse par spectrométrie de masse en mode d'ionisation electrospray positif (ESI+) du milieu réactionnel permet de détecter l'ion pseudomoléculaire M+= 329. Il correspond au composé (B1) formé.

Le composé (B1) synthétisé est analysé par LC/UV. Il est détecté par son spectre UV/visible à son maximum d'absorption λₘₐₓ = 578nm.

### Exemple 6

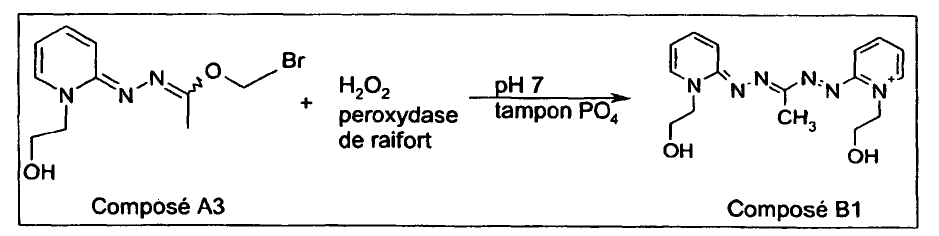

On dissout 10mg d'azine (composé A3) dans 2ml de tampon phosphate (0,2M-pH 7).

On ajoute alors 100µl de peroxyde d'hydrogène à 20 volumes, puis 30 U de peroxydase de raifort.

La réaction se développe à température ambiante.

Apres 15 minutes de réaction la solution est bleue.

L'analyse par spectrométrie de masse en mode d'ionisation electrospray positif (ESI+) du milieu réactionnel permet de détecter l'ion pseudomoléculaire M+= 329. Il correspond au composé (B1) formé.

Le composé (B1) synthétisé est analysé par LC/UV. Il est détecté par son spectre UV/visible à son maximum d'absorption λₘₐₓ = 578nm.

### Exemple 7

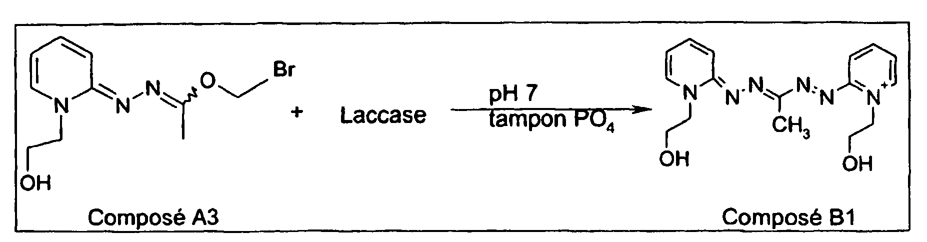

On dissout 10mg d'azine (composé A3) dans 2ml de tampon phosphate (0,2M-pH7).

On ajoute alors 50 U de laccase.

La réaction se développe à température ambiante.

Après 30 minutes de réaction la solution est bleue.

L'analyse par spectrométrie de masse en mode d'ionisation electrospray positif (ESI+) du milieu réactionnel permet de détecter l'ion pseudomoléculaire M+= 329. Il correspond au composé B1 formé.

Le composé B1 synthétisé est analysé par LC/UV. Il est détecté par son spectre UV/visible à son maximum d'absorption λₘₐₓ = 578nm.

### Exemple 8

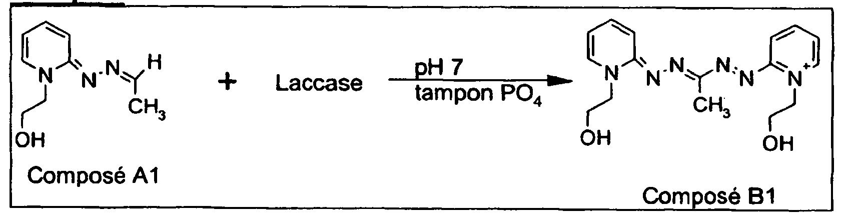

On dissout 10mg d'azine (composé A1) dans 2ml de tampon phosphate (0,2M-pH 7).

On ajoute 50 U de laccase.

La réaction se développe à température ambiante.

Après 30 minutes de réaction la solution est bleue.

L'analyse par spectrométrie de masse en mode d'ionisation electrospray positif (ESI+)du milieu réactionnel permet de détecter l'ion pseudomoléculaire M+= 329. Il correspond au composé B1 formé.

Le composé B1 synthétisé est analysé par LC/UV. Il est détecté par son spectre UV/visible à son maximum d'absorption λₘₐₓ = 578nm

### EXEMPLES D'APPLICATION SUR MÈCHES DE CHEVEUX

### Exemple 9

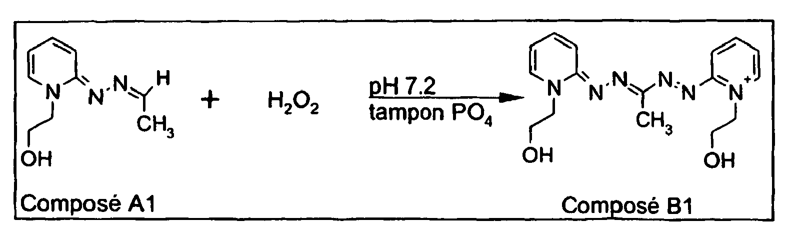

On dissout 10 mg d'azine (produit A1) dans 5ml de tampon phosphate (0,2M - pH 7,2).

On ajoute 0,57 ml de peroxyde d'hydrogène à 5 volumes.

Ce mélange est appliqué sur une mèche de 0,5g de cheveux à 90% blanc.

Après 30 minutes de contact la mèche de cheveux est rincée, shampouinée rincée et séchée.

La mèche obtenue est colorée en bleue.

### Exemple 10

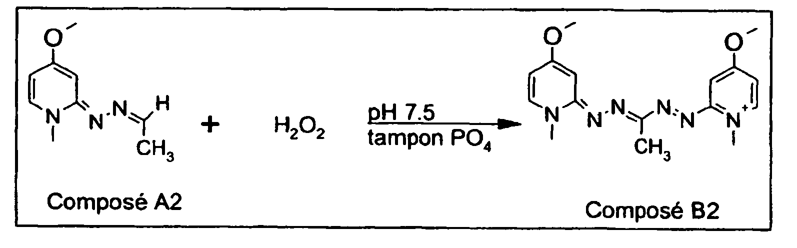

On dissout 100 mg d'azine (produit A2) dans 5ml de tampon phosphate (0,2M - pH 7,5).

On ajoute 0,57 ml de peroxyde d'hydrogène à 20 volumes.

Ce mélange est appliqué sur une mèche de 0,5g de cheveux à 90% blanc.

Après 30 minutes de contact la mèche de cheveux est rincée, shampouinée rincée et séchée.

La mèche obtenue est colorée violette.

### Exemple 11

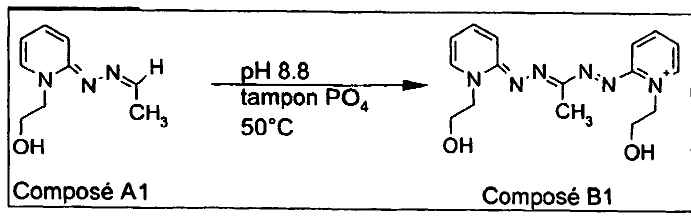

On pèse 10mg d'azine (composé A1) dans 2,5ml de tampon phosphate (0,2M - pH 8,8).

Ce mélange est appliqué sur une mèche de 0,25g de cheveux naturels à 90% de blanc.

Après 30 minutes de contact à 50°C la mèche de cheveu est rincée, shampouinée, rincée et séchée.

La mèche obtenue est colorée en bleu vert.

### Exemple 12

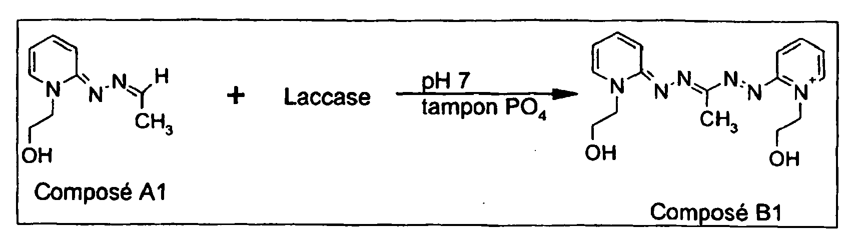

On pèse 10mg d'azine (composé A1) dans 2,5ml de tampon phosphate (0,2M pH 7).

On ajoute 50 U de l'enzyme laccase.

Ce mélange est appliqué sur une mèche de 0,25g cheveux naturels blancs à 90%.

Après 20 minutes de contact à température ambiante, la mèche de cheveux est rincée, shampouinée, rincée et séchée.

La mèche obtenue est colorée en bleue.

### Exemple 13

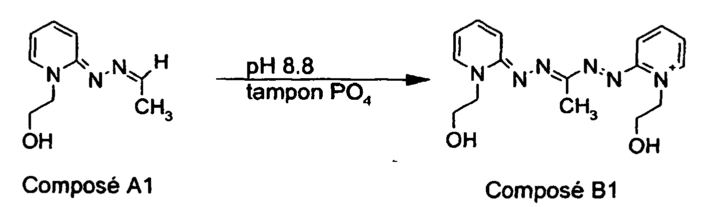

On pèse 10mg d'azine (composé A1 ) dans 2,5ml de tampon phosphate (0,2M - pH 8,8).

Ce mélange est appliqué sur une mèche de 0,25g de cheveux naturels à 90% de blanc.

Après 90 minutes de contact à température ambiante, la mèche de cheveux est rincée, shampouinée, rincée et séchée.

La mèche obtenue est légèrement colorée en vert.

## Revendications

1. Procédé de préparation de composés tétraazapentaméthiniques de formule (I) suivante : 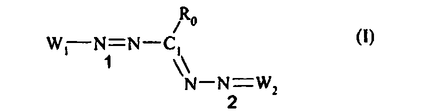 dans laquelle
- W₁ représente un radical hétéroaromatique cationique de formule (II) ou (III): 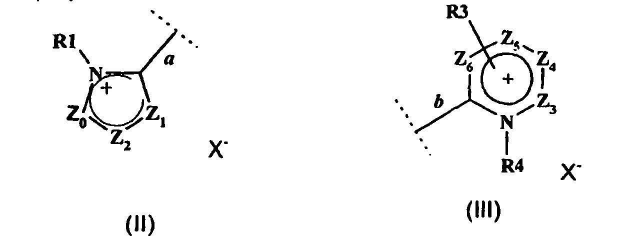
- W₂ représente un radical hétéroaromatique de formule (IV) ou (V) : 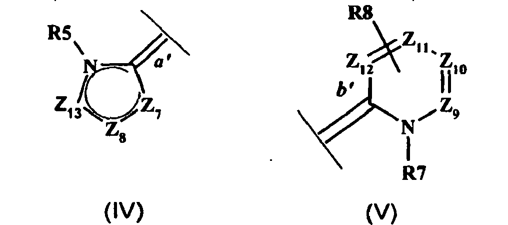
dans lesquelles :
- Z₀ représente un radical CR₂, un atome d'azote ou un radical NR₂₁,
- Z₁ représente un atome d'oxygène, de soufre ou un radical NR₉,
- Z₂ représente un atome d'azote ou un radical CR₁₀,
- Z₃ représente un atome d'azote ou un radical CR₁₁,
- Z₄ représente un atome d'azote ou un radical CR₁₂,
- Z₅ représente un atome d'azote ou un radical CR₁₃,
- Z₆ représente un atome d'azote ou un radical CR₁₄,
- Z₇ représente un atome d'oxygène, de soufre ou un radical NR₁₅,
- Z₈ représente un atome d'azote ou un radical CR₁₆,
- Z₉ représente un atome d'azote ou un radical CR₁₇,
- Z₁₀ représente un atome d'azote ou un radical CR₁₈,
- Z₁₁ représente un atome d'azote ou un radical CR₁₉,
- Z₁₂ représente un atome d'azote ou un radical CR₂₀,
- Z₁₃ représente un radical CR₆, un atome d'azote ou un radical NR₂₂,
- étant entendu que chacun des cycles des formules (II), (III), (IV) et (V) ne comportent pas plus de trois atomes d'azote et que deux des trois atomes d'azote peuvent être contigus,
- la liaison a du radical hétéroaromatique cationique à 5 chaînons de la formule (II) étant reliée à l'atome d'azote N₁ de la formule (I),
- la liaison b du radical hétéroaromatique cationique à 6 chaînons de la formule (III) étant reliée à l'atome d'azote N₁ de la formule (I).
- la double liaison a' du radical hétéroaromatique à 5 chaînons de la formule (IV) étant reliée à l'atome d'azote N₂ de la formule (I),
- la double liaison b' du radical hétéroaromatique à 6 chaînons de la formule (V) étant reliée à l'atome d'azote N₂ de la formule (I),
- la liaison b, reliant le radical hétéroaromatique cationique de la formule III à l'atome d'azote N₁ de la formule (I), étant située en position ortho ou para de l'atome d'azote portant le radical R₄ lorsque Z₅ représente un radical CR₁₃ ; la liaison b étant située en position ortho de l'atome d'azote portant le radical R₄ lorsque Z₅ représente un atome d'azote,
- la liaison b', reliant le radical hétéroaromatique de la formule V à l'atome d'azote N₂ de la formule (I), étant située en position ortho ou para de l'atome d'azote portant le radical R₇ lorsque Z₁₁ représente un radical CR₁₉; la liaison b' étant située en position ortho de l'atome d'azote portant le radical R₇ lorsque Z₁₁ représente un atome d'azote,
- R₂, R₆, R₁₀ et R₁₆ représentent, indépendamment l'un de l'autre, un atome d'hydrogène ; un radical alkyle en C₁-C₄ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle non substitué ou substitué par un ou deux radicaux choisis parmi les radicaux hydroxy, alcoxy en C₁-C₂, (poly)-hydroxyalcoxy en C₂-C₄, amino, (di)alkylamino en C₁-C₂ ; un radical carboxy ; un radical sulfonylamino,
- R₁, R₄, R₅, R₇, R₉, R₁₅, R₂₁, et R₂₂ représentent, indépendamment l'un de l'autre, un radical alkyle en C₁-C₈ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ;
- R₀, R₃, R₈, R₁₁, R₁₂, R₁₃, R₁₄, R₁₇, R₁₈, R₁₉, et R₂₀ représentent, indépendamment l'un de l'autre, un atome d'hydrogène, une chaîne hydrocarbonée en C₁-C₁₆ linéaire ou ramifiée, cette chaîne pouvant être saturée ou insaturée par une à trois insaturations, cette chaîne étant non substituée ou substituée par un à trois radicaux choisis parmi les radicaux hydroxy, alcoxy en C₁-C₂, (poly)-hydroxyalcoxy en C₁-C₄, amino, (di)alkylamino en C₁-C₂ , carboxy, sulfonique, sulfonylamino, (poly)-hydroxyalkylamino en C₂-C₄ ou un atome d'halogène tel que chlore, fluor ou brome ; un radical phényle non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique, sulfonylamino, (poly)-hydroxyalkylamino en C₂-C₄ ou un atome d'halogène tel que chlore, fluor ou brome ; un radical hétéroaryle choisi parmi les radicaux pyrazolyle, pyrrolyle, imidazolyle, thiazolyle, oxazolyle, triazolyle, pyridinyle, pyrimidinyle, triazinyle, pyrazinyle, pyridazinyle, en outre cette chaîne hydrocarbonée peut être interrompue par un ou deux atomes d'oxygène, d'azote, de soufre ou par un radical SO₂,
- étant entendu que R₀, R₃, R₈, R₁₁, R₁₂, R₁₃, R₁₄, R₁₇, R₁₈, R₁₉, et R₂₀ ne comportent pas de liaison peroxyde, ni de radicaux diazo ou nitroso,
- R₂ avec R₁₀, R₁₁ avec R₁₂, R₆ avec R₁₆, et R₁₇ avec R₁₈ pouvant former indépendamment les uns des autres un cycle aromatique carboné à 5 ou 6 chaînons non substitué ou substitué par un ou deux radicaux hydroxy, amino, (di)alkyl (C₁-C₂) amino, alcoxy C₁-C₂, (poly)hydroxyalkyl C₂-C₄ amino,
- X est un anion organique ou minéral ;
consistant à faire réagir, en présence d'au moins un agent oxydant, au moins un composé azine de formule (VI) et/ou (VII) : 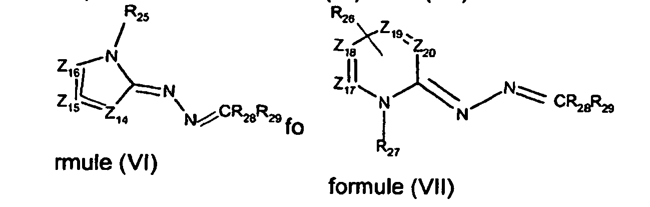 dans lesquelles :
- Z₁₄, Z₁₅, Z₁₆, R₂₅ ont respectivement les mêmes significations que Z₇, Z₈, Z₁₃, R₅ de la formule (IV), ou que Z₁, Z₂, Z₀, R₁ de la formule (II), définies ci-dessus,
- Z₁₇, Z₁₈, Z₁₉, Z₂₀, R₂₆, R₂₇ ont respectivement les mêmes significations que Z₉, Z₁₀, Z₁₁, Z₁₂, R₈, R₇ de la formule (V), ou que Z₃, Z₄, Z₅, Z₆, R₃, R₄ de la formule (III), définies ci-dessus,
- R₂₈, R₂₉, indépendamment l'un de l'autre, représente un atome d'hydrogène, un radical alkyle en C₁-C₆, de préférence en C₁-C₄, un radical alcoxy, linéaire ou ramifié, en C₁-C₆, de préférence en C₁-C₄, à la condition que l'un au moins des deux radicaux soit différent de l'hydrogène.

2. Procédé selon la revendication 1 dans laquelle R₀ représente un atome d'hydrogène ; un radical alkyle en C₁-C₆ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, (poly)hydroxyalkylamino en C₂-C₄, carboxy ou sulfonique : un radical phényle, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique ou un atome d'halogène tel que chlore, fluor ou brome; un radical hétéroaryle éventuellement cationique choisi parmi les radicaux pyrazolyle, pyrrolyle, imidazolyle, thiazolyle, oxazolyle, triazolyle, pyridinyle, pyrimidinyle, triazinyle, pyrazinyle, pyridazinyle.

3. Procédé selon l'une des revendications 1 à 2, **caractérisé en ce que** dans la formule (I), R₂, R₆, R₁₀ et R₁₆ représentent un atome d'hydrogène, un radical alkyle en C₁-C₄ non substitué ou substitué par un à deux radicaux choisis parmi les radicaux hydroxy, amino, (di)alkylamino en C₁-C₂, carboxy, un radical phényle.

4. Procédé selon l'une des revendications 1 à 3, **caractérisé en ce que** dans la formule (I), R₁, R₄, R₅, R₇, R₉, R₁₅, R₂₁ et R₂₂ représentent préférentiellement un radical alkyle en C₁-C₄, non substitué ou substitué par un à deux radicaux choisis parmi les radicaux hydroxy, alcoxy en C₁-C₂, amino, (di)alkylamino en C₁-C₂, carboxy, sulfonique.

5. Procédé selon l'une des revendications 1 à 4, **caractérisé en ce que** dans la formule (I), R₃, R₈, R₁₁, R₁₂, R₁₃, R₁₄, R₁₇, R₁₈, R₁₉, et R₂₀ représentent indépendamment l'un de l'autre, un atome d'hydrogène ; un radical alkyle en C₁-C₄ linéaire ou ramifié, non substitué ou substitué par un à deux radicaux choisis parmi les radicaux hydroxy, alcoxy en C₁-C₂, (poly)-hydroxyalcoxy en C₂-C₄, amino, (di)alkylamino en C₁-C₂ , carboxy ou sulfonique; un radical phényle , non substitué ou substitué par un à deux radicaux choisis parmi les radicaux hydroxy, alcoxy en C₁-C₂, (poly)-hydroxyalcoxy en C₂-C₄. amino, (di)alkylamino en C₁-C₂, carboxy, sulfonique ou un atome d'halogène tel que chlore, fluor ou brome ; un radical sulfonylamino ; un radical (poly)-hydroxyalkylamino en C₂-C₄.

6. Procédé selon l'une des revendications 1 à 5, **caractérisé en ce que** le composé de formule (I), dans lequel W₁ est un radical 2-pyridinium et W₂ est un radical 2-pyridine, est choisi parmi :
- Chlorure de 2-[5-(N-méthyl-2-pyridinylidène)-1-formazano]-N-méthylpyridinium.
- Chlorure de 2-[5-(N-méthyl-2-pyridinylidène)-3-méthyl-1-formazano]-N-méthyl pyridinium.
- Chlorure de 2-[5-(N-méthyl-2-pyridinylidène)-3-éthyl-1-formazano]-N-méthyl pyridinium.
- Chlorure de 2-[5-(N-méthyl-2-pyridinylidène)-3-isopropyl-1-formazano]-N-méthyl pyridinium.
- Chlorure de 2-[5-(N-méthyl-2-pyridinylidène)-3-phényl-1-formazano]-N-méthyl pyridinium.
- Chlorure de 2-[5-(N-méthyl-2-pyridinylidène)-3-(4'-méthoxyphényl)-1-formazano]-N-méthylpyridinium.
- Chlorure de 2-[5-(N-méthyl-2-pyridinylidène)-3-(4'-hydroxyphényl)-1-formazano]-N-méthylpyridinium.
- Chlorure de 2-[5-(N-méthyl-2-pyridinylidène)-3-(4'-N,N-diméthylaminophényl)-1-formazano]-N-méthylpyridinium.
- Chlorure de 2-[5-(4-pyrrolidino-N-méthyl-2-pyridinylidène)-1-formazano]-4-pyrrolidino-N-méthylpyridinium.
- Chlorure de 2-[5-(4-pyrrolidino-N-méthyl-2-pyridinylidène)-3-méthyl-1-formazano]-4-pyrrolidino-N-méthylpyridinium.
- Chlorure de 2-[5-(4-pyrrolidino-N-méthyl-2-pyridinylidène)-3-éthyl-1-formazano]- 4-pyrrolidino-N-méthylpyridinium.
- Chlorure de 2-[5-(4-pyrrolidino-N-méthyl-2-pyridinylidène)-3-isopropyl-1-formazano]- 4-pyrrolidino-N-méthylpyridinium.
- Chlorure de 2-[5-(4-pyrrolidino-N-méthyl-2-pyridinylidène)-3-phényl-1-formazano]-4-pyrrolidino-N-méthylpyridinium.
- Chlorure de 2-[5-(4-pyrrolidino-N-méthyl-2-pyridinylidène)-3-(4'-méthoxyphenyl)-1-formazano]- 4-pyrrolidino-N-méthylpyridinium.
- Chlorure de 2-[5-(4-pyrrolidino-N-méthyl-2-pyridinylidène)-3-(4'-hydroxyphényl)-1-formazano]- 4-pyrrolidino-N-méthylpyridinium.
- Chlorure de 2-[5-(4-pyrrolidino-N-méthyl-2-pyridinylidène)-3-(4'-N,N-diméthyl aminophényl)-1-formazano]- 4-pyrrolidino-N-méthylpyridinium.
- Chlorure de 2-[5-(N-hydroxyéthyl-2-pyridinylidène)-1-formazano]-N-hydroxy éthylpyridinium.
- Chlorure de 2-[5-(N-hydroxyéthyl-2-pyridinylidène)-3-méthyl-1-formazano]-N-hydroxyéthylpyridinium.
- Chlorure de 2-[5-(N-hydroxyéthyl-2-pyridinylidène)-3-ethyl-1-formazano]-N-hydroxyéthyl-pyridinium.
- Chlorure de 2-[5-(N- hydroxyethyl -2-pyridinylidene)-3-phényl-1-formazano]-N-hydroxyéthyl-pyridinium.
- Chlorure de 2-[5-(N- hydroxyéthyl -2-pyridinylidène)-3-isopropyl-1-formazano]-N-hydroxyéthyl -pyridinium.
- Chlorure de 2-[5-(N- hydroxyéthyl-2-pyridinylidène)-3-(4'-methoxyphényl)-1-formazano]-N- hydroxyéthyl -pyridinium.
- Chlorure de 2-[5-(N- hydroxyéthyl -2-pyridinylidène)-3-(4'-hydroxyphényl)-1-formazano]-N- hydroxyéthyl -pyridinium.
- Chlorure de 2-[5-(N- hydroxyéthyl -2-pyridinylidène)-3-(4'-N,N-diméthylamino phényl)-1-formazano]-N- hydroxyéthyl -pyridinium.
- Chlorure de 2-[5-(N-carboxyéthyl-2-pyridinylidène)-1-formazano]-N- carboxy éthyl - pyridinium.
- Chlorure de 2-[5-(N- carboxyéthyl -2-pyridinylidène)-3-méthyl-1-formazano]-N-carboxyéthyl -pyridinium.
- Chlorure de 2-[5-(N- carboxyéthyl -2-pyridinylidène)-3-éthyl-1-formazano]-N-carboxyéthyl -pyridinium.
- Chlorure de 2-[5-(N- carboxyéthyl -2-pyridinylidène)-3-phényl-1-formazano]-N-carboxyéthyl -pyridinium.
- Chlorure de 2-[5-(N- carboxyéthyl -2-pyridinylidène)-3-isopropyl-1-formazano]-N-carboxyéthyl -pyridinium.
- Chlorure de 2-[5-(N- carboxyéthyl -2-pyridinylidène)-3-(4'-méthoxyphényl)-1-formazano]-N- carboxyéthyl -pyridinium.
- Chlorure de 2-[5-(N- carboxyéthyl -2-pyridinylidèner3-(4'-hydroxyphényl)-1-formazano]-N- carboxyéthyl -pyridinium.
- Chlorure de 2-[5-(N- carboxyéthyl -2-pyridinylidène)-3-(4'-N,N-diméthylamino phényl)-1-formazano]-N- carboxyéthyl -pyridinium.

7. Procédé selon l'une des revendications 1 à 6, **caractérisé en ce que** le composé de formule (I), dans lequel W₁ est un radical 4-pyridinium et W₂ est un radical 4-pyridine, est choisi parmi :
- Chlorure de 4-[5-(N-méthyl-4-pyridinylidène)-1-formazano]-N-méthylpyridinium.
- Chlorure de 4-[5-(N-méthyl-4-pyridinylidène)-3-méthyl-1-formazano]-N-méthyl pyridinium.
- Chlorure de 4-[5-(N-méthyl-4-pyridinylidène)-3-éthyl-1-formazano]-N-méthyl pyridinium.
- Chlorure de 4-[5-(N-méthyl-4-pyridinylidène)-3-isopropyl-1-formazano]-N-méthyl pyridinium.
- Chlorure de 4-[5-(N-méthyl-4-pyridinylidène)-3-phényl-1-formazano]-N-méthyl pyridinium.
- Chlorure de 4-[5-(N-méthyl-4-pyridinylidène)-3-(4'-méthoxyphényl)-1-formazano]-N-méthylpyridinium.
- Chlorure de 4-[5-(N-méthyl-4-pyridinylidène)-3-(4'-hydroxyphényl)-1-formazano]-N-méthylpyridinium.
- Chlorure de 4-[5-(N-méthyl-4-pyridinylidène)-3-(4'-N,N-diméthylaminophényl)-1-formazano]-N-méthylpyridinium.
- Chlorure de 4-[5-(N-hydroxyéthyl-4-pyridinylidène)-1-formazano]-N-hydroxy-éthylpyridinium.
- Chlorure de 4-[5-(N-hydroxyéthyl-4-pyridinylidène)-3-méthyl-1-formazano]-N-hydroxyéthylpyridinium.
- Chlorure de 4-[5-(N-hydroxyéthyl-4-pyridinylidène)-3-éthyl-1-formazano]-N-hydroxyéthyl-pyridinium.
- Chlorure de 4-[5-(N- hydroxyéthyl -4-pyridinylidène)-3-phényl-1-formazano]-N-hydroxyéthyl-pyridinium.
- Chlorure de 4-[5-(N- hydroxyéthyl -4-pyridinylidène)-3-isopropyl-1-formazano]-N-hydroxyéthyl -pyridinium.
- Chlorure de 4-[5-(N- hydroxyéthyl-4-pyridinylidène)-3-(4'-méthoxyphényl)-1-formazano]-N- hydroxyéthyl -pyridinium.
- Chlorure de 4-[5-(N- hydroxyéthyl -4-pyridinylidène)-3-(4'-hydroxyphényl)-1-formazano]-N- hydroxyéthyl -pyridinium.
- Chlorure de 4-[5-(N- hydroxyéthyl -4-pyridinylidène)-3-(4'-N,N-diméthylamino-phényl)-1-formazano]-N- hydroxyéthyl -pyridinium.
- Chlorure de 4-[5-(N-carboxyéthyl-4-pyridinylidène)-1-formazano]-N- carboxy-éthylpyridinium.
- Chlorure de 4-[5-(N- carboxyéthyl -4-pyridinylidène)-3-méthyl-1-formazano]-N-carboxyéthyl -pyridinium.
- Chlorure de 4-[5-(N- carboxyéthyl -4-pyridinylidene)-3-ethyl-1-formazano]-N-carboxyéthyl -pyridinium.
- Chlorure de 4-[5-(N- carboxyéthyl -4-pyridinylidène)-3-phényl-1-formazano]-N-carboxyéthyl -pyridinium.
- Chlorure de 4-[5-(N- carboxyéthyl -4-pyridinylidène)-3-isopropyl-1-formazano]-N-carboxyéthyl -pyridinium.
- Chlorure de 4-[5-(N- carboxyéthyl -4-pyridinylidène)-3-(4'-méthoxyphényl)-1-formazano]-N- carboxyéthyl -pyridinium.
- Chlorure de 4-[5-(N- carboxyéthyl -4-pyridinylidène)-3-(4'-hydroxyphényl)-1-formazano]-N- carboxyéthyl -pyridinium.
- Chlorure de 4-[5-(N- carboxyéthyl-4-pyridinylidène)-3-(4'-N,N-diméthylaminophényl)-1-formazano]-N- carboxyéthyl -pyridinium.

8. Procédé selon l'une des revendications 1 à 7, **caractérisé en ce que** le composé de formule (I), dans lequel W₁ est un radical 2-imidazolium et W₂ est un radical 2-imidazole, est choisi parmi :
- Chlorure de 2-[5-(1,3-diméthyl-2-imidazolidène)-1-formazano]-1,3-diméthyl-imidazolium.
- Chlorure de 2-[5-(1,3-diméthyl-2-imidazolidène)-3-méthyl-1-formazano]-1,3-diméthyl-imidazolium.
- Chlorure de 2-[5-(1,3-diméthyl-2-imidazolidène)-3-éthyl-1-formazano]-1,3-diméthyl-imidazolium.
- Chlorure de 2-[5-(1,3-diméthyl-2-imidazolidene)-3-isopropyl-1-formazano]-1,3-diméthyl-imidazolium.
- Chlorure de 2-[5-(1,3-diméthyl-2-imidazolidène)-3-phényl-1-formazano]-1,3-diméthyl-imidazolium.
- Chlorure de 2-[5-(1,3-diméthyl-2-imidazolidène)-3-(4'-méthoxyphényl)-1-formazano]-1,3-diméthyl-imidazolium.
- Chlorure de 2-[5-(1,3-diméthyl-2-imidazolidène)-3-(4'-hydroxyphényl)-1-formazano]-1,3-diméthyl-imidazolium.
- Chlorure de 2-[5-(1,3-diméthyl-2-imidazolidène)-3-(4'-N,N-diméthylamino-phényl)-1-formazano]-1,3-diméthyl-imidazolium.
- Chlorure de 2-[5-(1,3-dihydroxyéthyl-2-imidazolidène)-1-formazano]-1,3-dihydroxyéthylimidazolium.
- Chlorure de 2-[5-(1,3-dihydroxyéthyl-2-imidazolidène)-3-méthyl-1-formazano]-1,3-dihydroxyéthylimidazolium.
- Chlorure de 2-[5-(1,3-dihydroxyéthyl-2-imidazolidène)-3-éthyl-1-formazano]-1,3-dihydroxyéthyl -imidazolium.
- Chlorure de 2-[5-(1,3-dihydroxyéthyl -2-imidazolidène)-3-phényl-1-formazano]-1,3-dihydroxyéthyl -imidazolium.
- Chlorure de 2-[5-(1,3-dihydroxyéthyl -2-imidazolidène)-3-isopropyl-1-formazano]-1,3-dihydroxyéthyl -imidazolium.
- Chlorure de 2-[5-(1,3-dihydroxyéthyl-2-imidazolidène)-3-(4'-méthoxyphényl)-1-formazano]-1,3-dihydroxyéthyl -imidazolium.
- Chlorure de 2-[5-(1,3-dihydroxyéthyl -2-imidazolidène)-3-(4'-hydroxyphényl)-1-formazano]-1,3-dihydroxyéthyl -imidazolium.
- Chlorure de 2-[5-(1,3-dihydroxyéthyl -2-imidazolidène)-3-(4'-N,N-diméthylamino phényl)-1-formazano]-1,3-dihydroxyéthyl -imidazolium.
- Chlorure de 2-[5-(1,3-dicarboxyéthyl-2-imidazolidène)-1-formazano]-1,3-dicarboxyéthyl -imidazolium.
- Chlorure de 2-[5-(1,3-dicarboxyéthyl -2-imidazolidène)-3-méthyl-1-formazano]-1,3-dicarboxyéthyl -imidazolium.
- Chlorure de 2-[5-(1,3-dicarboxyéthyl -2-imidazolidène)-3-éthyl-1-formazano]-1,3-dicarboxyéthyl -imidazolium.
- Chlorure de 2-[5-(1,3-dicarboxyéthyl -2-imidazolidène)-3-phényl-1-formazano]-1,3-dicarboxyéthyl -imidazolium.
- Chlorure de 2-[5-(1,3-dicarboxyéthyl -2-imidazolidène)-3-isopropyl-1-formazano]-1,3-dicarboxyéthyl -imidazolium.
- Chlorure de 2-[5-(1,3-dicarboxyéthyl -2-imidazolidène)-3-(4'-méthoxyphényl)-1-formazano]-1,3-dicarboxyéthyl -imidazolium.
- Chlorure de 2-[5-(1,3-dicarboxyéthyl -2-imidazolidène)-3-(4'-hydroxyphényl)-1-formazano]-1,3-dicarboxyéthyl -imidazolium.
- Chlorure de 2-[5-(1,3-dicarboxyéthyl -2-imidazolidène)-3-(4'-N,N-diméthylamino phényl)-1-formazano]-1,3-dicarboxyéthyl -imidazolium.

9. Procédé selon l'une des revendications 1 à 8, **caractérisé en ce que** le composé de formule (I), dans lequel W₁ est un radical 5-pyrazolium et W₂ est un radical 5-pyrazole, est choisi parmi :
- Chlorure de 5-[5-(1,2-diméthyl-5-pyrazolidène)-1-formazano]-1,2-diméthyl-pyrazolinium.
- Chlorure de 5-[5-(1,2-diméthyl-5-pyrazolidène)-3-rnéthyl-1-formazano]-1,2-diméthyl-pyrazolinium.
- Chlorure de 5-[5-(1,2-diméthyl-5-pyrazolidène)-3-éthyl-1-formazano]-1,2-diméthyl-pyrazolinium.
- Chlorure de 5-[5-(1,2-diméthyl-5-pyrazolidène)-3-isopropyl-1-formazano]-1,2-diméthyl-pyrazolinium.
- Chlorure de 5-[5-(1,2-diméthyl-5-pyrazolidène)-3-phényl-1 -formazano]-1,2-diméthyl-pyrazolinium.
- Chlorure de 5-[5-(1,2-diméthyl-5-pyrazolidène)-3-(4'-méthoxyphényl)-1-formazano]-1,2-diméthyl-pyrazolinium.
- Chlorure de 5-[5-(1,2-diméthyl-5-pyrazolidène)-3-(4'-hydroxyphényl)-1-formazano]-1,2-diméthyl-pyrazolinium.
- Chlorure de 5-[5-(1,2-diméthyl-5-pyrazolidène)-3-(4'-N,N-dimethylaminophényl)-1-formazano]-1,2-diméthyl-pyrazolinium.
- Chlorure de 5-[5-(1,2-dihydroxyéthyl-5-pyrazolidène)-1-formazano]-1,2-dihydroxyéthylpyrazolinium.
- Chlorure de 5-[5-(1,2-dihydroxyéthyt-5-pyrazolidène)-3-méthyl-1-formazano]-1,2-dihydroxyéthylpyrazolinium.
- Chlorure de 5-[5-(1,2-dihydroxyéthyl-5-pyrazolidène)-3-éthyl-1-formazano]-1,2-dihydroxyéthyl -pyrazolinium.
- Chlorure de 5-[5-(1,2-dihydroxyéthyl -5-pyrazolidène)-3-phényl-1-formazano]-1,2-dihydroxyéthyl -pyrazolinium.
- Chlorure de 5-[5-(1,2-dihydroxyéthyl -5-pyrazolidène)-3-isopropyl-1-formazano]-1,2-dihydroxyéthyl -pyrazolinium.
- Chlorure de 5-[5-(1,2-dihydroxyéthyl-5-pyrazolidène)-3-(4'-méthoxyphényl)-1-formazano]-1,2-dihydroxyéthyl -pyrazolinium.
- Chlorure de 5-[5-(1,2-dihydroxyéthyl -5-pyrazolidène)-3-(4'-hydroxyphényl)-1-formazano]-1,2-dihydroxyéthyl -pyrazolinium.
- Chlorure de 5-[5-(1,2-dihydroxyéthyl -5-pyrazolidène)-3-(4'-N,N-diméthylamino phényl)-1-formazano]-1,2-dihydroxyéthyl -pyrazolinium.
- Chlorure de 5-[5-(1,2-dicarboxyéthyl-5-pyrazolidène)-1-formazano]-1,2-dicarboxyéthyl -pyrazolinium.
- Chlorure de 5-[5-(1,2-dicarboxyéthyl -5-pyrazolidène)-3-méthyl-1-formazano]-1,2-dicarboxyéthyl -pyrazolinium.
- Chlorure de 5-[5-(1,2-dicarboxyéthyl -5-pyrazolidène)-3-éthyl-1-formazano]-1,2-dicarboxyéthyl -pyrazolinium.
- Chlorure de 5-[5-(1,2-dicarboxyéthyl -5-pyrazolidène)-3-phényl-1-formazano]-1,2-dicarboxyéthyl -pyrazolinium.
- Chlorure de 5-[5-(1,2-dicarboxyéthyl -5-pyrazolidène)-3-isopropyl-1-formazano]-1,2-dicarboxyéthyl -pyrazolinium.
- Chlorure de 5-[5-(1,2-dicarboxyéthyl -5-pyrazolidène)-3-(4'-méthoxyphényl)-1-formazano]-1,2-dicarboxyéthyl -pyrazolinium.
- Chlorure de 5-[5-(1,2-dicarboxyéthyl -5-pyrazolidène)-3-(4'-hydroxyphényl)-1-formazano]-1,2-dicarboxyéthyl -pyrazolinium.
- Chlorure de 5-[5-(1,2-dicarboxyéthyl -5-pyrazolidène)-3-(4'-N,N-diméthylamino phényl)-1-formazano]-1,2-dicarboxyéthyl -pyrazolinium.

10. Procédé selon l'une des revendications 1 à 9, **caractérisé en ce que** le composé de formule (I), dans lequel W₁ est un radical 2-benzimidazolium et W₂ est un radical 2-pyridine, est choisi parmi :
- Chlorure de 2-[5-(N-methyl-2-pyridinylidène)-1-formazano]-1,3-diméthyl-benzimidazolium.
- Chlorure de 2-[5-(N-methyl-2-pyridinylidène)-3-méthyl-1-formazano]-1,3-diméthyl-benzimidazolium.
- Chlorure de 2-[5-(N-méthyl-2-pyridinylidène)-3-éthyl-1-formazano]-1,3-diméthyl-benzimidazolium.
- Chlorure de 2-[5-(N-méthyl-2-pyridinylidène)-3-isopropyl-1-formazano]-1,3-diméthyl-benzimidazolium.
- Chlorure de 2-[5-(N-méthyl-2-pyridinylidène)-3-phényl-1-formazano]-1,3-diméthyl-benzimidazolium.
- Chlorure de 2-[5-(N-méthyl-2-pyridinylidène)-3-(4'-méthoxyphényl)-1-formazano]-1,3-diméthyl-benzimidazolium.
- Chlorure de 2-[5-(N-methyl-2-pyridinylidène)-3-(4'-hydroxyphényl)-1-formazano]-1,3-diméthyl-benzimidazolium.
- Chlorure de 2-[5-(N-méthyl-2-pyridinylidène)-3-(4'-N,N-diméthylaminophényl)-1-formazano]-1,3-diméthyl-benzimidazolium.
- Chlorure de 2-[5-(N-hydroxyéthyl-2-pyridinylidène)-1-formazano]-1,3-dihydroxy éthylbenzimidazolium.
- Chlorure de 2-[5-(N-hydroxyéthyl-2-pyridinylidène)-3-méthyl-1-formazano]-1,3-dihydroxyéthylbenzimidazolium.
- Chlorure de 2-[5-(N-hydroxyéthyl-2-pyridinylidène)-3-ethyl-1-formazano]-1,3-dihydroxyéthyl -benzimidazolium.
- Chlorure de 2-[5-(N-hydroxyéthyl-2-pyridinylidène)-3-phényl-1-formazano]-1,3-dihydroxyéthyl -benzimidazolium.
- Chlorure de 2-[5-(N-hydroxyéthyl-2-pyridinylidène)-3-isopropyl-1-formazano]-1,3-dihydroxyéthyl -benzimidazolium.
- Chlorure de 2-[5-(N-hydroxyéthyl-2-pyridinylidène)-3-(4'-méthoxyphényl)-1-formazano]-1,3-dihydroxyéthyl -benzimidazolium.
- Chlorure de 2-[5-(N-hydroxyéthyl-2-pyridinylidène)-3-(4'-hydroxyphényl)-1-formazano]-1,3-dihydroxyéthyl -benzimidazolium.
- Chlorure de 2-[5-(N-hydroxyéthyl-2-pyridinylidène)-3-(4'-N,N-diméthylamino phényl)1-formazano]-1,3-dihydroxyéthyl -benzimidazolium.
- Chlorure de 2-[5-(N-carboxyéthyl-2-pyridinylidène)-1-formazano]-1,3-dicarboxyéthyl -benzimidazolium.
- Chlorure de 2-[5-(N-carboxyéthyl-2-pyridinylidène)-3-méthyl-1-formazano]-1,3-dicarboxyéthyl -benzimidazolium.
- Chlorure de 2-[5-(N-carboxyéthyl-2-pyridinylidène)-3-ethyl-1-formazano]-1,3-dicarboxyéthyl -benzimidazolium.
- Chlorure de 2-[5-(N-carboxyéthyl-2-pyridinylidène)-3-phenyl-1-formazano]-1,3-dicarboxyéthyl -benzimidazolium.
- Chlorure de 2-[5-(N-carboxyéthyl-2-pyridinylidène)-3-isopropyl-1-formazano]-1,3-dicarboxyéthyl -benzimidazolium.
- Chlorure de 2-[5-(N-carboxyéthyl-2-pyridinylidène)-3-(4'-méthoxyphényl)-1-formazano]-1,3-dicarboxyéthyl -benzimidazolium.
- Chlorure de 2-[5-(N-carboxyéthyl-2-pyridinylidène)-3-(4'-hydroxyphényl)-1-formazano]-1,3-dicarboxyéthyl -benzimidazolium.
- Chlorure de 2-[5-(N-carboxyéthyl-2-pyridinylidène)-3-(4'-N,N-diméthylamino phényl)-1-formazano]-1,3-dicarboxyéthyl -benzimidazolium.

11. Procédé selon l'une des revendications 1 à 10, **caractérisé en ce que** le composé de formule (I), dans lequel W₁ est un radical 2-benzimidazolium et W₂ est un radical 4-pyridine, est choisi parmi:
- Chlorure de 2-[5-(N-méthyl-4-pyridinylidène)-1-formazano]-1,3-diméthyl-benzimidazolium.
- Chlorure de 2-[5-(N-méthyl-4-pyridinylidène)-3-méthyl-1-formazano]-1,3-diméthyl-benzimidazolium.
- Chlorure de 2-[5-(N-méthyl-4-pyridinylidène)-3-éthyl-1-formazano]-1,3-diméthyl-benzimidazolium.
- Chlorure de 2-[5-(N-méthyl-4-pyridinylidène)-3-isopropyl-1-formazano]-1,3-diméthyl-benzimidazolium.
- Chlorure de 2-[5-(N-méthyl-4-pyridinylidène)-3-phényl-1-formazano]-1,3-diméthyl-benzimidazolium.
- Chlorure de 2-[5-(N-méthyl-4-pyridinylidène)-3-(4'-méthoxyphényl)-1-formazano]-1,3-diméthyl-benzimidazolium.
- Chlorure de 2-[5-(N-méthyl-4-pyridinylidène)-3-(4'-hydroxyphényl)-1-formazano]-1,3-diméthyl-benzimidazolium.
- Chlorure de 2-[5-(N-méthyl-4-pyridinylidène)-3-(4'-N,N-diméthylaminophényl)-1-formazano]-1,3-diméthyl-benzimidazolium.
- Chlorure de 2-[5-(N-hydroxyéthyl-4-pyridinylidène)-1-formazano]-1,3-dihydroxyéthylbenzimidazolium.
- Chlorure de 2-[5-(N-hydroxyéthyl-4-pyridinylidène)-3-méthyl-1-formazano]-1,3-dihydroxyéthylbenzimidazolium.
- Chlorure de 2-[5-(N-hydroxyéthyl-4-pyridinylidène)-3-éthyl-1-formazano]-1,3-dihydroxyéthyl -benzimidazolium.
- Chlorure de 2-[5-(N-hydroxyéthyl-4-pyridinylidène)-3-phényl-1-formazano]-1,3-dihydroxyéthyl -benzimidazolium.
- Chlorure de 2-[5-(N-hydroxyéthyl-4-pyridinylidène)-3-isopropyl-1-formazano]-1,3-dihydroxyéthyl -benzimidazolium.
- Chlorure de 2-[5-(N-hydroxyéthyl-4-pyridinylidène)-3-(4'-méthoxyphényl)-1-formazano]-1,3-dihydroxyéthyl -benzimidazolium.
- Chlorure de 2-[5-(N-hydroxyéthyl-4-pyridinylidène)-3-(4'-hydroxyphényl)-1-formazano]-1,3-dihydroxyéthyl -benzimidazolium.
- Chlorure de 2-[5-(N-hydroxyéthyl-4-pyridinylidène)-3-(4'-N,N-diméthylamino phényl)-1-formazano]-1,3-dihydroxyéthyl -benzimidazolium.
- Chlorure de 2-[5-(N-carboxyéthyl-4-pyridinylidène)-1-formazano]-1,3-dicarboxyéthyl -benzimidazolium.
- Chlorure de 2-[5-(N-carboxyéthyl-4-pyridinylidène)-3-méthyl-1-formazano]-1,3-dicarboxyéthyl -benzimidazolium.
- Chlorure de 2-[5-(N-carboxyéthyl-4-pyridinylidène)-3-éthyl-1-formazano]-1,3-dicarboxyéthyl -benzimidazolium.
- Chlorure de 2-[5-(N-carboxyéthyl-4-pyridinylidène)-3-phényl-1-formazano]-1,3-dicarboxyéthyl -benzimidazolium.
- Chlorure de 2-[5-(N-carboxyéthyl-4-pyridinylidène)-3-isopropyl-1-formazano]-1,3-dicarboxyéthyl -benzimidazolium.
- Chlorure de 2-[5-(N-carboxyéthyl-4-pyridinylidène)-3-(4'-méthoxyphényl)-1-formazano]-1,3-dicarboxyéthyl -benzimidazolium.
- Chlorure de 2-[5-(N-carboxyéthyl-4-pyridinylidène)-3-(4'-hydroxyphényl)-1-formazano]-1,3-dicarboxyéthyl-benzimidazolium.
- Chlorure de 2-[5-(N-carboxyéthyl-4-pyridinylidène)-3-(4'-N,N-diméthylamino phényl)-1-formazano]-1,3-dicarboxyéthyl -benzimidazolium.

12. Procédé selon l'une des revendications 1 à 11, **caractérisé en ce que**, le composé de formule (I), dans lequel W₁ est un radical 2-imidazolium et W₂ est un radical 2-pyridine, est choisi parmi :
- Chlorure de 2-[5-(N-méthyl-2-pyridinylidène)-1-formazano]-1,3-diméthyl-imidazolium.
- Chlorure de 2-[5-(N-méthyl-2-pyridinylidène)-3-méthyl-1-formazano]-1,3-diméthyl-imidazolium.
- Chlorure de 2-[5-(N-méthyl-2-pyridinylidène)-3-éthyl-1-formazano]-1,3-diméthyl-imidazolium.
- Chlorure de 2-[5-(N-méthyl-2-pyridinylidène)-3-isopropyl-1-formazano]-1,3-diméthyl-imidazolium.
- Chlorure de 2-[5-(N-méthyl-2-pyridinylidène)-3-phényl-1-formazano]-1,3-diméthyl-imidazolium.
- Chlorure de 2-[5-(N-méthyl-2-pyridinylidène)-3-(4'-méthoxyphényl)-1-formazano]-1,3-diméthyl-imidazolium.
- Chlorure de 2-[5-(N-méthyl-2-pyridinylidène)-3-(4'-hydroxyphényl)-1-formazano]-1,3-diméthyl-imidazolium.
- Chlorure de 2-[5-(N-méthyl-2-pyridinylidène)-3-(4'-N,N-diméthylaminophényl)-1-formazano]-1,3-diméthyl-imidazolium.
- Chlorure de 2-[5-(N-hydroxyéthyl-2-pyridinylidène)-1-formazano]-1,3-dihydroxyéthylimidazolium.
- Chlorure de 2-[5-(N-hydroxyéthyl-2-pyridinylidène)-3-méthyl-1-formazano]-1,3-dihydroxyéthylimidazolium.
- Chlorure de 2-[5-(N-hydroxyéthyl-2-pyridinylidéne)-3-éthyl-1-formazano]-1,3-dihydroxyéthyl -imidazolium.
- Chlorure de 2-[5-(N-hydroxyéthyl-2-pyridinylidène)-3-phényl-1-formazano]-1,3-dihydroxyéthyl -imidazolium.
- Chlorure de 2-[5-(N-hydroxyéthyl-2-pyridinylidène)-3-isopropyl-1-formazano]-1,3-dihydroxyéthyl -imidazolium.
- Chlorure de 2-[5-(N-hydroxyéthyl-2-pyridinylidène)-3-(4'-méthoxyphényl)-1-formazano]-1,3-dihydroxyéthyl -imidazolium.
- Chlorure de 2-[5-(N-hydroxyéthyl-2-pyridinylidène)-3(4'-hydroxyphényl)-1-formazano]-1,3-dihydroxyéthyl -imidazolium.
- Chlorure de 2-[5-(N-hydroxyéthyl-2-pyridinylidène)-3-(4'-N,N-diméthylamino phényl)-1-formazano]-1,3-dihydroxyéthyl -imidazolium.
- Chlorure de 2-[5-(N-carboxyéthyl-2-pyridinylidène)-1-formazano]-1,3-dicarboxyéthyl -imidazolium.
- Chlorure de 2-[5-(N-carboxyéthyl-2-pyridinylidène)-3-méthyl-1-formazano]-1,3-dicarboxyéthyl -imidazolium.
- Chlorure de 2-[5-(N-carboxyéthyl-2-pyridinylidène)-3-éthyl-1-formazano]-1,3-dicarboxyéthyl -imidazolium.
- Chlorure de 2-[5-(N-carboxyéthyl-2-pyridinylidène)-3-phényl-1-formazano]-1,3-dicarboxyéthyl -imidazolium.
- Chlorure de 2-[5-(N-carboxyéthy)-2-pyridinylidène)-3-isopropyl-1-formazano]-1,3-dicarboxyéthyl -imidazolium.
- Chlorure de 2-[5-(N-carboxyéthyl-2-pyridinylidène)-3-(4'-méthoxyphényl)-1-formazano]-1,3-dicarboxyéthyl -imidazolium.
- Chlorure de 2-[5-(N-carboxyéthyl-2-pyridinylidène)-3-(4'-hydroxyphényl)-1-formazano]-1,3-dicarboxyéthyl -imidazolium.
- Chlorure de 2-[5-(N-carboxyéthyl-2-pyridinylidène)-3-(4'-N,N-diméthylamino phényl)-1-formazano]-1,3-dicarboxyéthyl -imidazolium.

13. Procédé selon l'une des revendications 1 à 12 **caractérisé en ce que**, le composé de formule (1), dans lequel W₁ est un radical 2-imidazolium et W₂ est un radical 4-pyridine est choisi parmi :
- Chlorure de 2-[5-(N-méthyl-4-pyridinylidène)-1-formazano]-1,3-diméthyl-imidazolium.
- Chlorure de 2-[5-(N-methyl-4-pyridinylidène)-3-méthyl-1-formazano]-1,3-diméthyl-imidazolium.
- Chlorure de 2-[5-(N-méthyl-4-pyridinylidène)-3-éthyl-1-formazano]-1,3-diméthyl-imidazolium.
- Chlorure de 2-[5-(N-méthyl-4-pyridinylidéne)-3-isopropyl-1-formazano]-1,3-diméthyl-imidazolium.
- Chlorure de 2-[5-(N-méthyl-4-pyridinylidène)-3-phényl-1-formazano]-1,3-diméthyl-imidazolium.
- Chlorure de 2-[5-(N-méthyl-4-pyridinylidène)-3-(4'-méthoxyphényl)-1-formazano]-1,3-diméthyl-imidazolium.
- Chlorure de 2-[5-(N-méthyl-4-pyrydinylidène)-3-(4'-hydroxyphényl)-1-formazano]-1,3-diméthyl-imidazolium.
- Chlorure de 2-[5-(N-méthyl-4-pyridinylidène)-3-(4' -N,N-diméthylaminophényl)-1-formazano]-1,3-diméthyl-imidazolium.
- Chlorure de 2-[5-(N-hydroxyéthyl-4-pyridinylidène)-1-formazano]-1,3-dihydroxyéthylmidazolium.
- Chlorure de 2-[5-(N-hydroxyéthyl-4-pyridinylidène)-3-méthyl-1-formazano]-1,3-dihydroxyéthylimidazolium.
- Chlorure de 2-[5-(N-hydroxyéthyl-4-pyridinylidène)-3-éthyl-1-formazano]-1,3-dihydroxyéthyl -imidazolium.
- Chlorure de 2-[5-(N-hydroxyéthyl-4-pyridinylidène)-3-phényl-1-formazano]-1,3-dihydroxyéthyl -imidazolium.
- Chlorure de 2-[5-(N-hydroxyéthyl-4-pyridinylidène)-3-isopropyl-1-formazano]-1,3-dihydroxyéthyl -imidazolium.
- Chlorure de 2-[5-(N-hydroxyéthyl-4-pyridinylidène)-3-(4'-méthoxyphényl)-1-formazano]-1,3-dihydroxyéthyl -imidazolium.
- Chlorure de 2-[5-(N-hydroxyéthyl-4-pyridinylidène)3-(4'-hydroxyphényl)-1-formazano]-1,3-dihydroxyéthyl -imidazolium.
- Chlorure de 2-[5-(N-hydroxyéthyl-4-pyridinylidène)-3-(4'-N,N-dimethylamino phényl)-1-formazano]-1,3-dihydroxyéthyl -imidazolium.
- Chlorure de 2-[5-(N-carboxyéthyl-4-pyridinylidène)-1-formazano]-1,3-dicarboxyéthyl -imidazolium.
- Chlorure de 2-[5-(N-carboxyéthyl-4-pyridinylidène)-3-méthyl-1-formazano]-1,3-dicarboxyéthyl -imidazolium.
- Chlorure de 2-[5-(N-carboxyéthyl-4-pyridinylidène)-3-éthyl-1-formazano]-1,3-dicarboxyéthyl -imidazolium.
- Chlorure de 2-[5-(N-carboxyéthyl-4-pyridinylidène)-3-phényl-1-formazano]-1,3-dicarboxyéthyl -imidazolium.
- Chlorure de 2-[5-(N-carboxyéthyl-4-pyridinylidène)-3-isopropyl-1-formazano]-1,3-dicarboxyéthyl -imidazolium.
- Chlorure de 2-[5-(N-carboxyéthyl-4-pyridinylidène)-3-(4'-méthoxyphényl)-1-formazano]-1,3-dicarboxyéthyl -imidazolium.
- Chlorure de 2-[5-(N-carboxyéthyl-4-pyridinylidène)-3-(4'-hydroxyphényl)-1 formazano]-1,3-dicarboxyéthyl -imidazolium.
- Chlorure de 2-[5-(N-carboxyéthyl-4-pyridinylidène)-3-(4'-N,N-diméthylamino phényl)-1-formazano]-1,3-dicarboxyéthyl-imida-zolium.

14. Procédé selon l'une quelconque des revendications précédentes, **caractérisée en ce que** l'agent oxydant est choisi parmi le peroxyde d'hydrogène ; le peroxyde d'urée ; les peracides organiques ; les persels , les persulfates ; les chromates ou les bichromates ; les hypochlorites ; les hypobromites ; les ferricyanures ; les bioxydes de manganèse ou de plomb, seuls ou en mélanges.

15. Procédé selon la revendication précédente, **caractérisé en ce que** l'agent oxydant est le peroxyde d'hydrogène.

16. Procédé selon l'une quelconque des revendications précédentes, **caractérisée en ce que** l'agent oxydant est choisi parmi les enzymes, en présence d'un substrat approprié pour ladite enzyme.

17. Procédé selon la revendication précédente, **caractérisé en ce que** l'enzyme est choisie parmi les oxydases produisant du peroxyde d'hydrogène, en présence d'un substrat pour ladite enzyme.

18. Procédé selon la revendication précédente, **caractérisée en ce que** l'enzyme est choisie parmi :
| | |
|---|---|
| PYRANOSE OXIDASE | EC 1.1.3.10 |
| L-SORBOSE OXIDASE | EC 1.1.3.11 |
| PYRIDOXINE 4 - OXIDASE | EC 1.1.3.12 |
| ALCOHOL OXIDASE | EC 1.1.3.13 |
| CATECHOL-OXIDASE (DIMERIZING). | EC 1.1.3.14 |
| (S)-2-HYDROXY-ACID OXIDASE. | EC 1.1.3.15 |
| ECDYSONE OXIDASE | EC 1.1.3.16 |
| CHOLINE OXIDASE | EC 1.1.3.17 |
| SEC-ALCOHOL OXIDASE | EC 1.1.3.18 |
| 4 - HYDROXYMANDALATE OXIDASE | EC 1.1.3.19 |
| LONG-CHAIN-ALCOHOL OXIDASE. | EC 1.1.3.20 |
| GLYCEROL-3-PHOSPHATE OXIDASE. | EC 1.1.3.21 |
| XANTHINE OXIDASE | EC 1.1.3.22 |
| THIAMIN OXIDASE | EC 1.1.3.23 |
| L - GALACTONOLACTONE OXIDASE | EC 1.1.3.24 |
| CELLOBIOSE OXIDASE | EC 1.1.3.25 |
| HYDROXYPHYTANATE OXIDASE | EC 1.1.3.27 |
| NUCLEOSIDE OXIDASE | EC 1.1.3.28 |
| N-ACYLHEXOSAMINE OXIDASE | EC 1.1.3.29 |
| MALATE OXIDASE | EC 1.1.3.3 |
| POLYVINYL-ALCOHOL OXIDASE. | EC 1.1.3.30 |
| METHANOL OXIDASE. | EC 1.1.3.31 |
| D-ARABINONO-1.4-LACTONE OXIDASE. | EC 1.1.3.37 |
| VANILLYL-ALCOHOL OXIDASE. | EC 1.1.3.38. |
| NUCLEOSIDE OXIDASE (H(2)O(2)-FORMING). | EC 1.1.3.39 |
| GLUCOSE OXIDASE | EC 1.1.3.4 |
| D-MANNITOL OXIDASE. | EC 1.1.3.40 |
| XYLITOL OXIDASE. | EC 1.1.3.41 |
| HEXOSE OXIDASE | EC 1.1.3.5 |
| CHOLESTROL OXIDASE | EC 1.1.3.6 |
| ARYL ALCOHOL OXIDASE | EC 1.1.3.7 |
| L-GULONOLACTONE OXIDASE. | EC 1.1.3.8 |
| GALACTOSE OXIDASE | EC 1.1.3.9 |
| PTERIDINE OXIDASE | EC 1.17.3.1 |
| RETINAL OXIDASE | EC 1.2.3.11 |
| ALDEHYDE OXIDASE. | EC 1.2.3.1 |
| CARBON MONOXIDE OXIDASE | EC 1.2.3.10 |
| VANILLATE DEMETHYLASE | EC 1.2.3.12 |
| 4-HYDROXYPHENYLPYRUVATE OXIDASE. | EC 1.2.3.13 |
| PYRUVATE OXIDASE | EC 1.2.3.3 |
| OXALATE OXIDASE | EC 1.2.3.4 |
| GLYOXALATE OXIDASE | EC 1.2.3.5 |
| PYRUVATE OXIDASE (CO-A ACETYLATING) | EC 1.2.3.6 |
| INDOLE-3-ACETALDEHYDE OXIDASE. | EC 1.2.3.7 |
| PYRIDOXAL OXIDASE. | EC 1.2.3.8 |
| ARYL ALDEHYDE OXIDASE | EC 1.2.3.9. |
| DIHYDROOROTATE OXIDASE | EC 1.3.3.1 |
| LATHOSTEROL OXIDASE | EC 1.3.3.2 |
| COPROPORPHYRINOGEN OXIDASE. | EC 1.3.3.3 |
| PROTOPORPHYRINOGEN OXIDASE. | EC 1.3.3.4 |
| BILIRUBIN OXIDASE. | EC 1.3.3.5 |
| ACYL CO-A OXIDASE | EC 1.3.3.6 |
| DIHYDROURACIL OXIDASE | EC 1.3.3.7 |
| TETRAHYDROBERBERINE OXIDASE | EC 1.3.3.8 |
| SECOLOGANIN SYNTHASE. | EC 1.3.3.9 |
| ASPARTATE OXIDASE | EC 1.4.3.1 |
| PUTRESCINE OXIDASE | EC 1.4.3.10 |
| L-GLUTAMATE OXIDASE | EC 1.4.3.11 |
| CYCLOHEXYLAMINE OXIDASE. | EC 1.4.3.12 |
| PROTEIN-LYSINE 6-OXIDASE | EC 1.4.3.13 |
| L-LYSINE OXIDASE | EC 1.4.3.14 |
| D-GLUTAMATE (D-ASPARTATE) OXIDASE | EC 1.4.3.15 |
| L-ASPARTATE OXIDASE | EC 1.4.3.16 |
| TRYPTOPHAN ALPHA, BETA-OXIDASE | EC 1.4.3.17 |
| GLYCINE OXIDASE. | EC 1.4.3.19 |
| L-AMINO ACID OXIDASE | EC 1.4.3.2 |
| D-AMINO ACID OXIDASE | EC 1.4.3.3 |
| AMINE OXIDASE (FLAVIN CONTAINING) | EC 1.4.3.4 |
| PYRIDOXAMINE PHOSPHATE OXIDASE | EC 1.4.3.5 |
| AMINE OXIDASE (COPPER CONTAINING) | EC 1.4.3.6 |
| D-GLUTAMATE OXIDASE | EC 1.4.3.7 |
| ETHANOLAMINE OXIDASE | EC 1.4.3.8 |
| SARCOSINE OXIDASE | EC 1.5.3.1 |
| DIMETHYLGLYCINE OXIDASE | EC 1.5.3.10 |
| POLYAMINE OXIDASE | EC 1.5.3.11 |
| DIHYDROBENZOPHENANTHRIDINE OXIDASE. | EC 1.5.3.12 |
| N-METHY-L-AMINO ACID OXIDASE | EC 1.5.3.2 |
| N-METHYL-LYSINE OXIDASE | EC 1.5.3.4 |
| (S)-6-HYDROXYNICOTINE OXIDASE | EC 1.5.3.5 |
| (R)-6-HYDROXYNICOTINE OXIDASE. | EC 1.5.3.6 |
| L-PIPECOLATE OXIDASE | EC 1.5.3.7 |
| RETICULATE OXIDASE | EC 1.5.3.9 |
| NITROETHANE OXIDASE | EC 1.7.3.1 |
| ACETYLINDOXYL OXIDASE. | EC 1.7.3.2 |
| URATE OXIDASE | EC 1.7.3.3 |
| HYDROXYLAMINE OXIDASE. | EC 1.7.3.4 |
| 3-ACYL-NITROPROPANOATE OXIDASE. | EC 1.7.3.5 |
| SULFITE OXIDASE | EC 1.8.3.1 |
| THIOL OXIDASE | EC 1.8.3.2 |
| GLUTATHIONE OXIDASE. | EC 1.8.3.3 |
| METHANETHIOL OXIDASE | EC 1.8.3.4 |
| PRENYLCYSTEINE OXIDASE. | EC 1.8.3.5 |

19. Procédé selon la revendication 16, **caractérisé en ce que** l'enzyme est choisie parmi les oxydoréductases à 4 électrons.

20. Procédé selon la revendication précédente, **caractérisé en ce que** l'enzyme est choisie parmi les enzymes du groupe EC 1.10.3 comme Catechol oxydase (polyphénol oxydase) EC 1.10.3.1, Laccase EC 1.10.3.2, L ascorbate oxydase EC 1.10.3.3 et Aminophenol oxydase EC 1.10.3.4., tyrosinase (monophenol oxydase) EC1.10.3.1 ou 1.14.18.1

21. Procédé selon la revendication 16, **caractérisé en ce que** l'enzyme est choisie parmi les peroxydases.

22. Procédé selon la revendication précédente, **caractérisé en ce que** l'enzyme est choisie parmi :
| | |
|---|---|
| NADH peroxidase. | E.C 1.11.1.1 |
| NADPH peroxidase. | E.C 1.11.1.2 |
| Fatty acid peroxidase. | E.C 1.11.1.3 |
| Cytochrome-c peroxidase. | E.C 1.11.1.5 |
| Peroxidase. | E.C 1.11.1.7 |
| Iodide peroxidase. | E.C 1.11.1.8 |
| Glutathione peroxidase. | E.C 1.11.1.9 |
| Chloride peroxidase. | E.C 1.11.1.10 |
| L-ascorbate peroxidase. | E.C 1.11.1.11 |
| Phospholipid-hydroperoxide glutathione peroxidase. | E.C 1.11.1.12 |
| Manganese peroxidase. | E.C 1.11.1.13 |
| Diarylpropane peroxidase. | E.C 1.11.1.14 |
| Tryptophan 2,3-dioxygenase. | E.C 1.13.11.11 |
| Lipoxygenase. | E.C 1.13.11.12 |

23. Procédé de coloration de fibres kératiniques, notamment humaines, dans lequel on met en oeuvre les étapes suivantes :
a) on applique sur les fibres sèches ou humides, une composition tinctoriale comprenant composé azine de formule (VI) et/ou (VII) : 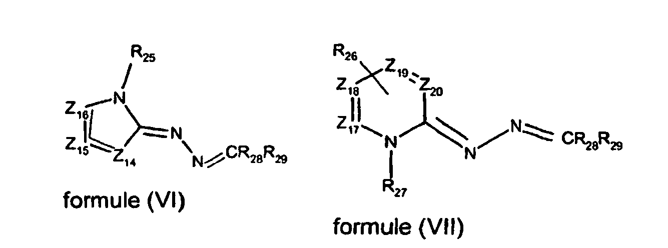 dans lesquelles :
- Z₁₄, Z₁₅, Z₁₆, R₂₅ ont respectivement les mêmes significations que Z₇, Z₈, Z₁₃, R₅ de la formule (IV), ou que Z₁, Z₂, Z₀, R₁ de la formule (II), définies ci-dessus,
- Z₁₇, Z₁₈, Z₁₉, Z₂₀, R₂₆, R₂₇ ont respectivement les mêmes significations que Z₉, Z₁₀, Z₁₁, Z₁₂, R₈, R₇ de la formule (V), ou que Z₃, Z₄, Z₅, Z₆, R₃, R₄ de la formule (III), définies ci-dessus,
- R₂₈, R₂₉, indépendamment l'un de l'autre, représente un atome d'hydrogène, un radical alkyle en C₁-C₆, de préférence en C₁-C₄, un radical alcoxy, linéaire ou ramifié, en C₁-C₆, de préférence en C₁-C₄, à la condition que l'un au moins des deux radicaux soit différent de l'hydrogène,
en présence d'un agent oxydant;
b) on laisse poser pendant une durée suffisante pour développer la coloration désirée.

24. Procédé de coloration selon la revendication 23, **caractérisé par le fait que** l'agent oxydant est mélangé au moment de l'emploi à la composition tinctoriale.

25. Procédé de coloration selon l'une quelconque des revendications 23 ou 24, **caractérisé par le fait que** l'agent oxydant est appliqué sur les fibres, simultanément ou séquentiellement à la composition tinctoriale.

26. Procédé de coloration selon l'une des revendications 23 à 25, **caractérisé par le fait que** la composition tinctoriale comprend une teneur en composés de formule (VI) et/ou (VII) comprise entre 0,001 à 10 % en poids, de préférence de 0,05 à 5 % en poids, par rapport au poids total de la composition tinctoriale.

27. Procédé de coloration selon l'une des revendications 23 à 26, **caractérisé en ce que** la composition tinctoriale comprend au moins une base d'oxydation et optionnellement au moins un coupleur, et/ou au moins un colorant direct.

28. Procédé de coloration selon l'une des revendications 23 à 27, **caractérisé en ce que** la composition comprend au moins une base d'oxydation choisie parmi les paraphénylènediamines, les bis-phénylalkylènediamines, les para-aminophénols, les ortho-aminophénols, les bases hétérocycliques et leurs sels d'addition.

29. Procédé de coloration selon la revendication 28, **caractérisé en ce que** la composition tinctoriale comprend une teneur en base d'oxydation comprise entre 0,001 et 10% en poids par rapport au poids total de la composition tinctoriale.

30. Procédé de coloration selon l'une des revendications 23 à 29, **caractérisé en ce qu**'elle comprend au moins un coupleur choisi dans le groupe formé par les métaphénylènediamines, les métaaminophénols, les métadiphénols, les coupleurs naphtaléniques, les coupleurs hétérocycliques ainsi que leurs sels d'addition.

31. Procédé de coloration selon la revendication 30, **caractérisé en ce que** la composition tinctoriale comprend une teneur en coupleurs comprise entre 0,001 et 10 % en poids par rapport au poids total de la composition tinctoriale.

32. Procédé de coloration selon l'une des revendications 23 à 31, **caractérisé en ce que** la composition tinctoriale comprend au moins un colorant direct.

33. Procédé de coloration selon la revendication 32 **caractérisé, en ce que** la composition tinctoriale comprend une teneur colorant direct comprise entre 0,001 et 20% en poids par rapport au poids total de la composition tinctoriale.

## Claims

1. Process for preparing tetraazapentamethine compounds of formula (I) below: 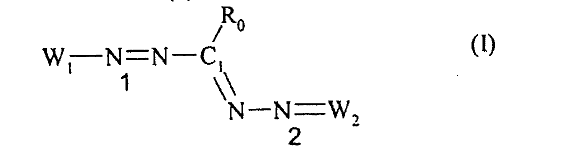 in which
- W₁ represents a cationic heteroaromatic radical of formula (II) or (III): 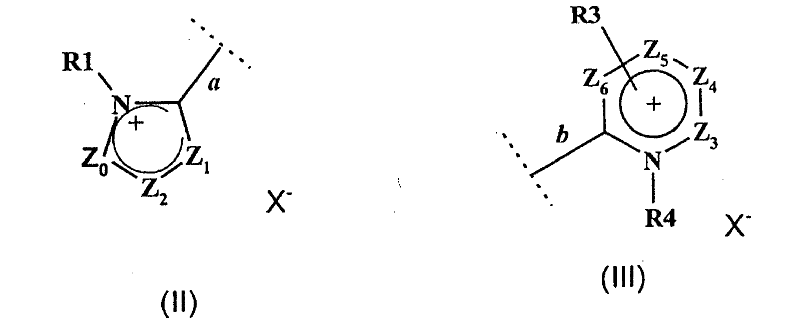
- W₂ represents a heteroaromatic radical of formula (IV) or (V): 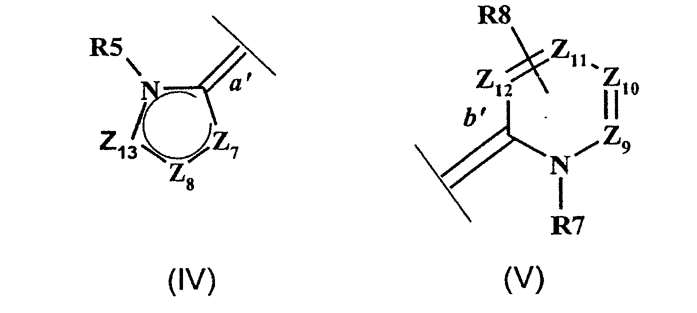
in which:
- Z₀ represents a radical CR₂, a nitrogen atom or a radical NR₂₁,
- Z₁ represents an oxygen or sulfur atom or a radical NR₉,
- Z₂ represents a nitrogen atom or a radical CR₁₀,
- Z₃ represents a nitrogen atom or a radical CR₁₁,
- Z₄ represents a nitrogen atom or a radical CR₁₂,
- Z₅ represents a nitrogen atom or a radical CR₁₃,
- Z₆ represents a nitrogen atom or a radical CR₁₄,
- Z₇ represents an oxygen or sulfur atom or a radical NR₁₅,
- Z₈ represents a nitrogen atom or a radical CR₁₆,
- Z₉ represents a nitrogen atom or a radical CR₁₇,
- Z₁₀ represents a nitrogen atom or a radical CR₁₈,
- Z₁₁ represents a nitrogen atom or a radical CR₁₉,
- Z₁₂ represents a nitrogen atom or a radical CR₂₀,
- Z₁₃ represents a radical CR₆, a nitrogen atom or a radical NR₂₂,
- it being understood that each of the rings of formulae (II), (III), (IV) and (V) comprise not more than three nitrogen atoms and that two of the three nitrogen atoms may be contiguous,
- the bond a of the 5-membered cationic heteroaromatic radical of formula (II) being linked to the nitrogen atom N₁ of formula (I),
- the bond b of the 6-membered cationic heteroaromatic radical of formula (III) being linked to the nitrogen atom N₁ of formula (I),
- the double bond a' of the 5-membered heteroaromatic radical of formula (IV) being linked to the nitrogen atom N₂ of formula (I),
- the double bond b' of the 6-membered heteroaromatic radical of formula (V) being linked to the nitrogen atom N₂ of formula (I),
- the bond b, linking the cationic heteroaromatic radical of formula (III) to the nitrogen atom N₁ of formula (I), being in an ortho or para position relative to the nitrogen atom bearing the radical R₄ when Z₅ represents a radical CR₁₃; the bond b being in an ortho position relative to the nitrogen atom bearing the radical R₄ when Z₅ represents a nitrogen atom,
- the bond b', linking the heteroaromatic radical of formula (V) to the nitrogen atom N₂ of formula (I), being in an ortho or para position relative to the nitrogen atom bearing the radical R₇ when Z₁₁ represents a radical CR₁₉; the bond b' being in an ortho position relative to the nitrogen atom bearing the radical R₇ when Z₁₁ represents a nitrogen atom,
- R₂, R₆, R₁₀ and R₁₆ represent, independently of each other, a hydrogen atom; a linear or branched C₁-C₄ alkyl radical, which is unsubstituted or substituted with one to three radicals chosen from hydroxyl, C₁-C₂ alkoxy, C₂-C₄ (poly)hydroxyalkoxy, amino, C₁-C₂ (di)alkylamino, carboxyl and sulfonic radicals; a phenyl radical, which is unsubstituted or substituted with one or two radicals chosen from hydroxyl, C₁-C₂ alkoxy, C₂-C₄ (poly) hydroxyalkoxy, amino and C₁-C₂ (di)alkylamino radicals; a carboxyl radical; a sulfonylamino radical,
- R₁, R₄, R₅, R₇, R₉, R₁₅, R₂₁ and R₂₂ represent, independently of each other, a linear or branched C₁-C₈ alkyl radical, which is unsubstituted or substituted with one to three radicals chosen from hydroxyl, C₁-C₂ alkoxy, C₂-C₄ (poly)hydroxyalkoxy, amino, C₁-C₂ (di)alkylamino, carboxyl and sulfonic radicals;
- R₀, R₃, R₈, R₁₁, R₁₂, R₁₃, R₁₄, R₁₇, R₁₈, R₁₉ and R₂₀ represent, independently of each other, a hydrogen atom, a linear or branched C₁-C₁₆ hydrocarbon-based chain, this chain possibly being saturated or unsaturated with one to three unsaturations, this chain being unsubstituted or substituted with one to three radicals chosen from hydroxyl, C₁-C₂ alkoxy, C₂-C₄ (poly) hydroxyalkoxy, amino, C₁-C₂ (di)alkylamino, carboxyl, sulfonic, sulfonylamino and C₂-C₄ (poly)hydroxyalkylamino radicals or a halogen atom such as chlorine, fluorine or bromine; a phenyl radical, which is unsubstituted or substituted with one to three radicals chosen from hydroxyl, C₁-C₂ alkoxy, C₂-C₄ (poly) hydroxyalkoxy, amino, C₁-C₂ (di)alkylamino, carboxyl, sulfonic, sulfonylamino and C₂-C₄ (poly)hydroxy-alkylamino radicals or a halogen atom such as chlorine, fluorine or bromine; a heteroaryl radical chosen from pyrazolyl, pyrrolyl, imidazolyl, thiazolyl, oxazolyl, triazolyl, pyridyl, pyrimidinyl, triazinyl, pyrazinyl and pyridazinyl radicals; in addition, this hydrocarbon-based chain may be interrupted with one or two oxygen, nitrogen or sulfur atoms or with an SO₂ radical,
- it being understood that R₀, R₃, R₈, R₁₁, R₁₂, R₁₃, R₁₄, R₁₇, R₁₈, R₁₉ and R₂₀ do not comprise a peroxide bond or diazo or nitroso radicals,
- R₂ with R₁₀, R₁₁ with R₁₂, R₆ with R₁₆, and, R₁₇ with R₁₈ can form, independently of each other, a 5- or 6-membered carbon-based aromatic ring, which is unsubstituted or substituted with one or two hydroxyl, amino, (di) (C₁-C₂) alkylamino, C₁-C₂ alkoxy or C₂-C₄ (poly)hydroxyalkylamino radicals,
- X is an organic or mineral anion;
consisting in reacting, in the presence of at least one oxidizing agent, at least one azine compound of formula (VI) and/or (VII): 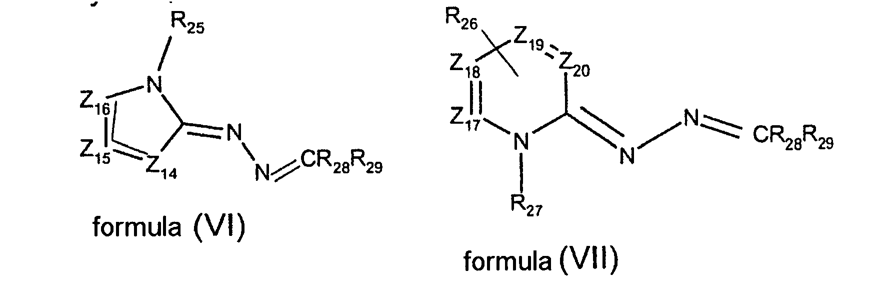 in which:
- Z₁₄, Z₁₅, Z₁₆ and R₂₅ respectively have the same meanings as Z₇, Z₈, Z₁₃ and R₅ of formula (IV) , or as Z₁, Z₂, Z₀ and R₁ of formula (II), defined above,
- Z₁₇, Z₁₈, Z₁₉, Z₂₀, R₂₆ and R₂₇ respectively have the same meanings as Z₉, Z₁₀, Z₁₁, Z₁₂, R₈ and R₇ of formula (V), or as Z₃, Z₄, Z₅, Z₆, R₃ and R₄ of formula (III) defined above,
- R₂₈ and R₂₉, independently of each other, represent a hydrogen atom, a C₁-C₆ and preferably C₁-C₄ alkyl radical, or a linear or branched C₁-C₆ and preferably C₁-C₄ alkoxy radical, on condition that at least one of the two radicals is other than hydrogen.

2. Process according to Claim 1, in which R₀ represents a hydrogen atom; a linear or branched C₁-C₆ alkyl radical, which is unsubstituted or substituted with one to three radicals chosen from hydroxyl, C₁-C₂ alkoxy, C₂-C₄ (poly) hydroxyalkoxy, amino, C₁-C₂ (di)alkylamino, C₂-C₄ (poly)hydroxyalkylamino, carboxyl and sulfonic radicals; a phenyl radical, which is unsubstituted or substituted with one to three radicals chosen from hydroxyl, C₁-C₂ alkoxy, C₂-C₄ (poly)hydroxyalkoxy, amino, C₁-C₂ (di)alkylamino, carboxyl and sulfonic radicals or a halogen atom such as chlorine, fluorine or bromine; an optionally cationic heteroaryl radical chosen from pyrazolyl, pyrrolyl, imidazolyl, thiazolyl, oxazolyl, triazolyl, pyridyl, pyrimidinyl, triazinyl, pyrazinyl and pyridazinyl radicals.

3. Process according to either of Claims 1 and 2, **characterized in that**, in formula (I), R₂, R₆, R₁₀ and R₁₆ represent a hydrogen atom, a C₁-C₄ alkyl radical, which is unsubstituted or substituted with one or two radicals chosen from hydroxyl, amino, C₁-C₂ (di)alkylamino and carboxyl radicals, or a phenyl radical.

4. Process according to one of Claims 1 to 3, **characterized in that**, in formula (I), R₁, R₄, R₅, R₇, R₉, R₁₅, R₂₁ and R₂₂ preferably represent a C₁-C₄ alkyl radical, which is unsubstituted or substituted with one or two radicals chosen from hydroxyl, C₁-C₂ alkoxy, amino, C₁-C₂ (di)alkylamino, carboxyl and sulfonic radicals.

5. Process according to one of Claims 1 to 4, **characterized in that**, in formula (I), R₃, R₈, R₁₁, R₁₂, R₁₃, R₁₄, R₁₇, R₁₈, R₁₉ and R₂₀ represent, independently of each other, a hydrogen atom; a linear or branched C₁-C₄ alkyl radical, which is unsubstituted or substituted with one or two radicals chosen from hydroxyl, C₁-C₂ alkoxy, C₂-C₄ (poly)hydroxyalkoxy, amino, C₁-C₂ (di)alkylamino, carboxyl and sulfonic radicals; a phenyl radical, which is unsubstituted or substituted with one or two radicals chosen from hydroxyl, C₁-C₂ alkoxy, C₂-C₄ (poly)hydroxyalkoxy, amino, C₁-C₂. (di)alkylamino, carboxyl and sulfonic radicals, or a halogen atom such as chlorine, fluorine or bromine; a sulfonylamino radical; a C₂-C₄ (poly)hydroxyalkylamino radical.

6. Process according to one of Claims 1 to 5, **characterized in that** the compound of formula (I), in which W₁ is a 2-pyridinium radical and W₂ is a 2-pyridine radical, is chosen from:
- 2-[5-(N-methyl-2-pyridinylidene)-1-formazano]-N-methylpyridinium chloride
- 2-[5-(N-methyl-2-pyridinylidene)-3-methyl-1-formazano]-N-methylpyridinium chloride
- 2-[5-(N-methyl-2-pyridinylidene)-3-ethyl-1-formazano]-N-methylpyridinium chloride
- 2-[5-(N-methyl-2-pyridinylidene)-3-isopropyl-1-formazano]-N-methylpyridinium chloride
- 2-[5-(N-methyl-2-pyridinylidene)-3-phenyl-1-formazano]-N-methylpyridinium chloride
- 2-[5-(N-methyl-2-pyridinylidene)-3-(4'-methoxyphenyl)-1-formazano]-N-methylpyridinium chloride
- 2-[5-(N-methyl-2-pyridinylidene)-3-(4'-hydroxyphenyl)-1-formazano]-N-methylpyridinium chloride
- 2-[5-(N-methyl-2-pyridinylidene)-3-(4'-N,N-dimethylaminophenyl)-1-formazano]-N-methylpyridinium chloride
- 2-[5-(4-pyrrolidino-N-methyl-2-pyridinylidene)-1-formazano]-4-pyrrolidino-N-methylpyridinium chloride
- 2-[5-(4-pyrrolidino-N-methyl-2-pyridinylidene)-3-methyl-1-formazano]-4-pyrrolidino-N-methylpyridinium chloride
- 2-[5-(4-pyrrolidino-N-methyl-2-pyridinylidene)-3-ethyl-1-formazano]-4-pyrrolidino-N-methylpyridinium chloride
- 2-[5-(4-pyrrolidino-N-methyl-2-pyridinylidene)-3-isopropyl-1-formazano]-4-pyrrolidino-N-methylpyridinium chloride
- 2-[5-(4-pyrrolidino-N-methyl-2-pyridinylidene)-3-phenyl-1-formazano]-4-pyrrolidino-N-methylpyridinium chloride
- 2-[5-(4-pyrrolidino-N-methyl-2-pyridinylidene)-3-(4'-methoxyphenyl)-1-formazano]-4-pyrrolidino-N-methylpyridinium chloride
- 2-[5-(4-pyrrolidino-N-methyl-2-pyridinylidene)-3-(4'-hydroxyphenyl)-1-formazano]-4-pyrrolidino-N-methylpyridinium chloride
- 2-[5-(4-pyrrolidino-N-methyl-2-pyridinylidene)-3-(4'-N,N-dimethylaminophenyl)-1-formazano]-4-pyrrolidino-N-methylpyridinium chloride
- 2-[5-(N-hydroxyethyl-2-pyridinylidene)-1-formazano]-N-hydroxyethylpyridinium chloride
- 2-[5-(N-hydroxyethyl-2-pyridinylidene)-3-methyl-1-formazano]-N-hydroxyethylpyridinium chloride
- 2-[5-(N-hydroxyethyl-2-pyridinylidene)-3-ethyl-1-formazano]-N-hydroxyethylpyridinium chloride
- 2-[5-(N-hydroxyethyl-2-pyridinylidene)-3-phenyl-1-formazano]-N-hydroxyethylpyridinium chloride
- 2-[5-(N-hydroxyethyl-2-pyridinylidene)-3-isopropyl-1-formazano]-N-hydroxyethylpyridinium chloride
- 2-[5-(N-hydroxyethyl-2-pyridinylidene)-3-(4'-methoxyphenyl)-1-formazano]-N-hydroxyethylpyridinium chloride
- 2-[5-(N-hydroxyethyl-2-pyridinylidene)-3-(4'-hydroxyphenyl)-1-formazano]-N-hydroxyethylpyridinium chloride
- 2-[5-(N-hydroxyethyl-2-pyridinylidene)-3-(4'-N,N-dimethylaminophenyl)-1-formazano]-N-hydroxyethyl-pyridinium chloride
- 2-[5-(N-carboxyethyl-2-pyridinylidene)-1-formazano]-N-carboxyethylpyridinium chloride
- 2-[5-(N-carboxyethyl-2-pyridinylidene)-3-methyl-1-formazano]-N-carboxyethylpyridinium chloride
- 2-[5-(N-carboxyethyl-2-pyridinylidene)-3-ethyl-1-formazano]-N-carboxyethylpyridinium chloride
- 2-[5-(N-carboxyethyl-2-pyridinylidene)-3-phenyl-1-formazano]-N-carboxyethylpyridinium chloride
- 2-[5-(N-carboxyethyl-2-pyridinylidene)-3-isopropyl-1-formazano]-N-carboxyethylpyridinium chloride
- 2-[5-(N-carboxyethyl-2-pyridinylidene)-3-(4'-methoxyphenyl)-1-formazano]-N-carboxyethylpyridinium chloride
- 2-[5-(N-carboxyethyl-2-pyridinylidene)-3-(4'-hydroxyphenyl)-1-formazano]-N-carboxyethylpyridinium chloride
- 2-[5-(N-carboxyethyl-2-pyridinylidene)-3-(4'-N,N-dimethylaminophenyl)-1-formazano]-N-carboxyethyl-pyridinium chloride.

7. Process according to one of Claims 1 to 6, **characterized in that** the compound of formula (I), in which W₁ is a 4-pyridinium radical and W₂ is a 4-pyridine radical, is chosen from:
- 4-[5-(N-methyl-4-pyridinylidene)-1-formazano]-N-methylpyridinium chloride
- 4-[5-(N-methyl-4-pyridinylidene)-3-methyl-1-formazano]-N-methylpyridinium chloride
- 4-[5-(N-methyl-4-pyridinylidene)-3-ethyl-1-formazano]-N-methylpyridinium chloride
- 4-[5-(N-methyl-4-pyridinylidene)-3-isopropyl-1-formazano]-N-methylpyridinium chloride
- 4-[5-(N-methyl-4-pyridinylidene)-3-phenyl-1-formazano]-N-methylpyridinium chloride
- 4-[5-(N-methyl-4-pyridinylidene)-3-(4'-methoxyphenyl)-1-formazano]-N-methylpyridinium chloride
- 4-[5-(N-methyl-4-pyridinylidene)-3-(4'-hydroxyphenyl)-1-formazano]-N-methylpyridinium chloride
- 4-[5-(N-methyl-4-pyridinylidene)-3-(4'-N,N-dimethylaminophenyl)-1-formazano]-N-methylpyridinium chloride
- 4-[5-(N-hydroxyethyl-4-pyridinylidene)-1-formazano]-N-hydroxyethylpyridinium chloride
- 4-[5-(N-hydroxyethyl-4-pyridihylidene)-3-methyl-1-formazano]-N-hydroxyethylpyridinium chloride
- 4-[5-(N-hydroxyethyl-4-pyridinylidene)-3-ethyl-1-formazano]-N-hydroxyethylpyridinium chloride
- 4-[5-(N-hydroxyethyl-4-pyridinylidene)-3-phenyl-1-formazano]-N-hydroxyethylpyridinium chloride
- 4-[5-(N-hydroxyethyl-4-pyridinylidene)-3-isopropyl-1-formazano]-N-hydroxyethylpyridinium chloride
- 4-[5-(N-hydroxyethyl-4-pyridinylidene)-3-(4'-methoxyphenyl)-1-formazano]-N-hydroxyethylpyridinium chloride
- 4-[5-(N-hydroxyethyl-4-pyridinylidene)-3-(4'-hydroxyphenyl)-1-formazano]-N-hydroxyethylpyridinium chloride
- 4-[5-(N-hydroxyethyl-4-pyridinylidene)-3-(4'-N,N-dimethylaminophenyl)-1-formazano]-N-hydroxyethyl-pyridinium chloride
- 4-[5-(N-carboxyethyl-4-pyridinylidene)-1-formazano]-N-carboxyethylpyridinium chloride
- 4-[5-(N-carboxyethyl-4-pyridinylidene)-3-methyl-1-formazano]-N-carboxyethylpyridinium chloride
- 4-[5-(N-carboxyethyl-4-pyridinylidene)-3-ethyl-1-formazano]-N-carboxyethylpyridinium chloride
- 4-[5-(N-carboxyethyl-4-pyridinylidene)-3-phenyl-1-formazano]-N-carboxyethylpyridinium chloride
- 4-[5-(N-carboxyethyl-4-pyridinylidene)-3-isopropyl-1-formazano]-N-carboxyethylpyridinium chloride
- 4-[5-(N-carboxyethyl-4-pyridinylidene)-3-(4'-methoxyphenyl)-1-formazano]-N-carboxyethylpyridinium chloride
- 4-[5-(N-carboxyethyl-4-pyridinylidene)-3-(4'-hydroxyphenyl)-1-formazano]-N-carboxyethylpyridinium chloride
- 4-[5-(N-carboxyethyl-4-pyridinylidene)-3-(4'-N,N-dimethylaminophenyl)-1-formazano]-N-carboxyethyl-pyridinium chloride.

8. Process according to one of Claims 1 to 7, **characterized in that** the compound of formula (I), in which W₁ is a 2-imidazolium radical and W₂ is a 2-imidazole radical, is chosen from:
- 2-[5-(1,3-dimethyl-2-imidazolidene)-1-formazano]-1,3-dimethylimidazolium chloride
- 2-[5-(1,3-dimethyl-2-imidazolidene)-3-methyl-1-formazano]-1,3-dimethylimidazolium chloride
- 2-[5-(1,3-dimethyl-2-imidazolidene)-3-ethyl-1-formazano]-1,3-dimethylimidazolium chloride
- 2-[5-(1,3-dimethyl-2-imidazolidene)-3-isopropyl-1-formazano]-1,3-dimethylimidazolium chloride
- 2-[5-(1,3-dimethyl-2-imidazolidene)-3-phenyl-1-formazano]-1,3-dimethylimidazolium chloride
- 2-[5-(1,3-dimethyl-2-imidazolidene)-3-(4'-methoxyphenyl)-1-formazano]-1,3-dimethylimidazolium chloride
- 2-[5-(1,3-dimethyl-2-imidazolidene)-3-(4'-hydroxyphenyl)-1-formazano]-1,3-dimethylimidazolium chloride
- 2-[5-(1,3-dimethyl-2-imidazolidene)-3-(4'-N,N-dimethylaminophenyl)-1-formazano]-1,3-dimethylimidazolium chloride
- 2-[5-(1,3-dihydroxyethyl-2-imidazolidene)-1-formazano]-1,3-dihydroxyethylimidazolium chloride
- 2-[5-(1,3-dihydroxyethyl-2-imidazolidene)-3-methyl-1-formazano]-1,3-dihydroxyethylimidazolium chloride
- 2-[5-(1,3-dihydroxyethyl-2-imidazolidene)-3-ethyl-1-formazano]-1,3-dihydroxyethylimidazolium chloride
- 2-[5-(1,3-dihydroxyethyl-2-imidazolidene)-3-phenyl-1-formazano]-1,3-dihydroxyethylimidazolium chloride
- 2-[5-(1,3-dihydroxyethyl-2-imidazolidene)-3-isopropyl-1-formazano]-1,3-dihydroxyethylimidazolium chloride
- 2-[5-(1,3-dihydroxyethyl-2-imidazolidene)-3-(4'-methoxyphenyl)-1-formazano]-1,3-dihydroxyethylimidazolium chloride
- 2-[5-(1,3-dihydroxyethyl-2-imidazolidene)-3-(4'-hydroxyphenyl)-1-formazano]-1,3-dihydroxyethylimidazolium chloride
- 2-[5-(1,3-dihydroxyethyl-2-imidazolidene)-3-(4'-N,N-dimethylaminophenyl)-1-formazano]-1,3-dihydroxyethylimidazolium chloride
- 2-[5-(1,3-dicarboxyethyl-2-imidazolidene)-1-formazano]-1,3-dicarboxyethylimidazolium chloride
- 2-[5-(1,3-dicarboxyethyl-2-imidazolidene)-3-methyl-1-formazano]-1,3-dicarboxyethylimidazolium chloride
- 2-[5-(1,3-dicarboxyethyl-2-imidazolidene)-3-ethyl-1-formazano]-1,3-dicarboxyethylimidazolium chloride
- 2-[5-(1,3-dicarboxyethyl-2-imidazolidene)-3-phenyl-1-formazano]-1,3-dicarboxyethylimidazolium chloride
- 2-[5-(1,3-dicarboxyethyl-2-imidazolidene)-3-isopropyl-1-formazano]-1,3-dicarboxyethylimidazolium chloride
- 2-[5-(1,3-dicarboxyethyl-2-imidazolidene)-3-(4'-methoxyphenyl)-1-formazano]-1,3-dicarboxyethylimidazolium chloride
- 2-[5-(1,3-dicarboxyethyl-2-imidazolidene)-3-(4'-hydroxyphenyl)-1-formazano]-1,3-dicarboxyethylimidazolium chloride
- 2-[5-(1,3-dicarboxyethyl-2-imidazolidene)-3-(4'-N,N-dimethylaminophenyl)-1-formazano]-1,3-dicarboxyethylimidazolium chloride.

9. Process according to one of Claims 1 to 8, **characterized in that** the compound of formula (I), in which W₁ is a 5-pyrazolium radical and W₂ is a 5-pyrazole radical, is chosen from:
- 5-[5-(1,2-dimethyl-5-pyrazolidene)-1-formazano]-1,2-dimethylpyrazolinium chloride
- 5-[5-(1,2-dimethyl-5-pyrazolidene)-3-methyl-1-formazano]-1,2-dimethylpyrazolinium chloride
- 5-[5-(1,2-dimethyl-5-pyrazolidene)-3-ethyl-1-formazano]-1,2-dimethylpyrazolinium chloride
- 5-[5-(1,2-dimethyl-5-pyrazolidene)-3-isopropyl-1-formazano]-1,2-dimethylpyrazolinium chloride
- 5-[5-(1,2-dimethyl-5-pyrazolidene)-3-phenyl-1-formazano]-1,2-dimethylpyrazolinium chloride
- 5-[5-(1,2-dimethyl-5-pyrazolidene)-3-(4'-methoxyphenyl)-1-formazano]-1,2-dimethylpyrazolinium chloride
- 5-[5-(1,2-dimethyl-5-pyrazolidene)-3-(4'-hydroxyphenyl)-1-formazano]-1,2-dimethylpyrazolinium chloride
- 5-[5-(1,2-dimethyl-5-pyrazolidene)-3-(4'-N,N-dimethylaminophenyl)-1-formazano]-1,2-dimethyl-pyrazolinium chloride
- 5-[5-(1,2-dihydroxyethyl-5-pyrazolidene)-1-formazano]-1,2-dihydroxyethylpyrazolinium chloride
- 5-[5-(1,2-dihydroxyethyl-5-pyrazolidene)-3-methyl-1-formazano]-1,2-dihydroxyethylpyrazolinium chloride
- 5-[5-(1,2-dihydroxyethyl-5-pyrazolidene)-3-ethyl-1-formazano]-1,2-dihydroxyethylpyrazolinium chloride
- 5-[5-(1,2-dihydroxyethyl-5-pyrazolidene)-3-phenyl-1-formazano]-1,2-dihydroxyethylpyrazolinium chloride
- 5-[5-(1,2-dihydroxyethyl-5-pyrazolidene)-3-isopropyl-1-formazano]-1,2-dihydroxyethylpyrazolinium chloride
- 5-[5-(1,2-dihydroxyethyl-5-pyrazolidene)-3-(4'-methoxyphenyl)-1-formazano]-1,2-dihydroxyethyl-pyrazolinium chloride
- 5-[5-(1,2-dihydroxyethyl-5-pyrazolidene)-3-(4'-hydroxyphenyl)-1-formazano]-1,2-dihydroxyethyl-pyrazolinium chloride
- 5-[5-(1,2-dihydroxyethyl-5-pyrazolidene)-3-(4'-N,N-dimethylaminophenyl)-1-formazano]-1,2-dihydroxy-ethylpyrazolinium chloride
- 5-[5-(1,2-dicarboxyethyl-5-pyrazolidene)-1-formazano]-1,2-dicarboxyethylpyrazolinium chloride
- 5-[5-(1,2-dicarboxyethyl-5-pyrazolidene)-3-methyl-1-formazano]-1,2-dicarboxyethylpyrazolinium chloride
- 5-[5-(1,2-dicarboxyethyl-5-pyrazolidene)-3-ethyl-1-formazano]-1,2-dicarboxyethylpyrazolinium chloride
- 5-[5-(1,2-dicarboxyethyl-5-pyrazolidene)-3-phenyl-1-formazano]-1,2-dicarboxyethylpyrazolinium chloride
- 5-[5-(1,2-dicarboxyethyl-5-pyrazolidene)-3-isopropyl-1-formazano]-1,2-dicarboxyethylpyrazolinium chloride
- 5-[5-(1,2-dicarboxyethyl-5-pyrazolidene)-3-(4'-methoxyphenyl)-1-formazano]-1,2-dicarboxyethyl-pyrazolinium chloride
- 5-[5-(1,2-dicarboxyethyl-5-pyrazolidene)-3-(4'-hydroxyphenyl)-1-formazano]-1,2-dicarboxyethyl-pyrazolinium chloride
- 5-[5-(1,2-dicarboxyethyl-5-pyrazolidene)-3-(4'-N,N-dimethylaminophenyl)-1-formazano]-1,2-dicarboxy-ethylpyrazolinium chloride.

10. Process according to one of Claims 1 to 9, **characterized in that** the compound of formula (I), in which W₁ is a 2-benzimidazolium radical and W₂ is a 2-pyridine radical, is chosen from:
- 2-[5-(N-methyl-2-pyridinylidene)-1-formazano]-1,3-dimethylbenzimidazolium chloride
- 2-[5-(N-methyl-2-pyridinylidene)-3-methyl-1-formazano]-1,3-dimethylbenzimidazolium chloride
- 2-[5-(N-methyl-2-pyridinylidene)-3-ethyl-1-formazano]-1,3-dimethylbenzimidazolium chloride
- 2-[5-(N-methyl-2-pyridinylidene)-3-isopropyl-1-formazano]-1,3-dimethylbenzimidazolium chloride
- 2-[5-(N-methyl-2-pyridinylidene)-3-phenyl-1-formazano]-1,3-dimethylbenzimidazolium chloride
- 2-[5-(N-methyl-2-pyridinylidene)-3-(4'-methoxyphenyl)-1-formazano]-1,3-dimethylbenzimidazolium chloride
- 2-[5-(N-methyl-2-pyridinylidene)-3-(4'-hydroxyphenyl)-1-formazano]-1,3-dimethylbenzimidazolium chloride
- 2-[5-(N-methyl-2-pyridinylidene)-3-(4'-N,N-dimethylaminophenyl)-1-formazano]-1,3-dimethylbenzimidazolium chloride
- 2-[5-(N-hydroxyethyl-2-pyridinylidene)-1-formazano]-1,3-dihydroxyethylbenzimidazolium chloride
- 2-[5-(N-hydroxyethyl-2-pyridinylidene)-3-methyl-1-formazano]-1,3-dihydroxyethylbenzimidazolium chloride
- 2-[5-(N-hydroxyethyl-2-pyridinylidene)-3-ethyl-1-formazano]-1,3-dihydroxyethylbenzimidazolium chloride
- 2-[5-(N-hydroxyethyl-2-pyridinylidene)-3-phenyl-1-formazano]-1,3-dihydroxyethylbenzimidazolium chloride
- 2-[5-(N-hydroxyethyl-2-pyridinylidene)-3-isopropyl-1-formazano]-1,3-dihydroxyethyl-benzimidazolium chloride
- 2-[5-(N-hydroxyethyl-2-pyridinylidene)-3-(4'-methoxyphenyl)-1-formazano]-1,3-dihydroxyethyl-benzimidazolium chloride
- 2-[5-(N-hydroxyethyl-2-pyridinylidene)-3-(4'-hydroxyphenyl)-1-formazano]-1,3-dihydroxyethyl-benzimidazolium chloride
- 2-[5-(N-hydroxyethyl-2-pyridinylidene)-3-(4'-N,N-dimethylaminophenyl)-1-formazano]-1,3-dihydroxyethyl-benzimidazolium chloride
- 2-[5-(N-carboxyethyl-2-pyridinylidene)-1-formazano]-1,3-dicarboxyethylbenzimidazolium chloride
- 2-[5-(N-carboxyethyl-2-pyridinylidene)-3-methyl-1-formazano]-1,3-dicarboxyethylbenzimidazolium chloride
- 2-[5-(N-carboxyethyl-2-pyridinylidene)-3-ethyl-1-formazano]-1,3-dicarboxyethylbenzimidazolium chloride
- 2-[5-(N-carboxyethyl-2-pyridinylidene)-3-phenyl-1-formazano]-1,3-dicarboxyethylbenzimidazolium chloride
- 2-[5-(N-carboxyethyl-2-pyridinylidene)-3-isopropyl-1-formazano]-1,3-dicarboxyethylbenzimidazolium chloride
- 2-[5-(N-carboxyethyl-2-pyridinylidene)-3,4'-methoxyphenyl)-1-formazano]-1,3-dicarboxyethylbenzimidazolium chloride
- 2-[5-(N-carboxyethyl-2-pyridinylidene)-3-(4'-hydroxyphenyl)-1-formazano]-1,3-dicarboxyethylbenzimidazolium chloride
- 2-[5-(N-carboxyethyl-2-pyridinylidene)-3-(4'-N,N-dimethylaminophenyl)-1-formazano]-1,3-dicarboxyethylbenzimidazolium chloride.

11. Process according to one of Claims 1 to 10,
**characterized in that** the compound of formula (I), in which W₁ is a 2-benzimidazolium radical and W₂ is a 4-pyridine radical, is chosen from:
- 2-[5-(N-methyl-4-pyridinylidene)-1-formazano]-1,3-dimethylbenzimidazolium chloride
- 2-[5-(N-methyl-4-pyridinylidene)-3-methyl-1-formazano]-1,3-dimethylbenzimidazolium chloride
- 2-[5-(N-methyl-4-pyridinylidene)-3-ethyl-1-formazano]-1,3-dimethylbenzimidazolium chloride
- 2-[5-(N-methyl-4-pyridinylidene)-3-isopropyl-1-formazano]-1,3-dimethylbenzimidazolium chloride
- 2-[5-(N-methyl-4-pyridinylidene)-3-phenyl-1-formazano]-1,3-dimethylbenzimidazolium chloride
- 2-[5-(N-methyl-4-pyridinylidene)-3-(4'-methoxyphenyl)-1-formazano]-1,3-dimethylbenzimidazolium chloride
- 2-[5-(N-methyl-4-pyridinylidene)-3-(4'-hydroxyphenyl)-1-formazano]-1,3-dimethylbenzimidazolium chloride
- 2-[5-(N-methyl-4-pyridinylidene)-3-(4'-N,N-dimethylaminophenyl)-1-formazano]-1,3-dimethylbenzimidazolium chloride
- 2-[5-(N-hydroxyethyl-4-pyridinylidene)-1-formazano]-1,3-dihydroxyethylbenzimidazolium chloride
- 2-[5-(N-hydroxyethyl-4-pyridinylidene)-3-methyl-1-formazano]-1,3-dihydroxyethylbenzimidazolium chloride
- 2-[5-(N-hydroxyethyl-4-pyridinylidene)-3-ethyl-1-formazano]-1,3-dihydroxyethylbenzimidazolium chloride
- 2-[5-(N-hydroxyethyl-4-pyridinylidene)-3-phenyl-1-formazano]-1,3-dihydroxyethylbenzimidazolium chloride
- 2-[5-(N-hydroxyethyl-4-pyridinylidene)-3-isopropyl-1-formazano]-1,3-dihydroxyethyl-benzimidazolium chloride
- 2-[5-(N-hydroxyethyl-4-pyridinylidene)-3-(4'-methoxyphenyl)-1-formazano]-1,3-dihydroxyethyl-benzimidazolium chloride
- 2-[5-(N-hydroxyethyl-4-pyridinylidene)-3-(4'-hydroxyphenyl)-1-formazano]-1,3-dihydroxyethyl-benzimidazolium chloride
- 2-[5-(N-hydroxyethyl-4-pyridinylidene)-3-(4'-N,N-dimethylaminophenyl)-1-formazano]-1,3-dihydroxyethyl-benzimidazolium chloride
- 2-[5-(N-carboxyethyl-4-pyridinylidene)-1-formazano]-1,3-dicarboxyethylbenzimidazolium chloride
- 2-[5-(N-carboxyethyl-4-pyridinylidene)-3-methyl-1-formazano]-1,3-dicarboxyethylbenzimidazolium chloride
- 2-[5-(N-carboxyethyl-4-pyridinylidene)-3-ethyl-1-formazano]-1,3-dicarboxyethylbenzimidazolium chloride
- 2-[5-(N-carboxyethyl-4-pyridinylidene)-3-phenyl-1-formazano]-1,3-dicarboxyethylbenzimidazolium chloride
- 2-[5-(N-carboxyethyl-4-pyridinylidene)-3-isopropyl-1-formazano]-1,3-dicarboxyethylbenzimidazolium chloride
- 2-[5-(N-carboxyethyl-4-pyridinylidene)-3,4'-methoxyphenyl)-1-formazano]-1,3-dicarboxyethylbenzimidazolium chloride
- 2-[5-(N-carboxyethyl-4-pyridinylidene)-3-(4'-hydroxyphenyl)-1-formazano]-1,3-dicarboxyethylbenzimidazolium chloride
- 2-[5-(N-carboxyethyl-4-pyridinylidene)-3-(4'-N,N-dimethylaminophenyl)-1-formazano]-1,3-dicarboxyethylbenzimidazolium chloride.

12. Process according to one of Claims 1 to 11, **characterized in that** the compound of formula (I), in which W₁ is a 2-imidazolium radical and W₂ is a 2-pyridine radical, is chosen from:
- 2-[5-(N-methyl-2-pyridinylidene)-1-formazano]-1,3-dimethylimidazolium chloride
- 2-[5-(N-methyl-2-pyridinylidene)-3-methyl-1-formazano]-1,3-dimethylimidazolium chloride
- 2-[5-(N-methyl-2-pyridinylidene)-3-ethyl-1-formazano]-1,3-dimethylimidazolium chloride
- 2-[5-(N-methyl-2-pyridinylidene)-3-isopropyl-1-formazano]-1,3-dimethylimidazolium chloride
- 2-[5-(N-methyl-2-pyridinylidene)-3-phenyl-1-formazano]-1,3-dimethylimidazolium chloride
- 2-[5-(N-methyl-2-pyridinylidene)-3-(4'-methoxyphenyl)-1-formazano]-1,3-dimethylimidazolium chloride
- 2-[5-(N-methyl-2-pyridinylidene)-3-(4'-hydroxyphenyl)-1-formazano]-1,3-dimethylimidazolium chloride
- 2-[5-(N-methyl-2-pyridinylidene)-3-(4'-N,N-dimethylaminophenyl)-1-formazano]-1,3-dimethylimidazolium chloride
- 2-[5-(N-hydroxyethyl-2-pyridinylidene)-1-formazano]-1,3-dihydroxyethylimidazolium chloride
- 2-[5-(N-hydroxyethyl-2-pyridinylidene)-3-methyl-1-formazano]-1,3-dihydroxyethylimidazolium chloride
- 2-[5-(N-hydroxyethyl-2-pyridinylidene)-3-ethyl-1-formazano]-1,3-dihydroxyethylimidazolium chloride
- 2-[5-(N-hydroxyethyl-2-pyridinylidene)-3-phenyl-1-formazano]-1,3-dihydroxyethylimidazolium chloride
- 2-[5-(N-hydroxyethyl-2-pyridinylidene)-3-isopropyl-1-formazano]-1,3-dihydroxyethylimidazolium chloride
- 2-[5-(N-hydroxyethyl-2-pyridinylidene)-3-(4'-methoxyphenyl)-1-formazano]-1,3-dihydroxyethylimidazolium chloride
- 2-[5-(N-hydroxyethyl-2-pyridinylidene)-3-(4'-hydroxyphenyl)-1-formazano]-1,3-dihydroxyethylimidazolium chloride
- 2-[5-(N-hydroxyethyl-2-pyridinylidene)-3-(4'-N,N-dimethylaminophenyl)-1-formazano]-1,3-dihydroxyethylimidazolium chloride
- 2-[5-(N-carboxyethyl-2-pyridinylidene)-1-formazano]-1,3-dicarboxyethylimidazolium chloride
- 2-[5-(N-carboxyethyl-2-pyridinylidene)-3-methyl-1-formazano]-1,3-dicarboxyethylimidazolium chloride
- 2-[5-(N-carboxyethyl-2-pyridinylidene)-3-ethyl-1-formazano]-1,3-dicarboxyethylimidazolium chloride
- 2-[5-(N-carboxyethyl-2-pyridinylidene)-3-phenyl-1-formazano]-1,3-dicarboxyethylimidazolium chloride
- 2-[5-(N-carboxyethyl-2-pyridinylidene)-3-isopropyl-1-formazano]-1,3-dicarboxyethylimidazolium chloride
- 2-[5-(N-carboxyethyl-2-pyridinylidene)-3-(4'-methoxyphenyl)-1-formazano]-1,3-dicarboxyethylimidazolium chloride
- 2-[5-(N-carboxyethyl-2-pyridinylidene)-3-(4'-hydroxyphenyl)-1-formazano]-1,3-dicarboxyethylimidazolium chloride
- 2-[5-(N-carboxyethyl-2-pyridinylidene)-3-(4'-N,N-dimethylaminophenyl)-1-formazano]-1,3-dicarboxyethylimidazolium chloride.

13. Process according to one of Claims 1 to 12, **characterized in that** the compound of formula (I), in which W₁ is a 2-imidazolium radical and W₂ is a 4-pyridine radical, is chosen from:
- 2-[5-(N-methyl-4-pyridinylidene)-1-formazano]-1,3-dimethylimidazolium chloride
- 2-[5-(N-methyl-4-pyridinylidene)-3-methyl-1-formazano]-1,3-dimethylimidazolium chloride
- 2-[5-(N-methyl-4-pyridinylidene)-3-ethyl-1-formazano]-1,3-dimethylimidazolium chloride
- 2-[5-(N-methyl-4-pyridinylidene)-3-isopropyl-1-formazano]-1,3-dimethylimidazolium chloride
- 2-[5-(N-methyl-4-pyridinylidene)-3-phenyl-1-formazano]-1,3-dimethylimidazolium chloride
- 2-[5-(N-methyl-4-pyridinylidene)-3-(4'-methoxyphenyl)-1-formazano]-1,3-dimethylimidazolium chloride
- 2-[5-(N-methyl-4-pyridinylidene)-3-(4'-hydroxyphenyl)-1-formazano]-1,3-dimethylimidazolium chloride
- 2-[5-(N-methyl-4-pyridinylidene)-3-(4'-N,N-dimethylaminophenyl)-1-formazano]-1,3-dimethylimidazolium chloride
- 2-[5-(N-hydroxyethyl-4-pyridinylidene)-1-formazano]-1,3-dihydroxyethylimidazolium chloride
- 2-[5-(N-hydroxyethyl-4-pyridinylidene)-3-methyl-1-formazano]-1,3-dihydroxyethylimidazolium chloride
- 2-[5-(N-hydroxyethyl-4-pyridinylidene)-3-ethyl-1-formazano]-1,3-dihydroxyethylimidazolium chloride
- 2-[5-(N-hydroxyethyl-4-pyridinylidene)-3-phenyl-1-formazano]-1,3-dihydroxyethylimidazolium chloride
- 2-[5-(N-hydroxyethyl-4-pyridinylidene)-3-isopropyl-1-formazano]-1,3-dihydroxyethylimidazolium chloride
- 2-[5-(N-hydroxyethyl-4-pyridinylidene)-3-(4'-methoxyphenyl)-1-formazano]-1,3-dihydroxyethylimidazolium chloride
- 2-[5-(N-hydroxyethyl-4-pyridinylidene)-3-(4'-hydroxyphenyl)-1-formazano]-1,3-dihydroxyethylimidazolium chloride
- 2-[5-(N-hydroxyethyl-4-pyridinylidene)-3-(4'-N,N-dimethylaminophenyl)-1-formazano]-1,3-dihydroxyethylimidazolium chloride
- 2-[5-(N-carboxyethyl-4-pyridinylidene)-1-formazano]-1,3-dicarboxyethylimidazolium chloride
- 2-[5-(N-carboxyethyl-4-pyridinylidene)-3-methyl-1-formazano]-1,3-dicarboxyethylimidazolium chloride
- 2-[5-(N-carboxyethyl-4-pyridinylidene)-3-ethyl-1-formazano]-1,3-dicarboxyethylimidazolium chloride
- 2-[5-(N-carboxyethyl-4-pyridinylidene)-3-phenyl-1-formazano]-1,3-dicarboxyethylimidazolium chloride
- 2-[5-(N-carboxyethyl-4-pyridinylidene)-3-isopropyl-1-formazano]-1,3-dicarboxyethylimidazolium chloride
- 2-[5-(N-carboxyethyl-4-pyridinylidene)-3-(4'-methoxyphenyl)-1-formazano]-1,3-dicarboxyethylimidazolium chloride
- 2-[5-(N-carboxyethyl-4-pyridinylidene)-3-(4'-hydroxyphenyl)-1-formazano]-1,3-dicarboxyethylimidazolium chloride
- 2-[5-(N-carboxyethyl-4-pyridinylidene)-3-(4'-N,N-dimethylaminophenyl)-1-formazano]-1,3-dicarboxyethylimidazolium chloride.

14. Process according to any one of the preceding claims, **characterized in that** the oxidizing agent is chosen from hydrogen peroxide; urea peroxide; organic peracids; persalts; persulfates; chromates or dichromates; hypochlorites; hypobromites; ferricyanides; manganese dioxide or lead dioxide, alone or as mixtures.

15. Process according to the preceding claim, **characterized in that** the oxidizing agent is hydrogen peroxide.

16. Process according to any one of the preceding claims, **characterized in that** the oxidizing agent is chosen from enzymes, in the presence of a substrate that is suitable for the said enzyme.

17. Process according to the preceding claim,
**characterized in that** the enzyme is chosen from oxidases that produce hydrogen peroxide, in the presence of a substrate for the said enzyme.

18. Process according to the preceding claim,
**characterized in that** the enzyme is chosen from:
| | |
|---|---|
| PYRANOSE OXIDASE | EC 1.1.3.10 |
| L-SORBOSE OXIDASE | EC 1.1.3.11 |
| PYRIDOXINE 4-OXIDASE | EC 1.1.3.12 |
| ALCOHOL OXIDASE | EC 1.1.3.13 |
| CATECHOL OXIDASE (DIMERIZING) | EC 1.1.3.14 |
| (S)-2-HYDROXY-ACID OXIDASE | EC 1.1.3.15 |
| ECDYSONE OXIDASE | EC 1.1.3.16 |
| CHOLINE OXIDASE | EC 1.1.3.17 |
| SEC-ALCOHOL OXIDASE | EC 1.1.3.18 |
| 4-HYDROXYMANDALATE OXIDASE | EC 1.1.3.19 |
| LONG-CHAIN-ALCOHOL OXIDASE | EC 1.1.3.20 |
| GLYCEROL-3-PHOSPHATE OXIDASE | EC 1.1.3.21 |
| XANTHINE OXIDASE | EC 1.1.3.22 |
| THIAMIN OXIDASE | EC 1.1.3.23 |
| L-GALACTONOLACTONE OXIDASE | EC 1.1.3.24 |
| CELLOBIOSE OXIDASE | EC 1.1.3.25 |
| HYDROXYPHYTANATE OXIDASE | EC 1.1.3.27 |
| NUCLEOSIDE OXIDASE | EC 1.1.3.28 |
| N-ACYLHEXOSAMINE OXIDASE | EC 1.1.3.29 |
| MALATE OXIDASE | EC 1.1.3.3 |
| POLYVINYL-ALCOHOL OXIDASE | EC 1.1.3.30 |
| METHANOL OXIDASE | EC 1.1.3.31 |
| D-ARABINONO-1, 4-LACTONE OXIDASE | EC 1.1.3.37 |
| VANILLYL-ALCOHOL OXIDASE | EC 1.1.3.38 |
| NUCLEOSIDE OXIDASE (H (2) o (2) - FORMING) | EC 1.1.3.39 |
| GLUCOSE OXIDASE | EC 1.1.3.4 |
| D-MANNITOL OXIDASE | EC 1.1.3.40 |
| XYLITOL OXIDASE | EC 1.1.3.41 |
| HEXOSE OXIDASE | EC 1.1.3.5 |
| CHOLESTEROL OXIDASE | EC 1.1.3.6 |
| ARYL ALCOHOL OXIDASE | EC 1.1.3.7 |
| L-GULONOLACTONE OXIDASE | EC 1.1.3.8 |
| GALACTOSE OXIDASE | EC 1.1.3.9 |
| PTERIDINE OXIDASE | EC 1.17.3.1 |
| RETINAL OXIDASE | EC 1.2.3.11 |
| ALDEHYDE OXIDASE | EC 1.2.3.1 |
| CARBON MONOXIDE OXIDASE | EC 1.2.3.10 |
| VANILLATE DEMETHYLASE | EC 1.2.3.12 |
| 4-HYDROXYPHENYLPYRUVATE OXIDASE | EC 1.2.3.13 |
| PYRUVATE OXIDASE | EC 1.2.3.3 |
| OXALATE OXIDASE | EC 1.2.3.4 |
| GLYOXALATE OXIDASE | EC 1.2.3.5 |
| PYRUVATE OXIDASE (CO-A ACETYLATING) | EC 1.2.3.6 |
| INDOLE-3-ACETALDEHYDE OXIDASE | EC 1.2.3.7 |
| PYRIDOXAL OXIDASE | EC 1.2.3.8 |
| ARYL ALDEHYDE OXIDASE | EC 1.2.3. 9 |
| DIHYDROOROTATE OXIDASE | EC 1.3.3.1 |
| LATHOSTEROL OXIDASE | EC 1.3.3.2 |
| COPROPORPHYRINOGEN OXIDASE | EC 1.3.3.3 |
| PROTOPORPHYRINOGEN OXIDASE | EC 1.3.3.4 |
| BILIRUBIN OXIDASE | EC 1.3.3.5 |
| ACYL CO-A OXIDASE | EC 1.3.3.6 |
| DIHYDROURACIL OXIDASE | EC 1.3.3.7 |
| TETRAHYDROBERBERINE OXIDASE | EC 1.3.3.8 |
| SECOLOGANIN SYNTHASE | EC 1.3.3.9 |
| ASPARTATE OXIDASE | EC 1.4.3.1 |
| PUTRESCINE OXIDASE | EC 1.4.3.10 |
| L-GLUTAMATE OXIDASE | EC 1.4.3.11 |
| CYCLOHEXYLAMINE OXIDASE | EC 1.4.3.12 |
| PROTEIN-LYSINE 6-OXIDASE | EC 1.4.3.13 |
| L-LYSINE OXIDASE | EC 1.4.3.14 |
| D-GLUTAMATE (D-ASPARTATE) OXIDASE | EC 1.4.3.15 |
| L-ASPARTATE OXIDASE | EC 1.4.3.16 |
| TRYPTOPHAN ALPHA, BETA-OXIDASE | EC 1.4.3.17 |
| GLYCINE OXIDASE | EC 1.4.3.19 |
| L-AMINO ACID OXIDASE | EC 1.4.3.2 |
| D-AMINO ACID OXIDASE | EC 1.4.3.3 |
| AMINE OXIDASE (FLAVIN CONTAINING) | EC 1.4.3.4 |
| PYRIDOXAMINE PHOSPHATE OXIDASE | EC 1.4.3.5 |
| AMINE OXIDASE (COPPER CONTAINING) | EC 1.4.3.6 |
| D-GLUTAMATE OXIDASE | EC 1.4.3.7 |
| ETHANOLAMINE OXIDASE | EC 1.4.3.8 |
| SARCOSINE OXIDASE | EC 1.5.3.1 |
| DIMETHYLGLYCINE OXIDASE | EC 1.5.3.10 |
| POLYAMINE OXIDASE | EC 1.5.3.11 |
| DIHYDROBENZOPHENANTHRIDINE OXIDASE | EC 1.5.3.12 |
| N-METHYL-L-AMINO ACID OXIDASE | EC 1.5.3.2 |
| N-METHYL-LYSINE OXIDASE | EC 1.5.3.4 |
| (S)-6-HYDROXYNICOTINE OXIDASE | EC 1.5.3.5 |
| (R)-6-HYDROXYNICOTINE OXIDASE | EC 1.5.3.6 |
| L-PIPECOLATE OXIDASE | EC 1.5.3.7 |
| RETICULATE OXIDASE | EC 1.5.3.9 |
| NITROETHANE OXIDASE | EC 1.7.3.1 |
| ACETYLINDOXYL OXIDASE | EC 1.7.3.2 |
| URATE OXIDASE | EC 1.7.3.3 |
| HYDROXYLAMINE OXIDASE | EC 1.7.3.4 |
| 3-ACYL-NITROPROPANOATE OXIDASE | EC 1.7.3.5 |
| SULFITE OXIDASE | EC 1.8.3.1 |
| THIOL OXIDASE | EC 1.8.3.2 |
| GLUTATHIONE OXIDASE | EC 1.8.3.3 |
| METHANETHIOL OXIDASE | EC 1.8.3.4 |
| PRENYLCYSTEINE OXIDASE | EC 1.8.3.5 |

19. Process according to Claim 16, **characterized in that** the enzyme is chosen from 4-electron oxidoreductases.

20. Process according to the preceding claim,
**characterized in that** the enzyme is chosen from enzymes of the group EC 1.10.3, for instance catechol oxidase (polyphenol oxidase) EC 1.10.3.1, laccase EC 1.10.3.2, L-ascorbate oxidase EC 1.10.3.3 and aminophenol oxidase EC 1.10.3.4, and tyrosinase (monophenol oxidase) EC 1.10.3.1 or 1.14.18.1.

21. Process according to Claim 16, **characterized in that** the enzyme is chosen from peroxidases.

22. Process according to the preceding claim, **characterized in that** the enzyme is chosen from:
| | |
|---|---|
| NADH peroxidase | E.C 1.11.1.1 |
| NADPH peroxidase | E.C 1.11.1.2 |
| Fatty acid peroxidase | E.C 1.11.1.3 |
| Cytochrome-c peroxidase | E.C 1.11.1.5 |
| Peroxidase | E.C 1.11.1.7 |
| Iodide peroxidase | E.C 1.11.1.8 |
| Glutathione peroxidase | E.C 1.11.1.9 |
| Chloride peroxidase | E.C 1.11.1.10 |
| L-ascorbate peroxidase | E.C 1.11.1.11 |
| Phospholipid-hydroperoxide glutathione peroxidase | E.C 1.11.1.12 |
| Manganese peroxidase | E.C 1.11.1.13 |
| Diarylpropane peroxidase | E.C 1.11.1.14 |
| Tryptophan 2,3-dioxygenase | E.C 1.13.11.11 |
| Lipoxygenase | E.C 1.13.11.12 |

23. Process for dyeing keratin fibres, especially human keratin fibres, in which the following steps are performed:
a) a dye composition comprising an azine compound of formula (VI) and/or (VII): 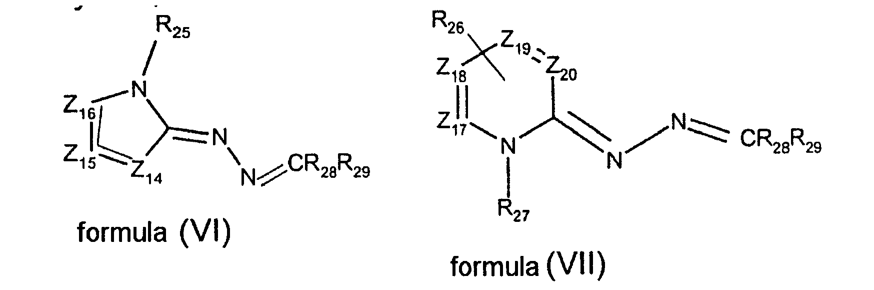 in which:
- Z₁₄, Z₁₅, Z₁₆ and R₂₅ respectively have the same meanings as Z₇, Z₈, Z₁₃ and R₅ of formula (IV), or as Z₁, Z₂, Z₀ and R₁ of formula (II), defined above,
- Z₁₇, Z₁₈, Z₁₉, Z₂₀, R₂₆ and R₂₇ respectively have the same meanings as Z₉, Z₁₀, Z₁₁, Z₁₂, R₈ and R₇ of formula (V), or as Z₃, Z₄, Z₅, Z₆, R₃ and R₄ of formula (III) defined above,
- R₂₈ and R₂₉, independently of each other, represent a hydrogen atom, a C₁-C₆ and preferably C₁-C₄ alkyl radical, or a linear or branched C₁-C₆ and preferably C₁-C₄ alkoxy radical, on condition that at least one of the two radicals is other than hydrogen,
is applied to the wet or dry fibres, in the presence of an oxidizing agent;
b) the composition is left to act for a time sufficient to develop the desired coloration.

24. Dyeing process according to Claim 23, **characterized in that** the oxidizing agent is mixed with the dye composition at the time of use.

25. Dyeing process according to either of Claims 23 and 24, **characterized in that** the oxidizing agent is applied to the fibres simultaneously with or sequentially to the dye composition.

26. Dyeing process according to one of Claims 23 to 25, **characterized in that** the dye composition comprises a content of compounds of formula (VI) and/or (VII) of between 0.001% and 10% by weight and preferably from 0.05% to 5% by weight relative to the total weight of the dye composition.

27. Dyeing process according to one of Claims 23 to 26, **characterized in that** the dye composition comprises at least one oxidation base and optionally at least one coupler, and/or at least one direct dye.

28. Dyeing process according to one of Claims 23 to 27, **characterized in that** the composition comprises at least one oxidation base chosen from para-phenylenediamines, bis(phenyl)alkylenediamines, para-aminophenols, ortho-aminophenols and heterocyclic bases, and the addition salts thereof.

29. Dyeing process according to Claim 28, **characterized in that** the dye composition comprises a content of oxidation base of between 0.001% and 10% by weight relative to the total weight of the dye composition.

30. Dyeing process according to one of Claims 23 to 29, **characterized in that** it comprises at least one coupler chosen from the group formed by meta-phenylenediamines, meta-aminophenols, meta-diphenols, naphthalene-based couplers and heterocyclic couplers, and also the addition salts thereof.

31. Dyeing process according to Claim 30, **characterized in that** the dye composition comprises a content of couplers of between 0.001% and 10% by weight relative to the total weight of the dye composition.

32. Dyeing process according to one of Claims 23 to 31, **characterized in that** the dye composition comprises at least one direct dye.

33. Dyeing process according to Claim 32,
**characterized in that** the dye composition comprises a content of direct dye of between 0.001% and 20% by weight relative to the total weight of the dye composition.

## Patentansprüche

1. Verfahren zur Herstellung von Tetraazapentamethin-Verbindungen der folgenden Formel (I): 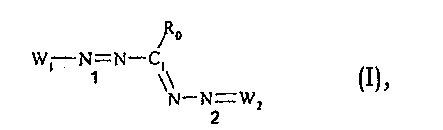 worin bedeuten:
- W₁ eine kationische heteroaromatische Gruppe der Formel (II) oder (III): 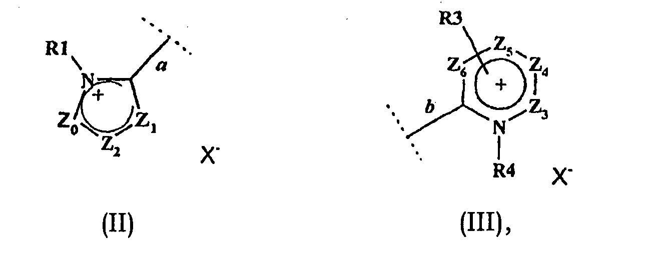
- W₂ eine heteroaromatische Gruppe der Formel (IV) oder (V): 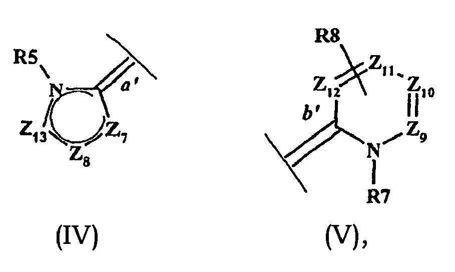
worin bedeuten:
- Z₀ eine Gruppe CR₂, ein Stickstoffatom oder eine Gruppe NR₂₁,
- Z₁ eine Sauerstoffatom, ein Schwefelatom oder eine Gruppe NR₉,
- Z₂ ein Stickstoffatom oder eine Gruppe CR₁₀,
- Z₃ ein Stickstoffatom oder eine Gruppe CR₁₁,
- Z₄ ein Stickstoffatom oder eine Gruppe CR₁₂,
- Z₅ ein Stickstoffatom oder eine Gruppe CR₁₃,
- Z₆ ein Stickstoffatom oder eine Gruppe CR₁₄,
- Z₇ ein Sauerstoffatom, ein Schwefelatom oder eine Gruppe NR₁₅,
- Z₈ ein Stickstoffatom oder eine Gruppe CR₁₆,
- Z₉ ein Stickstoffatom oder eine Gruppe CR₁₇,
- Z₁₀ ein Stickstoffatom oder eine Gruppe CR₁₈,
- Z₁₁ ein Stickstoffatom oder eine Gruppe CR₁₉,
- Z₁₂ ein Stickstoffatom oder eine Gruppe CR₂₀,
- Z₁₃ eine Gruppe CR₆, ein Stickstoffatom oder eine Gruppe NR₂₂,
- mit der Maßgabe, dass jeder der Ringe der Formeln (II), (III), (IV) und (V) nicht mehr als drei Stickstoffatome aufweist und zwei der drei Stickstoffatome benachbart sein können,
- die Bindung a der kationischen heteroaromatischen Gruppen mit 5 Ringgliedern der Formel (II) mit dem Stickstoffatom N₁ der Formel (I) verknüpft ist,
- die Bindung b der kationischen heteroaromatischen Gruppe mit 6 Ringgliedern der Formel (III) mit dem Stickstoffatom N₁ der Formel (I) verknüpft ist,
- die Doppelbindung a' der heteroaromatischen Gruppe mit 5 Ringgliedern der Formel (IV) mit dem Stickstoffatom N₂ der Formel (I) verknüpft ist ist,
- die Doppelbindung b' der heteroaromatischen Gruppe mit 6 Ringgliedern der Formel (V) mit dem Stickstoffatom N₂ der Formel (I) verknüpft ist,
- wobei sich die Bindung b, über welche die kationische heteroaromatische Gruppe der Formel (III) mit dem Stickstoffatom N₁ der Formel (I) verknüpft ist, in o- oder p-Position zu dem Stickstoffatom befindet, das die Gruppe R₄ trägt, wenn Z₅ eine Gruppe CR₁₃ darstellt, und sich die Bindung b in o-Position zu dem Stickstoffatom befindet, das die Gruppe R₄ trägt, wenn Z₅ ein Stickstoffatom bedeutet,
- wobei sich die Bindung b', über welche die heteroaromatische Gruppe der Formel (V) mit dem Stickstoffatom N₂ der Formel (I) verknüpft ist, in o-Position oder in p-Position zu dem Stickstoffatom befindet, das die Gruppe R₇ trägt, wenn Z₁₁ eine Gruppe CR₁₉ darstellt, und sich die Bindung b' in o-Position zu dem Stickstoffatom befindet, das die Gruppe R₇ trägt, wenn Z₁₁ ein Stickstoffatom darstellt,
- R₂, R₆, R₁₀ und R₁₆ unabhängig voneinander ein Wasserstoffatom; eine geradkettige oder verzweigte C₁-C₄-Alkylgruppe, die unsubstituiert ist oder mit einer bis drei Gruppen substituiert ist, die ausgewählt sind unter Hydroxy, C₁-C₂-Alkoxy, (Poly)-hydroxy(C₂-C₄)alkoxy, Amino, (Di)-C₁-C₂-alkylamino, Carboxy oder einer Sulfonsäuregruppe; eine Phenylgruppe, die unsubstituiert ist oder mit einer oder zwei Gruppen substituiert ist, die ausgewählt sind unter den Gruppen Hydroxy, C₁-C₂-Alkoxy, (Poly)-hydroxy(C₂-C₄)alkoxy, Amino, (Di)-C₁-C₂-alkylamino; eine Carboxygruppe; eine Sulfonylaminogruppe,
- R₁, R₄, R₅, R₇, R₉, R₁₅, R₂₁ und R₂₂ unabhängig voneinander eine geradkettige oder verzweigte C₁-C₈-Alkylgruppe, die unsubstituiert ist oder mit einer bis drei Gruppen substituiert ist, die ausgewählt sind unter den Gruppen Hydroxy, C₁-C₂-Alkoxy, (Poly)-hydroxy(C₂-C₄)alkoxy, Amino, (Di)-C₁-C₂-alkylamino, Carboxy oder einer Sulfonsäuregruppe;
- R₀, R₃, R₈, R₁₁, R₁₂, R₁₃, R₁₄, R₁₇, R₁₈, R₁₉ und R₂₀ unabhängig voneinander ein Wasserstoffatom, eine geradkettige oder verzweigte C₁-C₁₆-Kohlenwasserstoffkette, wobei diese Kette gesättigt oder durch eine bis drei ungesättigte Bindungen ungesättigt sein kann und wobei diese Kette unsubstituiert oder mit einer bis drei Gruppen substituiert ist, die ausgewählt sind unter den Gruppen Hydroxy, C₁-C₂-Alkoxy, (Poly)-hydroxy(C₂-C₄)alkoxy, Amino, (Di)-C₁-C₂-alkylamino, Carboxy, einer Sulfonsäuregruppe, Sulfonylamino, (Poly)-hydroxy(C₂-C₄)alkylamino oder einem Halogenatom wie Chlor, Fluor oder Brom; eine Phenylgruppe, die unsubstituiert ist oder mit einer bis drei Gruppen substituiert ist, die ausgewählt sind unter den Gruppen Hydroxy, C₁-C₂-Alkoxy, (Poly)-hydroxy(C₂-C₄)alkoxy, Amino, (Di)-C₁-C₂-alkylamino, Carboxy, einer Sulfonsäuregruppe, Sulfonylamino, (Poly)-hydroxy(C₂-C₄)alkylamino oder einem Halogenatom wie Chlor, Fluor oder Brom; eine Heteroarylgruppe, die ausgewählt ist unter den Gruppen Pyrazolyl, Pyrrolyl, Imidazolyl, Thiazolyl, Oxazolyl, Triazolyl, Pyridinyl, Pyrimidinyl, Triazinyl, Pyrazinyl, Pyridazinyl, wobei diese Kohlenwasserstoffkette durch ein oder zwei Sauerstoffatome, Stickstoffatome, Schwefelatome oder eine Gruppe SO₂ unterbrochen sein kann,
- mit der Maßgabe, dass R₀, R₃, R₈, R₁₁, R₁₂, R₁₃, R₁₄, R₁₇, R₁₈, R₁₉ und R₂₀ keine Peroxidbindung und weder Diazogruppen noch Nitrosogruppen aufweisen,
- wobei R₂ mit R₁₀, R₁₁ mit R₁₂, R₆ mit R₁₆ und R₁₇ mit R₁₈ unabhängig voneinander einen aromatischen Kohlenstoffring mit 5 oder 6 Ringgliedern bilden können, der unsubstituiert ist oder mit einer oder zwei der Gruppen Hydroxy, Amino, (Di)-C₁-C₂-Alkylamino, C₁-C₂-Alkoxy, (Poly)-hydroxy(C₂-C₄)alkylamino substituiert ist,
- X ein organisches oder anorganisches Anion,
das darin besteht, in Gegenwart mindestens eines Oxidationsmittels mindestens eine Azinverbindung der Formel (VI) und/oder (VII) 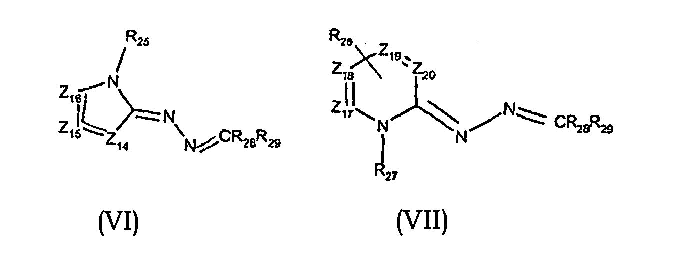 umzusetzen,
worin bedeuten:
- Z₁₄, Z₁₅, Z₁₆, R₂₅ entsprechend dasselbe wie Z₇, Z₈, Z₁₃, R₅ der Formel (IV) oder wie Z₁, Z₂, Z₀, R₁ der Formel (II), wie sie oben definiert wurden,
- Z₁₇, Z₁₈, Z₁₉, Z₂₀, R₂₆, R₂₇ entsprechend dasselbe wie Z₉, Z₁₀, Z₁₁, Z₁₂, R₈, R₇ der Formel (V) oder wie Z₃, Z₄, Z₅, Z₆, R₃, R₄ der Formel (III), wie sie oben definiert wurden,
- R₂₈, R₂₉ unabhängig voneinander ein Wasserstoffatom, eine C₁-C₆-Alkylgruppe, bevorzugt eine C₁-C₄-Alkylgruppe, eine geradkettige oder verzweigte C₁-C₆-Alkoxygruppe, bevorzugt eine C₁-C₄-Alkoxygruppe, mit der Maßgabe, dass mindestens eine der beiden Gruppen von Wasserstoff verschieden ist.

2. Verfahren nach Anspruch 1, wobei R₀ bedeutet: ein Wasserstoffatom; eine geradkettige oder verzweigte C₁-C₆-Alkylgruppe, die unsubstituiert ist oder mit einer bis drei Gruppen substituiert ist, die ausgewählt sind unter den Gruppen Hydroxy, C₁-C₂-Alkoxy, (Poly)-hydroxy(C₂-C₄)alkoxy, Amino, (Di)-C₁-C₂-alkylamino, (Poly)-hydroxy(C₂-C₄)alkylamino, Carboxy oder einer Sulfonsäuregruppe; eine Phenylgruppe, die unsubstituiert ist oder mit 1 bis 3 Gruppen substituiert ist, die ausgewählt sind unter den Gruppen Hydroxy, C₁-C₂-Alkoxy, (Poly)-hydroxy(C₂-C₄)alkoxy, Amino, (Di)-C₁-C₂-alkylamino, Carboxy, einer Sulfonsäuregruppe oder einem Halogenatom wie Chlor, Fluor oder Brom; eine gegebenenfalls kationische Heteroarylgruppe, die ausgewählt ist unter den Gruppen Pyrazolyl, Pyrrolyl, Imidazolyl, Thiazolyl, Oxazolyl, Triazolyl, Pyridinyl, Pyrimidinyl, Triazinyl, Pyrazinyl, Pyridazinyl.

3. Verfahren nach Anspruch 1 oder 2, **dadurch gekennzeichnet, dass** in Formel (I) R₂, R₆, R₁₀ und R₁₆ bedeuten: ein Wasserstoffatom, eine C₁-C₄-Alkylgruppe, die unsubstituiert ist oder mit einer bis zwei Gruppen substituiert ist, die ausgewählt sind unter den Gruppen Hydroxy, Amino, (Di)-C₁-C₂-alkylamino, Carboxy und einer Phenylgruppe.

4. Verfahren nach einem der Ansprüche 1 bis 3, **dadurch gekennzeichnet, dass** in Formel (I) R₁, R₄, R₅, R₇, R₉, R₁₅, R₂₁ und R₂₂ bevorzugt eine C₁-C₄-Alkylgruppe bedeuten, die unsubstituiert ist oder mit einer bis zwei Gruppen substituiert ist, die ausgewählt sind unter den Gruppen Hydroxy, C₁-C₂-Alkoxy, Amino, (Di)-C₁-C₂-alkylamino, Carboxy und einer Sulfonsäuregruppe.

5. Verfahren nach einem der Ansprüche 1 bis 4, **dadurch gekennzeichnet, dass** in Formel (I) R₃, R₈, R₁₁, R₁₂, R₁₃, R₁₄, R₁₇, R₁₈, R₁₉ und R₂₀ unabhängig voneinander darstellen: ein Wasserstoffatom; eine geradkettige oder verzweigte C₁-C₄-Alkylgruppe, die unsubstituiert ist oder mit einer bis zwei Gruppen substituiert ist, die ausgewählt sind unter den Gruppen Hydroxy, C₁-C₂-Alkoxy, (Poly)-hydroxy(C₂-C₄)alkoxy, Amino, (Di)-C₁-C₂-alkylamino, Carboxy oder einer Sulfonsäuregruppe; eine Phenylgruppe, die unsubstituiert ist oder substituiert ist mit einer bis zwei Gruppen, die ausgewählt sind unter den Gruppen Hydroxy, C₁-C₂-Alkoxy, (Poly)-hydroxy(C₂-C₄)alkoxy, Amino, (Di)-C₁-C₂-alkylamino, Carboxy, einer Sulfonsäuregruppe oder einem Halogenatom wie Chlor, Fluor oder Brom, eine Sulfonylaminogruppe; eine (Poly)-hydroxy(C₂-C₄)alkylamino-Gruppe.

6. Verfahren nach einem der Ansprüche 1 bis 5, **dadurch gekennzeichnet, dass** die Verbindung der Formel (I), in der W₁ eine 2-Pyridinium-Gruppe und W₂ eine 2-Pyridin-Gruppe bedeuten, ausgewählt ist unter:
- 2-[5-(N-Methyl-2-pyridinyliden)-1-formazano]-N-methylpyridinium-chlorid,
- 2-[5-(N-Methyl-2-pyridinyliden)-3-methyl-1-formazano]-N-methylpyridinium-chlorid,
- 2-[5-(N-Methyl-2-pyridinyliden)-3-ethyl-1-formazano]-N-methylpyridinium-chlorid,
- 2-[5-(N-Methyl-2-pyridinyliden)-3-isopropyl-1-formazano]-N-methylpyridinium-chlorid,
- 2-[5-(N-Methyl-2-pyridinyliden)-3-phenyl-1-formazano]-N-methylpyridinium-chlorid,
- 2-[5-(N-Methyl-2-pyridinyliden)-3-(4'-methoxyphenyl)-1-formazano]-N-methylpyridinium-chlorid,
- 2-[5-(N-Methyl-2-pyridinyliden)-3-(4'-hydroxyphenyl)-1-formazano]-N-methylpyridinium-chlorid,
- 2-[5-(N-Methyl-2-pyridinyliden)-3-(4'-N,N-dimethylaminophenyl)-1-formazano]-N-methylpyridinium-chlorid,
- 2-[5-(4-Pyrrolidino-N-methyl-2-pyridinyliden)-1-formazano]-4-pyrrolidino-N-methylpyridinium-chlorid,
- 2-[5-(4-Pyrrolidino-N-methyl-2-pyridinyliden)-3-methyl-1-formazano]-4-pyrrolidino-N-methylpyridinium-chlorid,
- 2-[5-(4-Pyrrolidino-N-methyl-2-pyridinyliden)-3-ethyl-1-formazano]-4-pyrrolidino-N-methylpyridinium-chlorid,
- 2-[5-(4-Pyrrolidino-N-methyl-2-pyridinyliden)-3-isopropyl-1-formazano]-4-pyrrolidino-N-methylpyridinium-chlorid,
- 2-[5-(4-Pyrrolidino-N-methyl-2-pyridinyliden)-3-phenyl-1-formazano]-4-pyrrolidino-N-methylpyridinium-chlorid,
- 2-[5-(4-Pyrrolidino-N-methyl-2-pyridinyliden)-3-(4'-methoxyphenyl)-1-formazano]-4-pyrrolidino-N-methylpyridinium-chlorid,
- 2-[5-(4-Pyrrolidino-N-methyl-2-pyridinyliden)-3-(4'-hydroxyphenyl)-1-formazano]-4-pyrrolidino-N-methylpyridinium-chlorid,
- 2-[5-(4-Pyrrolidino-N-methyl-2-pyridinyliden)-3-(4'-N,N-dimethylaminophenyl)-1-formazano]-4-pyrrolidino-N-methylpyridinium-chlorid,
- 2-[5-(N-Hydroxyethyl-2-pyridinyliden)-1-formazano]-N-hydroxyethylpyridinium-chlorid,
- 2-[5-(N-Hydroxyethyl-2-pyridinyliden)-3-methyl-1-formazano]-N-hydroxyethylpyridinium-chlorid,
- 2-[5-(N-Hydroxyethyl-2-pyridinyliden)-3-ethyl-1-formazano]-N-hydroxyethylpyridinium-chlorid,
- 2-[5-(N-Hydroxyethyl-2-pyridinyliden)-3-phenyl-1-formazano]-N-hydroxyethylpyridinium-chlorid,
- 2-[5-(N-Hydroxyethyl-2-pyridinyliden)-3-isopropyl-1-formazano]-N-hydroxyethylpyridinium-chlorid,
- 2-[5-(N-Hydroxyethyl-2-pyridinyliden)-3-(4'-methoxyphenyl)-1-formazano]-N-hydroxyethylpyridinium-chlorid,
- 2-[5-(N-Hydroxyethyl-2-pyridinyliden)-3-(4'-hydroxyphenyl)-1-formazano]-N-hydroxyethylpyridinium-chlorid,
- 2-[5-(N-Hydroxyethyl-2-pyridinyliden)-3-(4'-N,N-dimethylaminophenyl)-1-formazano]-N- hydroxyethylpyridiniumchlorid,
- 2-[5-(N-Carboxyethyl-2-pyridinyliden)-1-formazano]-N-carboxyethylpyridinium-chlorid,
- 2-[5-(N-Carboxyethyl-2-pyridinyliden)-3-methyl-1-formazano]-N-carboxyethylpyridinium-chlorid,
- 2-[5-(N-Carboxyethyl-2-pyridinyliden)-3-ethyl-1-formazano]-N-carboxyethylpyridinium-chlorid,
- 2-[5-(N-Carboxyethyl-2-pyridinyliden)-3-phenyl-1-formazano]-N-carboxyethylpyridinium-chlorid,
- 2-[5-(N-Carboxyethyl-2-pyridinyliden)-3-isopropyl-1-formazano]-N-carboxyethylpyridinium-chlorid,
- 2-[5-(N-Carboxyethyl-2-pyridinyliden)-3-(4'-methoxyphenyl)-1-formazano]-N-carboxyethylpyridinium-chlorid,
- 2-[5-(N-Carboxyethyl-2-pyridinyliden)-3-(4'-hydroxyphenyl)-1-formazano]-N-carboxyethylpyridinium-chlorid,
- 2-[5-(N- Carboxyethyl-2-pyridinyliden)-3-(4'-N,N-dimethylaminophenyl)-1-formazano]-N-carboxyethylpyridinium-chlorid,

7. Verfahren nach einem der Ansprüche 1 bis 6, **dadurch gekennzeichnet, dass** die Verbindung der Formel (I), in der W₁ eine 4-Pyridinium-Gruppe und W₂ eine 4-Pyridin-Gruppe bedeuten, ausgewählt ist unter:
- 4-[5-(N-Methyl-4-pyridinyliden)-1-formazano]-N-methylpyridinium-chlorid,
- 4-[5-(N-Methyl-4-pyridinyliden)-3-methyl-1-formazano]-N-methylpyridinium-chlorid,
- 4-[5-(N-Methyl-4-pyridinyliden)-3-ethyl-1-formazano]-N-methylpyridinium-chlorid,
- 4-[5-(N-Methyl-4-pyridinyliden)-3-isopropyl-1-formazano]-N-methylpyridinium-chlorid,
- 4-[5-(N-Methyl-4-pyridinyliden)-3-phenyl-1-formazano]-N-methylpyridinium-chlorid,
- 4-[5-(N-Methyl-4-pyridinyliden)-3-(4'-methoxyphenyl)-1-formazano]-N-methylpyridinium-chlorid,
- 4-[5-(N-Methyl-4-pyridinyliden)-3-(4'-hydroxyphenyl)-1-formazano]-N-methylpyridinium-chlorid,
- 4-[5-(N-Methyl-4-pyridinyliden)-3-(4'-N,N-dimethylaminophenyl)-1-formazano]-N-methylpyridinium-chlorid,
- 4-[5-(N-Hydroxyethyl-4-pyridinyliden)-1-formazano]-N-hydroxy-ethylpyridinium-chlorid,
- 4-[5-(N-Hydroxyethyl-4-pyridinyliden)-3-methyl-1-formazano]-N-hydroxyethylpyridinium-chlorid;
- 4-[5-(N-Hydroxyethyl-4-pyridinyliden)-3-ethyl-1-formazano]-N-hydroxyethylpyridinium-chlorid,
- 4-[5-(N-Hydroxyethyl-4-pyridinyliden)-3-phenyl-1-formazano]-N-hydroxyethylpyridinium-chlorid,
- 4-[5-(N-Hydroxyethyl-4-pyridinyliden)-3-isopropyl-1-formazano]-N-hydroxyethylpyridinium-chlorid,
- 4-[5-(N-Hydroxyethyl-4-pyridinyliden)-3-(4'-methoxyphenyl)-1-formazano]-N-Hydroxyethylpyridinium-chlorid,
- 4-[5-(N-Hydroxyethyl-4-pyridinyliden)-3-(4'-hydroxyphenyl)-1-formazano]-N-hydroxyethylpyridinium-chlorid,
- 4-[5-(N-Hydroxyethyl-4-pyridinyliden)-3-(4'-N,N-dimethylaminophenyl)-1-formazano]-N-hydroxyethylpyridinium-chlorid,
- 4-[5-(N-Carboxyethyl-4-pyridinyliden)-1-formazano]-N-carboxyethylpyridinium-chlorid,
- 4-[5-(N-Carboxyethyl-4-pyridinyliden)-3-methyl-1-formazano]-N-carboxyethylpyridinium-chlorid,
- 4-[5-(N-Carboxyethyl-4-pyridinyliden)-3-ethyl-1-formazano]-N-carboxyethylpyridinium-chlorid,
- 4-[5-(N-Carboxyethyl-4-pyridinyliden)-3-phenyl-1-formazano)-N-carboxyethylpyridinium-chlorid,
- 4-[5-(N-Carboxyethyl-4-pyridinyliden)-3-isopropyl-1-formazano]-N-carboxyethylpyridinium-chlorid,
- 4-[5-(N-Carboxyethyl-4-pyridinyliden)-3-(4'-methoxyphenyl)-1-formazano]-N-carboxyethylpyridinium-chlorid,
- 4-[5-(N-Carboxyethyl-4-pyridinyliden)-3-(4'-hydroxyphenyl)-1-formazanol-N-carboxyethylpyridinium-chlorid,
- 4-[5-(N-Carboxyethyl-4-pyridinyliden)-3-(4'-N,N-dimethylaminophenyl)-1-formazano]-N-carboxyethylpyridinium-chlorid.

8. Verfahren nach einem der Ansprüche 1 bis 7, **dadurch gekennzeichnet, dass** die Verbindung der Formel (I), in der W₁ eine 2-Imidazolium-Gruppe und W₂ eine 2-Imidazol-Gruppe bedeuten, ausgewählt ist unter:
- 2-[5-(1,3-Dimethyl-2-imidazoliden)-1-formazano]-1,3-dimethylimidazolium-chlorid,
- 2-[5-(1,3-Dimethyl-2-imidazoliden)-3-methyl-1-formazano]-1,3-dimethylimidazolium-chlorid,
- 2-[5-(1,3-Dimethyl-2-imidazoliden)-3-ethyl-1-formazano]-1,3-dimethylimidazolium-chlorid,
- 2-[5-(1,3-Dimethyl-2-imidazoliden)-3-isopropyl-1-formazano]-1, 3-dimethylimidazolium-chlorid,
- 2-[5-(1,3-Dimethyl-2-imidazoliden)-3-phenyl-1-formazano]-1, 3-dimethylimidazolium-chlorid,
- 2-[5-(1,3-Dimethyl-2-imidazoliden)-3-(4'-methoxyphenyl)-1-formazano]-1,3-dimethylimidazolium-chlorid,
- 2-[5-(1,3-Dimethyl-2-imidazoliden)-3-(4'-hydroxyphenyl)-1-formazano]-1,3-dimethylimidazolium-chlorid,
- 2-[5-(1,3-Dimethyl-2-imidazoliden)-3-(4'-N,N-dimethylaminophenyl)-1-formazano]-1,3-dimethylimidazolium-chlorid,
- 2-[5-(1,3-Dihydroxyethyl-2-imidazoliden)-1-formazano]-1,3-dihydroxyethylimidazolium-chlorid,
- 2-[5-(1,3-Dihydroxyethyl-2-imidazoliden)-3-methyl-1-formazano]-1,3-dihydroxyethylimidazolium-chlorid,
- 2-[5-(1,3-Dihydroxyethyl-2-imidazoliden)-3-ethyl-1-formazano]-1, 3-dihydroxyethylimidazolium-chlorid,
- 2-[5-(1,3-Dihydroxyethyl-2-imidazoliden)-3-phenyl-1-formazano]-1,3-dihydroxyethylimidazolium-chlorid,
- 2-[5-(1,3-Dihydroxyethyl-2-imidazoliden)-3-isopropyl-1-formazano]-1,3-dihydroxyethylimidazolium-chlorid,
- 2-[5-(1,3-Dihydroxyethyl-2-imidazoliden)-3-(4'-methoxyphenyl)-1-formazano]-1,3-dihydroxyethylimidazolium-chlorid,
- 2-[5-(1,3-Dihydroxyethyl-2-imidazoliden)-3-(4'-hydroxyphenyl)-1-formazano]-1,3-dihydroxyethylimidazolium-chlorid,
- 2-[5-(1,3-Dihydroxyethyl-2-imidazoliden)-3-(4'-N,N-dimethylaminophenyl)-1-formazano]-1,3-dihydroxyethylimidazolium-chlorid,
- 2-[5-(1,3-Dicarboxyethyl-2-imidazoliden)-1-formazano]-1,3-dicarboxyethylimidazolium-chlorid,
- 2-[5-(1,3-Dicarboxyethyl-2-imidazoliden)-3-methyl-1-formazano]-1,3-dicarboxyethylimidazolium-chlorid,
- 2-[5-(1,3-Dicarboxyethyl-2-imidazoliden)-3-ethyl-1-formazano]-1,3-dicarboxyethylimidazolium-chlorid,
- 2-[5-(1,3-Dicarboxyethyl-2-imidazoliden)-3-phenyl-1-formazano]-1,3-dicarboxyethylimidazolium-chlorid,
- 2-[5-(1,3-Dicarboxyethyl-2-imidazoliden)-3-isopropyl-1-formazano]-1,3-dicarboxyethyl-imidazolium-chlorid,
- 2-[5-(1,3-Dicarboxyethyl-2-imidazoliden)-3-(4'-methoxyphenyl)-1 -formazano]-1,3-dicarboxyethylimidazolium-chlorid,
- 2-[5-(1,3-Dicarboxyethyl-2-imidazoliden)-3-(4'-hydroxyphenyl)-1 -formazano]-1,3-dicarboxyethylimidazolium-chlorid,
- 2-[5-(1,3-Dicarboxyethyl-2-imidazoliden)-3-(4'-N,N-dimethylaminophenyl)-1-formazano]-1,3-dicarboxyethylimidazolium-chlorid.

9. Verfahren nach einem der Ansprüche 1 bis 8, **dadurch gekennzeichnet, dass** die Verbindung der Formel (I), in der W₁ eine 5-Pyrazolium-Gruppe und W₂ eine 5-Pyrazol-Gruppe bedeuten, ausgewählt ist unter:
- 5-[5-(1,2-Dimethyl-5-pyrazoliden)-1-formazano]-1,2-dimethylpyrazolinium-chlorid,
- 5-[5-(1,2-Dimethyl-5-pyrazoliden)-3-methyl-1-formazano]-1,2-dimethylpyrazolinium-chlorid,
- 5-[5-(1,2-Dimethyl-5-pyrazoliden)-3-ethyl-1-formazano]-1,2-dimethylpyrazolinium-chlorid,
- 5-[5-(1,2-Dimethyl-5-pyrazoliden)-3-isopropyl-1-formazano]-1,2-dimethylpyrazolinium-chlorid,
- 5-[5-(1,2-Dimethyl-5-pyrazoliden)-3-phenyl-1-formazano]-1,2-dimethylpyrazolinium-chlorid,
- 5-[5-(1,2-Dimethyl-5-pyrazoliden)-3-(4'-methoxyphenyl)-1-formazano]-1,2-dimethylpyrazolinium-chlorid,
- 5-[5-(1,2-Dimethyl-5-pyrazoliden)-3-(4'-hydroxyphenyl)-1-formazano]-1,2-dimethylpyrazolinium-chlorid,
- 5-[5-(1,2-Dimethyl-5-pyrazoliden)-3-(4'-N,N-dimethylaminophenyl)-1-formazano]-1,2-dimethylpyrazolinium-chlorid,
- 5-[5-(1,2-Dihydroxyethyl-5-pyrazoliden)-1-formazano]-1,2-dihydroxyethylpyrazolinium-chlorid,
- 5-[5-(1,2-Dihydroxyethyl-5-pyrazoliden)-3-methyl-1-formazano]-1,2-dihydroxyethylpyrazolinium-chlorid,
- 5-[5-(1,2- Dihydroxyethyl-5-pyrazoliden)-3-ethyl-1-formazano]-1,2-dihydroxyethylpyrazolinium-chlorid,
- 5-[5-(1,2-Dihydroxyethyl-5-pyrazoliden)-3-phenyl-1-formazano]-1,2-dihydroxyethylpyrazolinium-chlorid,
- 5-[5-(1,2-Dihydroxyethyl-5-pyrazoliden)-3-isopropyl-1-formazano]-1, 2-dihydroxyethylpyrazolinium-chlorid,
- 5-[5-(1,2-Dihydroxyethyl-5-pyrazoliden)-3-(4'-methoxyphenyl)-1 -formazano]-1,2-dihydroxyethylpyrazolinium-chlorid,
- 5-[5-(1,2-Dihydroxyethyl-5-pyrazoliden)-3-(4'-hydroxyphenyl)-1-formazano]-1,2-dihydroxyethylpyrazolinium-chlorid,
- 5-[5-(1,2-Dihydroxyethyl-5-pyrazoliden)-3-(4'-N,N-dimethylaminophenyl)-1-formazano]-1,2-dihydroxyethylpyrazolinium-chlorid,
- 5-[5-(1,2-Dicarboxyethyl-5-pyrazoliden)-1-formazano]-1,2-dicarboxyethylpyrazolinium-chlorid,
- 5-[5-(1,2-Dicarboxyethyl-5-pyrazoliden)-3-methyl-1-formazano]-1,2-dicarboxyethylpyrazolinium-chlorid,
- 5-[5-(1,2-Dicarboxyethyl-5-pyrazoliden)-3-ethyl-1-formazano]-1,2-dicarboxyethylpyrazolinium-chlorid,
- 5-[5-(1,2-Dicarboxyethyl-5-pyrazoliden)-3-phenyl-1-formazano]-1,2-dicarboxyethylpyrazolinium-chlorid,
- 5-[5-(1,2-Dicarboxyethyl-5-pyrazoliden)-3-isopropyl-1-formazano]-1,2-dicarboxyethylpyrazolinium-chlorid,
- 5-[5-(1,2-Dicarboxyethyl-5-pyrazoliden)-3-(4'-methoxyphenyl)-1-formazano]-1,2-dicarboxyethylpyrazolinium-chlorid,
- 5-[5-(1,2-Dicarboxyethyl-5-pyrazoliden)-3-(4'-hydroxyphenyl)-1-formazano]-1,2-dicarboxyethylpyrazolinium-chlorid,
- 5-[5-(1,2-Dicarboxyethyl-5-pyrazoliden)-3-(4'-N,N-dimethylaminophenyl)-1-formazano]-1,2-dicarboxyethyl-pyrazolinium-chlorid.

10. Verfahren nach einem der Ansprüche 1 bis 9, **dadurch gekennzeichnet, dass** die Verbindung der Formel (I), in der W₁ eine 2-Benzimidazolium-Gruppe und W₂ eine 2-Pyridin-Gruppe bedeuten, ausgewählt ist unter:
- 2-[5-(N-Methyl-2-pyridinyliden)-1-formazano]-1,3-dimethylbenzimidazolium-chlorid,
- 2-[5-(N-Methyl-2-pyridinyliden)-3-methyl-1-formazano]-1,3-dimethylbenzimidazolium-chlorid,
- 2-[5-(N-Methyl-2-pyridinyliden)-3-ethyl-1-formazano]-1,3-dimethylbenzimidazolium-chlorid,
- 2-[5-(N-Methyl-2-pyridinyliden)-3-isopropyl-1-formazano]-1,3-dimethylbenzimidazolium-chlorid,
- 2-[5-(N-Methyl-2-pyridinyliden)-3-phenyl-1-formazano]-1,3-dimethylbenzimidazolium-chlorid,
- 2-[5-(N-Methyl-2-pyridinyliden)-3-(4'-methoxyphenyl)-1-formazano]-1,3-dimethylbenzimidazolium-chlorid,
- 2-[5-(N-Methyl-2-pyridinyliden)-3-(4'-hydroxyphenyl)-1-formazano]-1,3-dimethylbenzimidazolium-chlorid,
- 2-[5-(N-Methyl-2-pyridinyliden)-3-(4'-N,N-dimethylaminophenyl)-1-formazano]-1,3-dimethylbenzimidazolium-chlorid,
- 2-[5-(N-Hydroxyethyl-2-pyridinyliden)-1-formazano]-1,3-dihydroxyethylbenzimidazolium-chlorid,
- 2-[5-(N-Hydroxyethyl-2-pyridinyliden)-3-methyl-1-formazano]-1,3-dihydroxyethylbenzimidazolium-chlorid,
- 2-[5-(N-Hydroxyethyl-2-pyridinyliden)-3-ethyl-1-formazano]-1,3-dihydroxyethylbenzimidazolium-chlorid,
- 2-[5-(N-Hydroxyethyl-2-pyridinyliden)-3-phenyl-1-formazano]-1,3-dihydroxyethylbenzimidazolium-chlorid,
- 2-[5-(N-Hydroxyethyl-2-pyridinyliden)-3-isopropyl-1-formazano]-1,3-dihydroxyethylbenzimidazolium-chlorid,
- 2-[5-(N-Hydroxyethyl-2-pyridinyliden)-3-(4'-methoxyphenyl)-1-formazano]-1,3-dihydroxyethylbenzimidazolium-chlorid,
- 2-[5-(N-Hydroxyethyl-2-pyridinyliden)-3-(4'-hydroxyphenyl)-1-formazano]-1,3-dihydroxyethylbenzimidazolium-chlorid,
- 2-[5-(N-Hydroxyethyl-2-pyridinyliden)-3-(4'-N,N-dimethylaminophenyl)-1-formazano]-1,3-dihydroxyethyl-benzimidazolium-chlorid,
- 2-[5-(N-Carboxyethyl-2-pyridinyliden)-1-formazano]-1,3-dicarboxyethylbenzimidazolium-chlorid,
- 2-[5-(N-Carboxyethyl-2-pyridinyliden)-3-methyl-1-formazano]-1,3-dicarboxyethylbenzimidazolium-chlorid,
- 2-[5-(N-Carboxyethyl-2-pyridinyliden)-3-ethyl-1-formazano]-1,3-dicarboxyethylbenzimidazolium-chlorid,
- 2-[5-(N-Carboxyethyl-2-pyridinyliden)-3-phenyl-1-formazano]-1,3-dicarboxyethylbenzimidazolium-chlorid,
- 2-[5-(N-Carboxyethyl-2-pyridinyliden)-3-isopropyl-1-formazano]-1, 3-dicarboxyethylbenzimidazolium-chlorid,
- 2-[5-(N-Carboxyethyl-2-pyridinyliden)-3-(4'-methoxyphenyl)-1-formazano]-1,3-dicarboxyethylbenzimidazolium-chlorid,
- 2-[5-(N-Carboxyethyl-2-pyridinyliden)-3-(4'-hydroxyphenyl)-1-formazano]-1, 3-dicarboxyethylbenzimidazolium-chlorid,
- 2-[5-(N-Carboxyethyl-2-pyridinyliden)-3-(4'-N,N-dimethylaminophenyl)-1-formazano]-1,3-dicarboxyethylbenzimidazolium-chlorid.

11. Verfahren nach einem der Ansprüche 1 bis 10, **dadurch gekennzeichnet, dass** die Verbindung der Formel (I), in der W₁ eine 2-Benzimidazolium-Gruppe und W₂ eine 4-Pyridin-Gruppe bedeuten, ausgewählt ist unter:
- 2-[5-(N-Methyl-4-pyridinyliden)-1-formazano]-1,3-dimethylbenzimidazolium-chlorid,
- 2-[5-(N-Methyl-4-pyridinyliden)-3-methyl-1-formazano]-1,3-dimethylbenzimidazolium-chlorid,
- 2-[5-(N-Methyl-4-pyridinyliden)-3-ethyl-1-formazano]-1,3-dimethylbenzimidazolium-chlorid,
- 2-[5-(N-Methyl-4-pyridinyliden)-3-isopropyl-1-formazano]-1,3-dimethylbenzimidazolium-chlorid,
- 2-[5-(N-Methyl-4-pyridinyliden)-3-phenyl-1-formazano]-1,3-dimethylbenzimidazolium-chlorid,
- 2-[5-(N-Methyl-4-pyridinyliden)-3-(4'-methoxyphenyl)-1-formazano]-1,3-dimethylbenzimidazolium-chlorid,
- 2-[5-(N-Methyl-4-pyridinyliden)-3-(4'-hydroxyphenyl)-1-formazano]-1,3-dimethylbenzimidazolium-chlorid,
- 2-[5-(N-Methyl-4-pyridinyliden)-3-(4'-N,N-dimethylaminophenyl)-1-formazano]-1,3-dimethylbenzimidazolium-chlorid,
- 2-[5-(N-Hydroxyethyl-4-pyridinyliden)-1-formazano]-1,3-dihydroxyethylbenzimidazolium-chlorid,
- 2-[5-(N-Hydroxyethyl-4-pyridinyliden)-3-methyl-1-formazano]-1,3-dihydroxyethylbenzimidazolium-chlorid,
- 2-[5-(N-Hydroxyethyl-4-pyridinyliden)-3-ethyl-1-formazano]-1,3-dihydroxyethylbenzimidazolium-chlorid,
- 2-[5-(N-Hydroxyethyl-4-pyridinyliden)-3-phenyl-1-formazano]-1,3-dihydroxyethylbenzimidazolium-chlorid,
- 2-[5-(N-Hydroxyethyl-4-pyridinyliden)-3-isopropyl-1-formazano]-1,3-dihydroxyethylbenzimidazolium-chlorid,
- 2-[5-(N-Hydroxyethyl-4-pyridinyliden)-3-(4'-methoxyphenyl)-1-formazano]-1,3-dihydroxyethylbenzimidazolium-chlorid,
- 2-[5-(N-Hydroxyethyl-4-pyridinyliden)-3-(4'-hydroxyphenyl)-1-formazano]-1,3-dihydroxyethylbenzimidazolium-chlorid,
- 2-[5-(N-Hydroxyethyl-4-pyridinyliden)-3-(4'-N,N-dimethylaminophenyl)-1-formazano]-1,3-dihydroxyethyl-benzimidazolium-chlorid,
- 2-[5-(N-Carboxyethyl-4-pyridinyliden)-1-formazano]-1,3-dicarboxyethylbenzimidazolium-chlorid,
- 2-[5-(N-Carboxyethyl-4-pyridinyliden)-3-methyl-1-formazano]-1,3-dicarboxyethylbenzimidazolium-chlorid,
- 2-[5-(N-Carboxyethyl-4-pyridinyliden)-3-ethyl-1-formazano]-1,3-dicarboxyethylbenzimidazolium-chlorid,
- 2-[5-(N-Carboxyethyl-4-pyridinyliden)-3-phenyl-1-formazano]-1,3-dicarboxyethylbenzimidazolium-chlorid,
- 2-[5-(N-Carboxyethyl-4-pyridinyliden)-3-isopropyl-1-formazano]-1,3-dicarboxyethylbenzimidazolium-chlorid,
- 2-[5-(N-Carboxyethyl-4-pyridinyliden)-3-(4'-methoxyphenyl)-1-formazano]-1,3-dicarboxyethylbenzimidazolium-chlorid,
- 2-[5-(N-Carboxyethyl-4-pyridinyliden)-3-(4'-hydroxyphenyl)-1-formazano]-1,3-dicarboxyethylbenzimidazolium-chlorid,
- 2-[5-(N-Carboxyethyl-4-pyridinyliden)-3-(4'-N,N-dimethylaminophenyl)-1-formazano]-1,3-dicarboxyethylbenzimidazolium-chlorid,

12. Verfahren nach einem der Ansprüche 1 bis 11, **dadurch gekennzeichnet, dass** die Verbindung der Formel (I), in der W₁ eine 2-Imidazolium-Gruppe und W₂ eine 2-Pyridin-Gruppe bedeuten, ausgewählt ist unter:
- 2-[5-(N-Methyl-2-pyridinyliden)-1-formazano]-1,3-dimethylimidazolium-chlorid,
- 2-[5-(N-Methyl-2-pyridinyliden)-3-methyl-1-formazano]-1,3-dimethylimidazolium-chlorid,
- 2-[5-(N-Methyl-2-pyridinyliden)-3-ethyl-1-formazano]-1,3-dimethylimidazolium-chlorid,
- 2-[5-(N-Methyl-2-pyridinyliden)-3-isopropyl-1-formazano]-1,3-dimethylimidazolium-chlorid,
- 2-[5-(N-Methyl-2-pyridinyliden)-3-phenyl-1-formazano]-1,3-dimethylimidazolium-chlorid,
- 2-[5-(N-Methyl-2-pyridinyliden)-3-(4'-methoxyphenyl)-1-formazano]-1,3-dimethylimidazolium-chlorid,
- 2-[5-(N-Methyl-2-pyridinyliden)-3-(4'-hydroxyphenyl)-1-formazano]- 1,3-dimethylimidazolium-chlorid,
- 2-[5-(N-Methyl-2-pyridinyliden)-3-(4'-N,N-dimethylaminophenyl)-1 -formazano]-1,3-dimethylimidazolium-chlorid,
- 2-[5-(N-Hydroxyethyl-2-pyridinyliden)-1-formazano]-1,3-dihydroxyethylimidazolium-chlorid,
- 2-[5-(N-Hydroxyethyl-2-pyridinyliden)-3-methyl-1-formazano]-1,3-dihydroxyethylimidazolium-chlorid,
- 2-[5-(N-Hydroxyethyl-2-pyridinyliden)-3-ethyl-1-formazano]-1,3-dihydroxyethylimidazolium-chlorid,
- 2-[5-(N-Hydroxyethyl-2-pyridinyliden)-3-phenyl-1-formazano]-1,3-dihydroxyethylimidazolium-chlorid,
- 2-[5-(N-Hydroxyethyl-2-pyridinyliden)-3-isopropyl-1-formazano]-1,3-dihydroxyethylimidazolium-chlorid,
- 2-[5-(N-Hydroxyethyl-2-pyridinyliden)-3-(4'-methoxyphenyl)-1-formazano]-1,3-dihydroxyethylimidazolium-chlorid,
- 2-[5-(N-Hydroxyethyl-2-pyridinyliden)-3-(4'-hydroxyphenyl)-1-formazano]-1,3-dihydroxyethylimidazolium-chlorid,
- 2-[5-(N-Hydroxyethyl-2-pyridinyliden)-3-(4'-N,N-dimethylaminophenyl)-1-formazano]-1,3-dihydroxyethylimidazolium-chlorid,
- 2-[5-(N-Carboxyethyl-2-pyridinyliden)-1-formazano]-1,3-dicarboxyethylimidazolium-chlorid,
- 2-[5-(N-Carboxyethyl-2-pyridinyliden)-3-methyl-1-formazano]-1,3-dicarboxyethylimidazolium-chlorid,
- 2-[5-(N-Carboxyethyl-2-pyridinyliden)-3-ethyl-1-formazano]-1,3-dicarboxyethylimidazolium-chlorid,
- 2-[5-(N-Carboxyethyl-2-pyridinyliden)-3-phenyl-1-formazano]-1,3-dicarboxyethylimidazolium-chlorid,
- 2-[5-(N-Carboxyethyl-2-pyridinyliden)-3-isopropyl-1-formazano]-1,3-dicarboxyethylimidazolium-chlorid,
- 2-[5-(N-carboxyethyl-2-pyridinyliden)-3-(4'-methoxyphenyl)-1-formazano]-1,3-dicarboxyethylimidazolium-chlorid,
- 2-[5-(N-Carboxyethyl-2-pyridinyliden)-3-(4'-hydroxyphenyl)-1-formazano]-1,3-dicarboxyethylimidazolium-chlorid,
- 2-[5-(N-Carboxyethyl-2-pyridinyliden)-3-(4'-N,N-dimethylaminophenyl)-1-formazano]-1,3-dicarboxyethylimidazolium-chlorid.

13. Verfahren nach einem der Ansprüche 1 bis 12, **dadurch gekennzeichnet, dass** die Verbindung der Formel (I); in der W₁ eine 2-Imidazolium-Gruppe und W₂ eine 4-Pyridin-Gruppe bedeuten, ausgewählt ist unter:
- 2-[5-(N-Methyl-4-pyridinyliden)-1-formazano]-1,3-dimethylimidazolium-chlorid,
- 2-[5-(N-Methyl-4-pyridinyliden)-3-methyl-1-formazano]-1,3-dimethylimidazolium-chlorid,
- 2-[5-(N-Methyl-4-pyridinyliden)-3-ethyl-1-formazano]-1,3-dimethylimidazolium-chlorid,
- 2-[5-(N-Methyl-4-pyridinyliden)-3-isopropyl-1-formazano]-1,3-dimethylimidazolium-chlorid,
- 2-[5-(N-Methyl-4-pyridinyliden)-3-phenyl-1-formazano]-1,3-dimethylimidazolium-chlorid,
- 2-[5-(N-Methyl-4-pyridinyliden)-3-(4'-methoxyphenyl)-1-formazano]-1,3-dimethylimidazolium-chlorid,
- 2-[5-(N-Methyl-4-pyridinyliden)-3-(4'-hydroxyphenyl)-1-formazano]-1,3-dimethylimidazolium-chlorid,
- 2-[5-(N-Methyl-4-pyridinyliden)-3-(4'-N,N-dimethyl-aminophenyl)-1-formazano]-1,3-dimethylimidazolium-chlorid,
- 2-[5-(N-Hydroxyethyl-4-pyridinyliden)-1-formazano]-1,3-dihydroxyethylimidazolium-chlorid,
- 2-[5-(N-Hydroxyethyl-4-pyridinyliden)-3-methyl-1-formazano]-1,3-dihydroxyethylimidazolium-chlorid,
- 2-[5-(N-Hydroxyethyl-4-pyridinyliden)-3-ethyl-1-formazano]-1,3-dihydroxyethylimidazolium-chlorid,
- 2-[5-(N-Hydroxyethyl-4-pyridinyliden)-3-phenyl-1-formazano]-1,3-dihydroxyethylimidazolium-chlorid,
- 2-[5-(N-Hydroxyethyl-4-pyridinyliden)-3-isopropyl-1-formazano]-1,3-dihydroxyethylimidazolium-chlorid,
- 2-[5-(N-Hydroxyethyl-4-pyridinyliden)-3-(4'-methoxyphenyl)-1-formazano]-1,3-dihydroxyethylimidazolium-chlorid,
- 2-[5-(N-Hydroxyethyl-4-pyridinyliden)-3-(4'-hydroxyphenyl)-1-formazano]-1,3-dihydroxyethylimidazolium-chlorid,
- 2-[5-(N-Hydroxyethyl-4-pyridinyliden)-3-(4'-N,N-dimethylaminophenyl)-1-formazano]-1,3-dihydroxyethylimidazolium-chlorid,
- 2-[5-(N-Carboxyethyl-4-pyridinyliden)-1-formazano]-1,3-dicarboxyethylimidazolium-chlorid,
- 2-[5-(N-Carboxyethyl-4-pyridinyliden)-3-methyl-1-formazano]-1,3-dicarboxyethylimidazolium-chlorid,
- 2-[5-(N-Carboxyethyl-4-pyridinyliden)-3-ethyl-1-formazano]-1,3-dicarboxyethylimidazolium-chlorid,
- 2-[5-(N-Carboxyethyl-4-pyridinyliden)-3-phenyl-1-formazano]-1,3-dicarboxyethylimidazolium-chlorid,
- 2-[5-(N-Carboxyethyl-4-pyridinyliden)-3-isopropyl-1-formazano]- 1,3-dicarboxyethylimidazolium-chlorid,
- 2-[5-(N-Carboxyethyl-4-pyridinyliden)-3-(4'-methoxyphenyl)-1-formazano]-1,3-dicarboxyethylimidazolium-chlorid,
- 2-[5-(N-Carboxyethyl-4-pyridinyliden)-3-(4'-hydroxyphenyl)-1-formazano]-1,3-dicarboxyethylimidazolium-chlorid,
- 2-[5-(N-Carboxyethyl-4-pyridinyliden)-3-(4'-N,N-dimethylaminophenyl)-1-formazano]-1,3-dicarboxyethylimidazolium-chlorid.

14. Verfahren nach einem der vorhergehenden Ansprüche, **dadurch gekennzeichnet, dass** das Oxidationsmittel ausgewählt wird unter Wasserstoffperoxid; Harnstoffperoxid; organischen Persäuren; Persalzen; Persulfaten; Chromaten oder Bichromaten; Hypochloriten; Hypobromiten; Hexacyanoferraten(III); Mangandioxid und Bleidioxid, die allein oder in Form von Gemischen eingesetzt werden.

15. Verfahren nach dem vorhergehenden Anspruch, **dadurch gekennzeichnet, dass** das Oxidationsmittel Wasserstoffperoxid ist.

16. Verfahren nach einem der vorhergehenden Ansprüche, **dadurch gekennzeichnet, dass** das Oxidationsmittel unter Enzymen in Gegenwart eines für das Enzym geeigneten Substrats ausgewählt wird.

17. Verfahren nach dem vorhergehenden Anspruch, **dadurch gekennzeichnet, dass** das Enzym unter den in Gegenwart eines Substrats für dieses Enzym Wasserstoffperoxid erzeugenden Oxidasen ausgewählt wird.

18. Verfahren nach dem vorhergehenden Anspruch, **dadurch gekennzeichnet, dass** das Enzym ausgewählt wird unter:
| | |
|---|---|
| PYRANOSEOXIDASE | EC 1.1.3.10 |
| L-SORBOSEOXIDASE | EC 1.1.3.11 |
| PYRIDOXIN-4-OXIDASE | EC 1.1.3.12 |
| ALKOHOLOXIDASE | EC 1.1.3.13 |
| CATECHOLOXIDASE (DIMERISIEREND) | EC 1.1.3.14 |
| (S)-2-HYDROXYSÄUREOXIDASE | EC 1.1.3.15 |
| ECDYSONOXIDASE | EC 1.1.3.16 |
| CHOLINOXIDASE | EC 1.1.3.17 |
| SEC-ALKOHOL-OXIDASE | EC 1.1.3.18 |
| 4-HYDROXYMANDRLATOXIDASE | EC 1.1.3.19 |
| LONG-CHAIN-ALKOHOL-OXIDASE | EC 1.1.3.20 |
| GLYCERIN-3-PHOSPHAT-OXIDASE | EC 1.1.3.21 |
| XANTHINOXIDASE | EC 1.1.3.22 |
| THIAMINOXIDASE | EC 1.1.3.23 |
| L-GALACTONOLACTONOXIDASE | EC 1.1.3.24 |
| CELLOBIOSEOXIDASE | EC 1.1.3.25 |
| HYDROXYPHYTANATOXIDASE | EC 1.1.3.27 |
| NUCLEOSIDOXIDASE | EC 1.1.3.28 |
| N-ACYLHEXOSAMIN-OXIDASE | EC 1.1.3.29 |
| MALATOXIDASE | EC 1.1.3.3 |
| POLYVINYLALKOHOLOXIDASE | EC 1.1.3.30 |
| METHANOLOXIDASE | EC 1.1.3.31 |
| D-ARABINONO-1,4-LACTON-OXIDASE | EC 1.1.3.37 |
| VANILLYLALKOHOLOXIDASE | EC 1.1.3.38 |
| NUCLEOSIDOXIDASE (H₂O₂-ERZEUGEND) | EC 1.1.3.39 |
| GLUCOSEOXIDASE | EC 1.1.3.4 |
| D-MANNITOXIDASE | EC 1.1.3.40 |
| XYLITOXIDASE | EC 1.1.3.41 |
| HEXOSEOXIDASE | EC 1.1.3.5 |
| CHOLESTERINOXIDASE | EC 1.1.3.6 |
| ARYLALKOHOLOXIDASE | EC 1.1.3.7 |
| L-GULONOLACTONOXIDASE | EC 1.1.3.8 |
| GALACTOSEOXIDASE | EC 1.1.3.9 |
| PTERIDINOXIDASE | EC 1.17.3.1 |
| RETINALOXIDASE | EC 1.2.3.11 |
| ALDEHYDOXIDASE | EC 1.2.3.1 |
| KOHLENMONOXIDOXIDASE | EC 1.2.3.10 |
| VANILLATDEMETHYLASE | EC 1.2.3.12 |
| 4-HYDROXYPHENYLPYRUVAT-OXIDASE | EC 1.2.3.13 |
| PYRUVATOXIDASE | EC 1.2.3.3 |
| OXALATOXIDASE | EC 1.2.3.4 |
| GLYOXALATOXIDASE | EC 1.2.3.5 |
| PYRUVATOXIDASE (Co-A-ACETYLIEREND) | EC 1.2.3.6 |
| INDOL-3-ACETALDEHYD-OXIDASE | EC 1.2.3.7 |
| PYRIDOXALOXIDASE | EC 1.2.3.8 |
| ARYLALDEHYDOXIDASE | EC 1.2.3.9 |
| DIHYDROOROTAT-OXIDASE | EC 1.3.3.1 |
| LATHOSTEROLOXIDASE | EC 1.3.3.2 |
| COPROPORPHYRINOGEN-OXIDASE | EC 1.3.3.3 |
| PROTOPORPHYRINOGEN-OXIDASE | EC 1.3.3.4 |
| BILIRUBINOXIDASE | EC 1.3.3.5 |
| ACYL-Co-A-OXIDASE | EC 1.3.3.6 |
| DIHYDROURACILOXIDASE | EC 1.3.3.7 |
| TETRAHYDROBERBERINOXIDASE | EC 1.3.3.8 |
| SECOLOGANINSYNTHASE | EC 1.3.3.9 |
| ASPARTATOXIDASE | EC 1.4.3.1 |
| PUTRESCINOXIDASE | EC 1.4.3.10 |
| L-GLUTAMATOXIDASE, | EC 1.4.3.11 |
| CYCLOHEXYLAMINOXIDASE | EC 1.4.3.12 |
| PROTEIN-LYSIN-6-OXIDASE | EC 1.4.3.13 |
| L-LYSINOXIDASE | EC 1.4.3.14 |
| D-GLUTAMAT(D-ASPARTAT)-OXIDASE | EC 1.4.3.15 |
| L-ASPARTATOXIDASE | EC 1.4.3.16 |
| TRYPTOPHAN-ALPHA. BETA-OXIDASE | EC 1.4.3.17 |
| GLYCINOXIDASE | EC 1.4.3.19 |
| L-AMINOSÄUREOXIDASE | EC 1.4.3.2 |
| D-AMINOSÄUREOXIDASE | EC 1.4.3.3 |
| AMINOXIDASE (FLAVIN ENTHALTEND) | EC 1.4.3.4 |
| PYRIDOXAMINPHOSPHAT-OXIDASE | EC 1.4.3.5 |
| AMINOXIDASE (KUPFER ENTHALTEND) | EC 1.4.3.6 |
| D-GLUTAMAT-OXIDASE | EC 1.4.3.7 |
| ETHANOLAMIN-OXIDASE | EC 1.4.3.8 |
| SARCOSIN-OXIDASE | EC 1.5.3.1 |
| DIMETHYLGLYCIN-OXIDASE | EC 1.5.3.10 |
| POLYAMINOXIDASE | EC 1.5.3.11 |
| DIHYDROBENZOPHENANTHRIDIN-OXIDASE | EC 1.5.3.12 |
| N-METHY-L-AMINOSÄURE-OXIDASE | EC 1.5.3.2 |
| N-METHYL-LYSIN-OXIDASE | EC 1.5.3.4 |
| (S)-6-HYDROXYNICOTIN-OXIDASE | EC 1.5.3.5 |
| (R)-6-HYDROXYNICOTIN-OXIDASE | EC 1.5.3.6 |
| L-PIPECOLATOXIDASE | EC 1.5.3.7 |
| RETICULATOXIDASE | EC 1.5.3.9 |
| NITROETHANOXIDASE | EC 1.7.3.1 |
| ACETYLINDOXYLOXIDASE | EC 1.7.3.2 |
| URATOXIDASE | EC 1.7.3.3 |
| HYDROXYLAMINOXIDASE | EC 1.7.3.4 |
| 3-ACYL-NITROPROPANOAT-OXIDASE | EC 1.7.3.5 |
| SULFITOXIDASE | EC 1.8.3.1 |
| THIOLOXIDASE | EC 1.8.3.2 |
| GLUTATHIONOXIDASE | EC 1.8.3.3 |
| METHANTHIOLOXIDASE | EC 1.8.3.4 |
| PRENYLCYSTEINOXIDASE | EC 1.8.3.5 |

19. Verfahren nach Anspruch 16, **dadurch gekennzeichnet, dass** das Enzym unter den Oxidoreductasen mit 4 Elektronen ausgewählt wird.

20. Verfahren nach dem vorhergehenden Anspruch, **dadurch gekennzeichnet, dass** das Enzym ausgewählt wird unter den Enzymen der Gruppe EC 1.10.3, wie Catechol-Oxidase (Polyphenol-Oxidase) EC 1.10.3.1, Laccase EC 1.10.3.2, L-Ascorbatoxidase EC 1.10.3.3 und Aminophenoloxidase EC 1.10.3.4, Tyrosinase (Monophenoloxidase) EC1.10.3.1 oder 1.14.18.1

21. Verfahren nach Anspruch 16, **dadurch gekennzeichnet, dass** das Enzym unter den Peroxidasen ausgewählt wird.

22. Verfahren nach dem vorhergehenden Anspruch, **dadurch gekennzeichnet, dass** das Enzym ausgewählt wird unter:
| | |
|---|---|
| NADH-Peroxidase | E.C 1.11.1.1 |
| NADPH-Peroxidase | E.C 1.11.1.2 |
| Fettsäureperoxidase | E.C 1.11.1.3 |
| Cytochrom-c-Peroxidase | E.C 1.11.1.5 |
| Peroxidase | E.C 1.11.1.7 |
| Iodidperoxidase | E.C 1.11.1.8 |
| Glutathionperoxidase | E.C 1.11.1.9 |
| Chloridperoxidase | E.C 1.11.1.10 |
| L-Ascorbat-Peroxidase | E.C 1.11.1.11 |
| Phospholipid-Hydroperoxid-Glutathionperoxidase | E.C 1.11.1.12 |
| Manganperoxidase | E.C 1.11.1.13 |
| Diarylpropanperoxidase | E.C 1.11.1.14 |
| Tryptophan-2,3-dioxygenase | E.C 1.13.11.11 |
| Lipoxygenase | E.C 1.13.11.12 |

23. Verfahren zum Färben von Keratinfasern, insbesondere menschlichen Keratinfasern, bei dem folgende Schritte durchgeführt werden:
a) Anwendung einer Färbezusammensetzung auf die trockenen oder feuchten Fasern, die eine Azinverbindung der Formel (VI) und/oder (VII) enthält 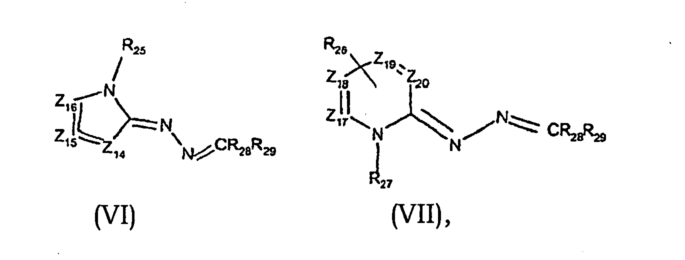 worin bedeuten:
- Z₁₄, Z₁₅, Z₁₆, R₂₅ entsprechend dasselbe wie Z₇, Z₈, Z₁₃, R₅ der Formel (IV) oder wie Z₁, Z₂, Z₀, R₁ der Formel (II), wie sie oben definiert wurden,
- Z₁₇, Z₁₈, Z₁₉, Z₂₀, R₂₆, R₂₇ entsprechend dasselbe wie Z₉, Z₁₀, Z₁₁, Z₁₂, R₈, R₇ der Formel (V) oder wie Z₃, Z₄, Z₅, Z₆, R₃, R₄ der Formel (III), wie sie oben definiert wurden,
- R₂₈, R₂₉ unabhängig voneinander ein Wasserstoffatom, eine C₁-C₆-Alkylgruppe, bevorzugt eine C₁-C₄-Alkylgruppe, eine geradkettige oder verzweigte C₁-C₆-Alkoxygruppe, bevorzugt eine C₁-C₄-Alkoxygruppe, mit der Maßgabe, dass mindestens eine der beiden Gruppen von Wasserstoff verschieden ist,
in Gegenwart eines Oxidationsmittels;
b) Einwirkenlassen während einer ausreichenden Dauer zur Entwicklung der gewünschten Färbung.

24. Färbeverfahren nach Anspruch 23, **dadurch gekennzeichnet, dass** das Oxidationsmittel unmittelbar bei der Anwendung mit der Färbezusammensetzung gemischt wird.

25. Färbeverfahren nach Anspruch 23 oder 24, **dadurch gekennzeichnet, dass** das Oxidationsmittel gleichzeitig mit der Färbezusammensetzung oder in einer Abfolge auf die Fasern angewandt wird.

26. Färbeverfahren nach einem der Ansprüche 23 bis 25, **dadurch gekennzeichnet, dass** die Färbezusammensetzung einen Gehalt an Verbindungen der Formel (VI) und/oder (VII) von 0,001 bis 10 Gew.-% und bevorzugt von 0,05 bis 5 Gew.-% aufweist, bezogen auf das Gesamtgewicht der Färbezusammensetzung.

27. Färbeverfahren nach einem der Ansprüche 23 bis 26, **dadurch gekennzeichnet, dass** die Färbezusammensetzung mindestens eine Oxidationsbase und wahlweise mindestens einen Kuppler und/oder mindestens einen Direktfarbstoff enthält.

28. Färbeverfahren nach einem der Ansprüche 23 bis 27, **dadurch gekennzeichnet, dass** die Zusammensetzung mindestens eine Oxidationsbase enthält, die ausgewählt ist unter den p-Phenylendiaminen, den Bis-phenylalkylendiaminen, den p-Aminophenolen, den o-Aminophenolen und den heterocyclischen Basen sowie den Additionssalzen dieser Verbindungen.

29. Färbeverfahren nach Anspruch 28, **dadurch gekennzeichnet, dass** dieFärbezusammensetzung einen Gehalt an Oxidationsbase von 0,001 bis 10 Gew.-% aufweist, bezogen auf das Gesamtgewicht der Färbezusammensetzung.

30. Färbeverfahren nach einem der Ansprüche 23 bis 29, **dadurch gekennzeichnet, dass** sie mindestens einen Kuppler enthält, der aus der Gruppe ausgewählt ist, die besteht aus m-Phenylendiaminen, m-Aminophenolen, m-Diphenolen, Naphthalin-Kupplern und heterocyclischen Kupplern sowie den Additionssalzen dieser Verbindungen.

31. Färbeverfahren nach Anspruch 30, **dadurch gekennzeichnet, dass** die Färbezusammensetzung einen Gehalt an Kupplern von 0,001 bis 10 Gew.-% aufweist, bezogen auf das Gesamtgewicht der Färbezusammensetzung.

32. Färbeverfahren nach einem der Ansprüche 23 bis 31, **dadurch gekennzeichnet, dass** die Färbezusammensetzung mindestens einen Direktfarbstoff enthält.

33. Färbeverfahren nach Anspruch 32, **dadurch gekennzeichnet, dass** die Färbezusammensetzung einen Gehalt an Direktfarbstoff von 0,001 bis 20 Gew.-% aufweist, bezogen auf das Gesamtgewicht der Färbezusammensetzung.
